# FASCICULE DE BREVET EUROPEEN

(11) **EP 2 741 765 B1**
(45) Date de publication et mention de la délivrance du brevet: **30.03.2016**
(21) Numéro de dépôt: 12758562.8
(22) Date de dépôt: 09.08.2012
(51) Int. Cl.: A61K 38/28, A61K 47/36, A61K 9/00

(54) **SOLUTION INJECTABLE D'AU MOINS UNE INSULINE BASALE**
INJEKTIONSLÖSUNG MIT MINDESTENS EINER ART VON BASALINSULIN
INJECTABLE SOLUTION OF AT LEAST ONE TYPE OF BASAL INSULIN

(30) Priorité: 10.08.2011 FR 1157291; 10.08.2011 US 201161522031 P; 23.12.2011 FR 1162445; 23.12.2011 US 201161579966 P
(43) Date de publication de la demande: 18.06.2014
(73) Titulaire: Adocia, 69003 Lyon (FR)
(72) Inventeur: SOULA, Olivier, F-69330 Meyzieu (FR); SOULA, Gérard, F-69330 Meyzieu (FR); TONNAR, Jeff, F-69003 Lyon (FR); GEISSLER ALEXANDRE, 69007 Lyon (FR)
(74) Mandataire: Tripoz, Inès
(86) Numéro de dépôt international: PCT/FR2012/051880
(87) Numéro de publication internationale: WO 2013/021143

(56) Documents cités:
- WO-A1-2007/116143
- WO-A1-2010/122385
- WO-A1-2011/077405
- WO-A2-2007/121256
- WO-A2-2010/041138
- WO-A2-2011/098962
- US-A1- 2011 178 011

## Description

L'invention concerne les thérapies par Injection d'insuline(s) pour traiter le diabète.

L'insulinothérapie, ou thérapie du diabète par injection d'insuline, a connu ces dernières années des progrès remarquables grâce notamment à la mise au point de nouvelles insulines offrant une correction de la glycémie des patients, ce qui permet de mieux simuler l'activité physiologique du pancréas.

Pour couvrir ses besoins journaliers en insuline, un patient diabétique dispose, actuellement, de façon schématisée, de deux types d'insulines ayant des actions complémentaires : les insulines prandiales (ou Insulines dites à action rapide) et les Insulines basales (ou insulines dites à action lente).

Les insulines prandiales permettent une prise en charge rapide (métabolisation et/ou stockage) du glucose apporté lors des repas et collations. Le patient doit s'injecter une insuline prandiale avant chaque prise alimentaire, soit environ 2 à 3 injections par jour. Les Insulines prandiales les plus utilisées sont : l'insuline humaine recombinante, Novotog^{®} (insuline aspart de NOVO NORDISK), Humalog^{®} (insuline lispro de ELI LILLY) et Apidra^{®} (insuline glulisine de SANOFI-AVENTIS).

Les Insulines basales assurent le maintien de l'homéostasie glycémique du patient, en dehors des périodes de prise alimentaire. Elles agissent essentiellement pour bloquer la production endogène de glucose (glucose hépatique). La dose journalière d'insuline basale correspond généralement à 40-50 % des besoins totaux journaliers en insuline. Selon l'insuline basale utilisée, cette dose est dispensée en 1 ou 2 injections, régulièrement réparties au cours de la journée. Les insulines basales les plus utilisées sont Levernir^{®} (insuline detemir de NOVO NORDISK) et Lantus^{®} (insuline glargine de SANOFI-AVENTIS).

On notera pour être exhaustif que la NPH (Insuline NPH pour Neutral Protamine Hagedom ; Humuline NPH^{®}, Insulatard^{®}) est la plus ancienne insuline basale. Cette formulation est le résultat d'une précipitation de l'insuline humaine (anionique à pH neutre) par une protéine cationique, la protamine. Ces microcristaux sont dispersés dans une suspension aqueuse et se dissolvent lentement après injection sous-cutanée. Cette dissolution lente assure une libération prolongée de l'insuline. Cependant cette libération n'assure pas une concentration constante d'insuline au cours du temps. Le profil de libération est en forme de cloche et dure seulement entre 12 et 16 heures. Elle est donc injectée deux fois par jour. Cette insuline basale NPH est bien moins performante que les insulines basales modernes, levemir^{®} et Lantus^{®}. La NPH est une insuline basale à action intermédiaire.

Le principe de la NPH a évolué avec l'apparition des insulines analogues rapides pour donner des produits appelés "Premix" offrant à la fois une action rapide et une action intermédiaire. NovoLog Mix^{®} (NOVO NORDISK) et Humalog Mix^{®} (EU LILLY) sont des formulations comprenant une insuline analogue rapide, Novolog^{®} et Humalog^{®}, complexée partiellement par la protamine. Ces formulations contiennent ainsi des microcristaux d'insuline dont l'action est dite intermédiaire et une partie d'insuline restée soluble dont l'action est rapide. Ces formulations offrent bien l'avantage d'une insutine rapide mais elles ont aussi le défaut de la NPH, c.-à-d. une durée d'action limitée entre 12 et 16 heures et une insuline libérée en « cloche ». Cependant, ces produits permettent au patient de s'injecter en une seule fois une insuline basale à action intermédiaire avec une insuline prandiale à action rapide. Or nombreux sont les patients soucieux de réduire leur nombre d'injections.

Les insulines basales actuellement commercialisées et actuellement en développement clinique peuvent être classées en fonction de la solution technique qui permet d'obtenir l'action prolongée et à ce jour deux approches sont utilisées.

La première, celle de l'insuline detemir est la liaison à l'albumine *in vivo.* Il s'agit d'un analogue, soluble à pH 7, qui comprend une chaine latérale d'acide gras (tetradecanoyl) fixée à la position B29 qui, *in vivo,* permet à cette insuline de s'associer à l'albumine. Son action prolongée est principalement due à cette affinité pour l'albumine après injection sous-cutanée.

Cependant son profil pharmacocinétique ne permet pas de couvrir une journée, ce qui fait qu'elle est le plus souvent utilisée en deux injections par jour.

D'autres Insulines basales solubles à pH 7, comme Degludec^{®} sont actuellement en développement. Degludec^{®} comprend également une chaîne latérale d'acide gras fixée sur l'insuline (hexadecandioyl-y-L-Glu).

La seconde, celle de l'insuline glargine, est la précipitation à pH physiologique. Il s'agit d'un analogue de l'insuline humaine obtenu par élongation de la partie C-terminale de la chaîne B de l'insuline humaine par deux résidus d'arginine, et par substitution du résidu d'asparagine A21, par un résidu de glycine (US 5,656,722). L'addition des deux résidus d'arginine a été pensée pour ajuster le pI (point isoélectrique) d'insuline glargine au pH physiologique, et ainsi rendre cet analogue de l'insuline insoluble en milieu physiologique.

La substitution de l'A21 a été pensée afin de rendre l'insuline glargine stable à pH acide et pouvoir ainsi la formuler sous forme de solution injectable à pH acide. Lors de l'injection sous-cutanée, le passage de l'insuline glargine d'un pH acide (pH 4-4,5) à un pH physiologique (pH neutre) provoque sa précipitation sous la peau. La redissolution lente des micro-particules d'insuline glargine assure une action lente et prolongée.

L'effet hypoglycémiant de l'insuline glargine est quasi-constant sur une durée de 24 heures ce qui permet à la plupart des patients de se limiter à une seule injection par jour.

L'insuline glargine est considérée aujourd'hui comme la meilleure insuline basale commercialisée.

Cependant le pH acide des formulations des insulines basales, dont le point isoélectrique est compris entre 5,8 et 8,5, de type insuline glargine, empêche toute combinaison pharmaceutique avec d'autres protéines et en particulier les insulines prandiales car ces dernières ne sont pas stables à pH adde.

Cependant, personne n'a à ce jour, cherché à solubiliser ces insulines basales dont le point isoélectrique est compris entre 5,8 et 8,5 de type insuline glargine, à pH neutre, tout en maintenant une différence de solubilité entre le milieu *in-vitro* (le contenant) et le milieu *in-vivo* (sous la peau), indépendamment du pH.

En effet, le principe de fonctionnement des insulines basales, de type insuline glargine, exposé ci-dessus à savoir qu'elles sont solubles à pH acide et précipitent à pH physiologique détourne l'homme de l'art de toute solution dans laquelle l'insuline de type insuline glargine serait solubilisée à pH 6-8 tout en gardant sa propriété essentielle qui est de précipiter en milieu sous cutané.

Par ailleurs, l'impossibilité de formuler une insuline prandiale, à pH acide, tient au fait qu'une insuline prandiale subit, dans ces conditions, une réaction secondaire de déamidation en position A21 ; ce qui ne permet pas de répondre à l'exigence de la Pharmacopée US, à savoir moins de 5 % de produit secondaire après 4 semaines à 30°C.

De plus, ce pH acide des formulations des insulines basales, dont le point isoélectrique est compris entre 5,8 et 8,5, de type insuline glargine, empêche même toute combinaison extemporanée avec les insulines prandiales à pH neutre.

Une étude clinique récente, présentée au 69th Scientific Sessions of the American Diabetes Association, New Orleans, Louisiana, 5-9 Juin 2009, a permis de vérifier cette limitation d'usage d'insuline glargine. Une dose d'insuline glargine et une dose d'insuline prandiale (en l'occurrence, l'insuline lispro) ont été mélangées juste avant injection (E. Cengiz et al., 2010 ; Diabetes care - 33(5):1009-12). Cette expérience a permis de mettre en évidence un retard significatif dans les profils pharmacocinétique et pharmacodynamique de l'insuline prandiale, pouvant donner lieu à des hyperglycémies postprandiales et à des hypoglycémies nocturnes. Cette étude confirme bien l'incompatibilité de l'insuline glargine avec les Insulines à action rapide actuellement commercialisées.

D'ailleurs la notice d'utilisation de Lantus^{®}, le produit commercial à base d'insuline glargine de la société SANOFI-AVENTIS, indique explicitement aux utilisateurs de ne pas réaliser de mélange avec une solution d'insuline prandiale, quelle qu'elle soit, en raison du risque sérieux de modifier la pharmacocinétique et la pharmacodynamique de l'insuline glargine et/ou de l'insuline prandiale mélangée.

Pourtant, d'un point de vue thérapeutique, des études cliniques rendues publiques lors des 70èmes séances scientifiques annuelles de l'American Diabetes Association (ADA) de 2010. abstract 2163-PO et abstract numéro 0001-LB, en particulier celles menées par la société SANOFI-AVENTIS, ont montré que les traitements qui associent Lantus^{®}, insuline glargine, et une insulines prandiale sont bien plus efficaces que les traitements à base de produits du type « Premix », Novolog Mix^{®} ou Humalog Mix^{®}.

S'agissant des combinaisons d'insuline glargine et d'insuline rapide, la société Biodel, a décrit notamment dans la demande de brevet US 7,718,609 des compositions comprenant une insuline basale et une insuline prandiale à un pH compris entre 3,0 et 4,2 en présence d'un agent chélatant et de polyacides. Ce brevet enseigne comment rendre compatible une insuline prandiale à pH acide en présence d'une insuline de type insuline glargine. Il n'enseigne pas comment préparer une combinaison d'insuline de type insuline glargine et d'une insuline prandiale à pH neutre.

De l'analyse des compositions décrites dans la littérature et les brevets il apparait que l'insolubilité à pH 7 des insulines basales du type Insuline glargine est un prérequis pour avoir son action lente. De ce fait, toutes les solutions proposées pour les combiner à d'autres produits comme les insulines prandiales sont basées sur des essais de solubilisation ou stabilisation des insulines prandiales à pH acide, voir par exemple WO 2007/121256 et WO 2009/021955.

De façon surprenante, les compositions selon l'invention permettent de solubiliser à pH 7 une Insuline basale dont le point isoélectrique est compris entre 5,8 et 8,5.

De façon surprenante les compositions selon l'invention permettent un maintien de la durée de l'activité hypoglycémiante de l'insuline basale dont le point isoélectrique est compris entre 5,8 et 8,5 malgré sa solubilisation à pH 7 avant injection. Cette propriété remarquable provient du fait que l'insuline de type insuline glargine solubilisée à pH 7 dans la composition de l'invention précipite en milieu sous-cutané par un changement de composition du milieu. L'élément déclenchant la précipitation de l'insuline de type insuline glargine n'est plus la modification de pH mais une modification de composition de l'environnement lors du passage de la composition pharmaceutique au milieu physiologique.

La présente invention, en résolvant ce problème de solubilité à pH 7 permet :
- de proposer une composition injectable, destinée au traitement du diabète, comprenant une Insuline basale dont le point isoélectrique est compris entre 5,8 et 8,5, sous la forme d'une solution homogène à pH 7, tout en conservant son activité biologique et son profil d'action;
- de proposer une composition sous la forme d'une formulation comprenant une combinaison d'une insuline basale dont le point isoélectrique est compris entre 5,8 et 8,5 et d'une insuline prandiale sans modification du profit d'activité de l'insuline prandiale soluble à pH 6-8 et instable à pH acide tout en maintenant le profil d'action basale propre à l'insuline basale ;
- de proposer une composition injectable, destinée au traitement du diabète, comprenant en outre une combinaison d'une insuline basale dont le point isoélectrique est compris entre 5,8 et 8,5 et un dérivé ou un analogue d'une hormone gastro intestinale comme le GLP-1 ou « glucagon like peptide-1 » ;
- aux patients de diminuer le nombre de leurs injections ;
- aux dites compositions d'être conformes aux exigences des Pharmacopées américaines et européennes.

De façon surprenante, dans les combinaisons de l'insuline de type insuline glargine avec une insuline prandiale, objets de l'invention, l'action rapide de l'insuline prandiale est préservée malgré la précipitation de l'insuline de type insuline glargine en milieu sous-cutané.

L'invention concerne une composition sous forme d'une solution aqueuse injectable, dont le pH est compris entre 6,0 et 8,0, comprenant au moins :
a) une insuline basale dont le point isoélectrique pI est compris entre 5,8 et 8,5 ;
b) un dextrane substitué par des radicaux porteurs de charges carboxylates et des radicaux hydrophobes de formule I ou de formule II : 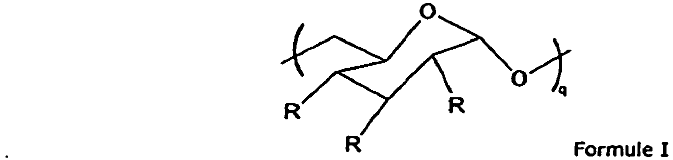 dans laquelle,
   - R est -OH ou choisi dans le groupe constitué par les radicaux :
      ∘ -(*f*-[A]-COOH)ₙ ;
      ∘ -(*g*-[8]-*k*-[D])ₘ, D comportant au moins une chaîne alkyle comportant au moins 8 atomes de carbone ;
   - n représente le degré de substitution des unités glucosidiques par -*f*-[A]-COOH et 0,1 ≤ n ≤2 ;
   - m représente le degré de substitution des unités glucosidiques par *-*g-[B]-*k-*[D] et 0 < m ≤ 0,5 ;
   - q représente le degré de polymérisation en unités glucosidiques, c'est-à-dire le nombre moyen d'unités glucosidiques par chaîne de polysaccharide et 3 ≤ q ≤ 50 ;
   - -(*f*-tA]-COOH)ₙ ;
      ∘ -A- est un radical linéaire ou ramifié comprenant 1 à 4 atomes de carbone ; ledit radical -A- :
      ∘ étant lié à une unité glucosidique par une fonction *f* choisie dans le groupe constitué par les fonctions éther, ester et carbamate ;
   - -(*g*-[8]-*k*-[D]ₘ ;
      ∘ -B- est un radical au moins divalent linéaire ou ramifié comprenant 1 à 4 atomes de carbone ; ledit radical -B- :
      ∘ étant lié à une unité glucosidique par une fonction *g* choisie dans le groupe constitué par les fonctions éther, ester et carbamate ;
      ∘ étant lié à un radical -D par une fonction *k* ; *k* choisie dans le groupe constitué par les fonctions ester, amide, et carbamate ; ledit radical - D :
         - étant un radical -X(-*I*-Y)ₚ, X étant un radical au moins divalent comprenant de 1-à 12 atomes choisis dans le groupe constitué par des atomes de C, N ou O, éventuellement porteur de fonctions carboxyle ou amine et/ou issu d'un acide aminé, d'un dialcool, d'une diamine ou d'un mono- ou polyéthylène glycol mono- ou diamine ; Y étant un groupe alkyle, linéaire ou cyclique, un alkylaryle ou un arylalkyle, en C8 à C30, éventuellement substitué par un ou plusieurs groupes alkyles en C1 à C3 ; p ≥ 1 et *I* une fonction choisie dans le groupe constitué par les fonctions ester, amide et carbamate ;
   - *f, g* et *k* étant identiques ou différentes ;
   - les fonctions acides libres étant sous forme de sels de cations alcalins choisis dans le groupe constitué par Na⁺ et K⁺ ;
   - et lorsque p = 1, si Y est un alkyle en C8 à C14, alors q*m ≥ 2, si Y est un alkyle en C15, alors q*m ≥ 2 ; et si Y est un alkyle en C16 à C20, alors q*m ≥ 1 ;
   - et lorsque p ≥ 2, si Y est un alkyle en C8 à C9, alors q*m ≥ 2 et, si Y est alkyle en C10 à C16, alors q*m ≥ 0,2. 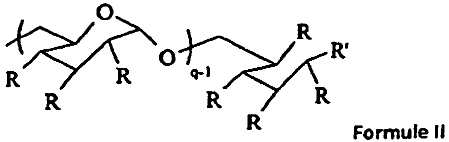 dans laquelle,
   - R est -OH ou un radical -(*f*-[A]-COOH)ₙ :
      ∘ -A- est un radical linéaire ou ramifié comprenant 1 à 4 atomes de carbone ; ledit radical -A- :
      ∘ étant lié à une unité glucosidique par une fonction f choisie dans le groupe constitué par les fonctions éther, ester ou carbamate ;
      ∘ n représente le degré de substitution des unités glucosidiques par -f-[A]-COOH et 0,1 5 n ≤ 2 ;
   - R' est choisi dans le groupe constitué par les radicaux :
      ∘ -C(O)NH-[E]-(*o*-[F])ₜ ;
      ∘ -CH₂N(L)_{z}-[E]-(*o*-[F])ₜ ;
      dans lesquels,
      ∘ z est un entier positif égal à 1 ou 2,
      ∘ L est choisi dans le groupe constitué de :
         • -H et z est égal à 1, et/ou
         • -[A]-COOH et z est égal à 1 ou 2, si *f* est une fonction éther,
         • -CO-[A]-COOH et z est égal à 1 si *f* est une fonction ester, et
         • -CO-NH-[A]-COOH et z est égal à 1 si *f* est une fonction carbamate ;
      ∘ -[E]-(*o*-[F])ₜ :
         • -E- est un radical au moins divalent linéaire ou ramifié comprenant 1 à 8 atomes de carbone et comprenant éventuellement des hétéroatomes tels que O, N ou S ;
         ▪ -F étant un groupe alkyle, linéaire ou cyclique, un alkylaryle ou un arylalkyle, en C12 à C30, éventuellement substitué par un ou plusieurs groupes alkyles en C1 à C3 ;
         • *o* est une fonction choisie dans le groupe constitué par les fonctions éther, ester, amide ou carbamate ;
         • t est un entier positif égal à 1 ou 2 ;
   - q représente le degré de polymérisation en unités glucosidiques, c'est-à-dire le nombre moyen d'unités glucosidiques par chaîne de polysaccharide et 3 ≤ q ≤ 50 ;
   - les fonctions acides libres étant sous forme de sels de cations alcalins choisis dans le groupe constitué par Na⁺ et K⁺ ;
   - lorsque z = 2, l'atome d'azote est sous forme d'un ammonium quaternaire.

Dans un mode de réalisation, lorsque p = 1, si Y est un groupe en C21 à C30, alors q*m ≥ 1.

Dans un mode de réalisation, lorsque p = 1, si Y est un groupe en C21 à C30, alors q*m ≥ 0,1.

Dans un mode de réalisation le radical -(*f*-[A]-COOH)ₙ est tel que :
- -A- est un radical comprenant 1 atome de carbone ; ledit radical -A- étant lié à une unité glucosidique par une fonction *f* éther.

Dans un mode de réalisation le radical -(*g*-[B]-*k*-[D])ₘ est tel que :
- -B- est un radical comprenant 1 atome de carbone ; ledit radical -B- étant lié à une unité glucosidique par une fonction *g* éther, et,
- X est un radical issu d'un acide aminé.

Dans un mode de réalisation le radical -(*f*-[A]-COOH)ₙ est tel que :
- -A- est un radical comprenant 1 atome de carbone ; ledit radical -A- étant lié à une unité glucosidique par une fonction *f* éther, et,
- le radical -(*g*-[B]-*k*-[D])ₘ est tel que :
- -B- est un radical comprenant 1 atome de carbone ; ledit radical -B- étant lié à une unité glucosidique par une fonction *g* éther, et,
- X est un radical issu d'un acide aminé,
- *k* est une fonction amide.

Dans un mode de réalisation, le dextrane substitué par des radicaux porteurs de charges carboxylates et des radicaux hydrophobes est de formule III : 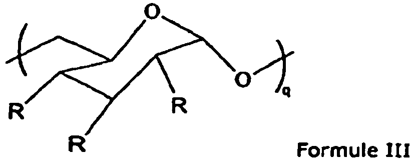 dans laquelle,
- R est -OH ou choisi dans le groupe constitué par les radicaux :
   ∘ -(*f*-[A]-COOH)ₙ ;
   ∘ -(*g*-[B]-*k*-[D])ₘ, D comportant au moins une chaîne alkyle comportant au moins 8 atomes de carbone ;
- n représente le degré de substitution des unités glucosidiques par -*f*-[A]-COOH et 0,1 ≤ n ≤ 2 ;
- m représente le degré de substitution des unités glucosidiques par -*g*-[B]-*k*-[D] et 0 < m ≤ 0,5 ;
- q représente le degré de polymérisation en unités glucosidiques, c'est-à-dire le nombre moyen d'unités glucosidiques par chaîne de polysaccharide et 3 ≤ q ≤ 50 ;
- -(*f*-[A]-COOH)ₙ:
   ∘ -A- est un radical linéaire ou ramifié comprenant 1 à 4 atomes de carbone ; ledit radical -A- :
   ∘ étant lié à une unité glucosidique par une fonction *f* choisie dans le groupe constitué par les fonctions éther, ester et carbamate ;
- -(*g*-[B]-*k*-[D])ₘ :
   ∘ -B- est un radical au moins divalent linéaire ou ramifié comprenant 1 à 4 atomes de carbone ; ledit radical -B- :
   ∘ étant lié à une unité glucosidique par une fonction *g* choisie dans le groupe constitué par les fonctions éther, ester et carbamate ;
   ∘ étant lié à un radical -D par une fonction *k* ; *k* choisie dans le groupe constitué par les fonctions ester, amide, et carbamate ; ledit radical - D:
      - étant un radical -X(-*I*-Y)ₚ, X étant un radical au moins divalent comprenant de 1 à 12 atomes choisis dans le groupe constitué par des atomes de C, N ou O, éventuellement porteur de fonctions carboxyle ou amine et/ou issu d'un acide aminé, d'un dialcool, d'une diamine ou d'un mono- ou polyéthylène glycol mono- ou diamine ; Y étant un groupe alkyle, linéaire ou cyclique, un alkylaryle ou un arylalkyle, en C8 à C20, éventuellement substitué par un ou plusieurs groupes alkyles en C1 à C3 ; p ≥ 1 et *I* une fonction choisie dans le groupe constitué par les fonctions ester, amide et carbamate ;
- *f, g* et *k* étant identiques ou différentes ;
- les fonctions acides libres étant sous forme de sels de cations alcalins choisis dans le groupe constitué par Na⁺ et K⁺ ;
- et lorsque p = 1, si Y est un alkyle en C8 à C14, alors q*m ≥ 2, si Y est un alkyle en C15, alors q*m ≥ 2 ; et si Y est un alkyle en C16 à C20, alors q*m ≥ 1 ;
- et lorsque p ≥ 2, si Y est un alkyle en C8 à C11, alors q*m ≥ 2 et, si Y est alkyle en C12 à C16, alors q*m ≥ 0,3.

Dans un mode de réalisation, le dextrane substitué par des radicaux porteurs de charges carboxylates et des radicaux hydrophobes est de formule IV : 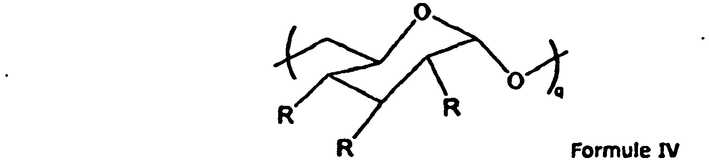 dans laquelle,
- R est -OH ou choisi dans le groupe constitué par les radicaux :
   ∘ -(*f*-[A]-COOH)ₙ ;
   ∘ -(*g*-[B]-*k*-[D])ₘ, D comportant au moins une chaîne alkyle comportant au moins 8 atomes de carbone ;
- n représente le degré de substitution des fonctions hydroxyles -OH par -*f*-[A]-COOH par unité glucosidique ; et 0,1 ≤ n S 2 ;
- m représente le degré de substitution des fonctions hydroxyles -OH par -*g*-(B]-*k*-[D] par unité glucosidique ; et 0 < m ≤ 0,5 ;
- q représente le degré de polymérisation en unités glucosidiques, c'est-à-dire le nombre moyen d'unités glucosidiques par chaîne de polysaccharide et 3 ≤ q ≤ 50;
- -(*f*-[A]-COOH)ₙ ;
   ∘ -A- est un radical linéaire ou ramifié comprenant 1 à 4 atomes de carbone ; ledit radical -A- :
   ∘ étant lié à une unité glucosidique par une fonction *f* choisie dans le groupe constitué par les fonctions éther, ester et carbamate ;
- -(*g*-[8]-*k*-[D])ₘ ;
   ∘ -B- est un radical au moins divalent linéaire ou ramifié comprenant 1 à 4 atomes de carbone ; ledit radical -B- :
   ∘ étant lié à une unité glucosidique par une fonction *g* choisie dans le groupe constitué par les fonctions éther, ester et carbamate ;
   ∘ étant lié à un radical -D par une fonction *k* ; *k* choisie dans le groupe constitué par les fonctions ester, amide, et carbamate ; ledit radical-D:
      - étant un radical -X(-*I*-Y)*ₚ*, X étant un radical au moins divalent comprenant de 1 à 12 atomes choisis dans le groupe constitué par des atomes de C, N ou O, éventuellement porteur de fonctions carboxyle ou amine et,/ou issu d'un acide aminé, d'un dialcool, d'une diamine ou d'un mono- ou polyéthylène glycol mono- ou diamine ; Y étant un groupe alkyle, linéaire ou cyclique, un alkylaryle ou un arylalkyle, en C8 à C30, éventuellement substitué par un ou plusieurs groupes alkyles en C1 à C3 ; p ≥ 1 et *I* une fonction choisie dans le groupe constitué par les fonctions ester, amide et carbamate ;
- *f, g* et *k* étant identiques ou différentes ;
- les fonctions acides libres étant sous forme de sels de cations alcalins choisis dans le groupe constitué par Na⁺ et K⁺ ;
- et lorsque p = 1, si Y est un alkyle en C8 à C14, alors q*m ≥ 2, si Y est un alkyle en C15, alors q*m ≥ 2 ; et si Y est un alkyle en C16 à C30, alors q*m ≥ 1 ;
- et lorsque p ≥ 2, si Y est un alkyle en C8 à C9, alors q*m ≥ 2 et, si Y est alkyle en C10 à C16, alors q*m ≥ 0,2.

La structure dessinée correspond à la représentation couramment utilisée pour représenter le dextrane qui est un polysaccharide constitué en majorité d'enchaînements (1,6) entre unités glucosidiques ce qui est la représentation adoptée. Le dextrane contient également des enchaînements (1,3) à environ 5% en général qui ne sont volontairement pas représentés, mais qui sont bien sûr inclus dans la portée de l'invention.

On entend par insuline basale dont le point isoélectrique est compris entre 5,8 et 8,5 une insuline insoluble à pH 7 et dont la durée d'action est comprise entre 8 et 24 heures ou supérieure dans les modèles standards de diabète.

Ces insulines basales dont le point isoélectrique est compris entre 5,8 et 8,5 sont des insulines recombinantes dont la structure primaire a été modifiée principalement par introduction d'aminoacides basiques comme l'Arginine ou la Lysine. Elles sont décrites par exemple dans les brevets, demandes de brevets ou publications suivants WO 2003/053339, WO 2004/096854, US 5,656,722 et US 6,100,376.

Dans un mode de réalisation, l'insuline basale dont le point isoélectrique est compris entre 5,8 et 8,5 est l'insuline glargine.

Dans un mode de réalisation les compositions selon l'invention comprennent 100 UI/mL (soit environ 3,6 mg/mL) d'insuline basale dont le point isoétectrique est compris entre 5,8 et 8,5.

Dans un mode de réalisation les compositions selon l'invention comprennent 40 UI/mL d'insuline basale dont le point isoélectrique est compris entre 5,8 et 8,5.

Dans un mode de réalisation les compositions selon l'invention comprennent 200 UI/mL d'insuline basale dont le point isoélectrique est compris entre 5,8 et 8,5.

Dans un mode de réalisation les compositions selon l'invention comprennent 300 UI/mL d'insuline basale dont le point isoélectrique est compris entre 5,8 et 8,5.

Dans un mode de réalisation les compositions selon l'invention comprennent 400 UI/mL d'insuline basale dont le point isoélectrique est compris entre 5,8 et 8,5.

Dans un mode de réalisation les compositions selon l'invention comprennent 500 UI/mL d'insuline basale dont le point isoélectrique est compris entre 5,8 et 8,5.

Dans un mode de réalisation le ratio massique entre l'insuline basale, dont le point isoélectrique est compris entre 5,8 et 8,5, et le dextrane substitué, soit dextrane substitué/insuline basale, est compris entre 0,2 et 5.

Dans un mode de réalisation le ratio massique entre l'insuline basale, dont le point Isoélectrique est compris entre 5,8 et 8,5, et le dextrane substitué, soit dextrane substitué/insuline basale, est compris entre 0,2 et 4.

Dans un mode de réalisation le ratio massique entre l'insuline basale, dont le point isoélectrique est compris entre 5,8 et 8,5, et le dextrane substitué, soit dextrane substitué/insuline basale, est compris entre 0,2 et 3.

Dans un mode de réalisation le ratio massique entre l'insuline basale, dont le point isoélectrique est compris entre 5,8 et 8,5, et le dextrane substitué, soit dextrane substitué/insuline basale, est compris entre 0,5 et 3.

Dans un mode de réalisation le ratio massique entre l'insuline basale, dont le point isoélectrique est compris entre 5,8 et 8,5, et le dextrane substitué, soit dextrane substitué/insuline basale, est compris entre 0,8 et 3

Dans un mode de réalisation le ratio massique entre l'insuline basale, dont le point isoélectrique est compris entre 5,8 et 8,5, et le dextrane substitué, soit dextrane substitué/insuline basale, est compris entre 1 et 3.

Dans un mode de réalisation la concentration en dextrane substitué est comprise entre 1 et 100 mg/ml.

Dans un mode de réalisation la concentration en dextrane substitué est comprise entre 1 et 80 mg/ml.

Dans un mode de réalisation la concentration en dextrane substitué est comprise entre 1 et 60 mg/ml.

Dans un mode de réalisation la concentration en dextrane substitué est comprise entre 1 et 50 mg/ml.

Dans un mode de réalisation la concentration en dextrane substitué est comprise entre 1 et 30 mg/ml.

Dans un mode de réalisation la concentration en dextrane substitué est comprise entre 1 et 20 mg/ml.

Dans un mode de réalisation la concentration en dextrane substitué est comprise entre 1 et 10 mg/ml.

Dans un mode de réalisation la concentration en polysaccharide est comprise entre 5 et 20 mg/ml.

Dans un mode de réalisation la concentration en polysaccharide est comprise entre 5 et 10 mg/ml.

Dans un mode de réalisation les compositions selon l'invention comprennent en outre une insuline prandiale. Les insulines prandiales sont solubles à pH 7.

On entend par insuline prandiale une insuline dite rapide ou « regular ».

Les insulines prandiales dites rapides sont des insulines qui doivent répondre aux besoins provoqués par l'ingestion des protéines et des glucides durant un repas, elles doivent agir en moins de 30 minutes.

Dans un mode de réalisation les insulines prandiales dites « regular » sont choisies dans le groupe comprenant Humulin^{®} (insuline humaine) et Novolin^{®} (insuline humaine).

Les insulines prandiales dites très rapides (fast acting) sont des insulines qui sont obtenues par recombinaison et qui sont modifiées pour diminuer leur temps d'action.

Dans un mode de réalisation les insulines prandiales dites très rapides (fast acting) sont choisies dans le groupe comprenant l'insuline lispro (Humalog^{®}), l'insuline glulisine (Apidra^{®}) et l'Insuline aspart (NovoLog^{®}),

Dans un mode de réalisation les compositions selon l'invention comprennent au total 100 UI/mL (soit environ 3,6 mg/mL) d'insuline avec une combinaison d'insuline prandiale et d'insuline basale dont le point isoélectrique est compris entre 5,8 et 8,5.

Dans un mode de réalisation les compositions selon l'invention comprennent au total 40 UI/mL d'insuline avec une combinaison d'insuline prandiale et d'insuline basale dont le point Isoélectrique est compris entre 5,8 et 8,5.

Dans un mode de réalisation les compositions selon l'invention comprennent au total 200 UI/mL d'insuline avec une combinaison d'insuline prandiale et d'insuline basale dont le point Isoélectrique est compris entre 5,8 et 8,5.

Dans un mode de réalisation les compositions selon l'invention comprennent au total 300 UI/mL d'insuline avec une combinaison d'insuline prandiale et d'insuline basale dont le point isoélectrique est compris entre 5,8 et 8,5.

Dans un mode de réalisation les compositions selon l'invention comprennent au total 400 UI/mL d'insuline avec une combinaison d'insuline prandiale et d'insuline basale dont le point isoélectrique est compris entre 5,8 et 8,5.

Dans un mode de réalisation les compositions selon l'invention comprennent au total 500 UI/mL d'insuline avec une combinaison d'insuline prandiale et d'insuline basale dont le point isoélectrique est compris entre 5,8 et 8,5.

Dans un mode de réalisation les compositions selon l'invention comprennent au total 600 UI/mL d'insuline avec une combinaison d'insuline prandiale et d'insuline basale dont le point isoélectrique est compris entre 5,8 et 8,5.

Dans un mode de réalisation les compositions selon l'invention comprennent au total 700 UI/mL d'insuline avec une combinaison d'insuline prandiale et d'insuline basale dont le point isoélectrique est compris entre 5,8 et 8,5.

Dans un mode de réalisation les compositions selon l'invention comprennent au total 800 UI/mL d'insuline avec une combinaison d'insuline prandiale et d'insuline basale dont le point isoélectrique est compris entre 5,8 et 8,5.

Les proportions entre l'insuline basale dont le point isoélectrique est compris entre 5,8 et 8,5 et l'insuline prandiale exprimées en pourcentage par rapport à la quantité d'insuline totale sont par exemple de 25/75, 30/70, 40/60, 50/50, 60/40, 70/30, 80/20, 90/10 pour des formulations telles que décrites ci-dessus de 40 à 800 UI/mL. Cependant toute autre proportion peut être réalisée.

Pour une formulation à 100 UI/mL en insuline totale, les proportions entre l'insuline basale dont le point isoélectrique est compris entre 5,8 et 8,5 et l'insuline prandiale sont par exemple en UI/mL de 25/75, 30/70, 40/60, 50/50, 60/40, 70/30, 80/20, 90/10. Cependant toute autre proportion peut être réalisée.

Dans un mode de réalisation, la composition selon l'invention contient en outre un GLP-1, un analogue ou un dérivé de GLP-1.

Dans un mode de réalisation, les analogues ou dérivés de GLP-1 sont choisis dans le groupe constitué par l'exenatide ou Byetta^{®}, développé par Eli Lilly & Co et Amylin Pharmaceuticals, le liraglutlde ou Victoza^{®} développé par Novo Nordisk, ou le llxisenatide ou Lyxumia^{®} développé par Sanofi, leurs analogues ou dérivés et leurs sels pharmaceutiquement acceptables.

Dans un mode de réalisation, l'analogue ou dérivé de GLP-1 est l'exenatide ou Byetta^{®} ses analogues ou dérivés et leurs sels pharmaceutiquement acceptables.

Dans un mode de réalisation, l'analogue ou dérivé de GLP-1 est le liraglutide ou Victoza^{®} ses analogues ou dérivés et leurs sels pharmaceutiquement acceptables.

Dans un mode de réalisation, l'analogue ou dérivé de GLP-1 est le lixisenatide ou Lyxumia^{®} ses analogues ou dérivés et leurs sels pharmaceutiquement acceptables.

On entend par « analogue », lorsqu'il est utilisé par référence à un peptide ou une protéine, un peptide ou une protéine dans lequel un ou plusieurs résidus d'acides aminés constitutifs ont été substitués par d'autres résidus d'acides aminés et/ou dans lequel un ou plusieurs résidus d'acides aminés constitutifs ont été supprimés et/ou dans lequel un ou plusieurs résidus d'acides aminés constitutifs ont été ajoutés. Le pourcentage d'homologie admis pour la présente définition d'un analogue est de 50 %.

On entend par « dérivé », lorsqu'il est utilisé par référence à un peptide ou une protéine, un peptide ou une protéines ou un analogue chimiquement modifié par un substituant qui n'est pas présent dans le peptide ou la protéine ou l'analogue de référence, c'est-à-dire un peptide ou une protéine qui a été modifié par création de liaisons covalentes, pour introduire des substituants.

Dans un mode de réalisation, la concentration en GLP-1, en analogue ou en dérivé de GLP-1 est comprise dans un intervalle de 0,01 à 10 mg/mL.

Dans un mode de réalisation, la concentration en exenatide, ses analogues ou dérivés et leurs sels pharmaceutiquement acceptables est comprise dans un intervalle de 0,05 à 0,5 mg/mL.

Dans un mode de réalisation, la concentration en liraglutide, ses analogues ou dérivés et leurs sels pharmaceutiquement acceptables est comprise dans un intervalle de 1 à 10 mg/mL.

Dans un mode de réalisation, la concentration en lixisenatide ses analogues ou dérivés et leurs sels pharmaceutiquement acceptables est comprise dans un intervalle de 0,01 à 1 mg/mL.

Dans un mode de réalisation, les compositions selon l'invention sont réalisées par mélange de solutions commerciales d'insuline basale dont le point isoélectrique est compris entre 5,8 et 8,5 et de solutions commerciales de GLP-1, d'analogue ou de dérivé de GLP-1 en ratios volumiques compris dans un intervalle de 10/90 à 90/10.

Dans un mode de réalisation, la composition selon l'invention comprend une dose journalière d'insuline basale et une dose journalière de GLP-1, d'analogue ou de dérivé de GLP-1.

Dans un mode de réalisation, les compositions selon l'invention comprennent 500 UI/mL d'insuline basale dont le point isoélectrique est compris entre 5,8 et 8,5 et de 0,05 à 0,5 mg/mL d'exenatide.

Dans un mode de réalisation, les compositions selon l'invention comprennent 500 UI/mL d'insuline basale dont le point isoélectrique est compris entre 5,8 et 8,5 et de 1 à 10 mg/mL de liraglutide.

Dans un mode de réalisation, les compositions selon l'invention comprennent 500 UI/mL d'insuline basale dont le point isoélectrique est compris entre 5,8 et 8,5 et de 0,05 à 0,5 mg/mL de lixisenatide.

Dans un mode de réalisation, les compositions selon l'invention comprennent 100 UI/mL d'insuline basale dont le point isoélectrique est compris entre 5,8 et 8,5 et de 0,05 à 0,5 mg/mL d'exenatide.

Dans un mode de réalisation, les compositions selon l'invention comprennent 100 UI/mL d'insuline basale dont le point isoélectrique est compris entre 5,8 et 8,5 et de 1 à 10 mg/mL de liraglutide.

Dans un mode de réalisation, les compositions selon l'invention comprennent 100 UI/mL d'insuline basale dont le point isoélectrique est compris entre 5,8 et 8,5 et de 0,05 à 0,5 mg/mL de lixisenatide.

Dans un mode de réalisation, les compositions selon l'invention comprennent 40 UI/mL d'insuline basale dont le point isoélectrique est compris entre 5,8 et 8,5 et de 0,05 à 0,5 mg/mL d'exenatide.

Dans un mode de réalisation, les compositions selon l'invention comprennent 40 UI/mL d'insuline basale dont le point isoélectrique est compris entre 5,8 et 8,5 et de 1 à 10 mg/mL de liraglutide.

Dans un mode de réalisation, les compositions selon l'invention comprennent 40 UI/mL d'insuline basale dont le point isoélectrique est compris entre 5,8 et 8,5 et de 0,05 à 0,5 mg/mL de lixisenatide.

Dans un mode de réalisation, les compositions selon invention comprennent 200 UI/mL d'insuline basale dont le point isoélectrique est compris entre 5,8 et 8,5 et de 0,05 à 0,5 mg/mL d'exenatide.

Dans un mode de réalisation, les compositions selon l'invention comprennent 200 UI/mL d'insuline basale dont le point isoélectrique est compris entre 5,8 et 8,5 et de 1 à 10 mg/mL de liraglutide.

Dans un mode de réalisation, les compositions selon l'invention comprennent 200 UI/mL d'insuline basale dont le point isoétectrique est compris entre 5,8 et 8,5 et de 0,05 à 0,5 mg/mL de lixisenatide.

Dans un mode de réalisation, les compositions selon l'invention comprennent en outre des sels de zinc à une concentration comprise entre 0 et 5000 µM.

Dans un mode de réalisation, les compositions selon l'invention comprennent en outre des sels de zinc à une concentration comprise entre 50 et 4000 µM.

Dans un mode de réalisation, les compositions selon l'invention comprennent en outre des sels de zinc à une concentration comprise entre 200 et 3000 µM.

Dans un mode de réalisation, les compositions selon l'invention comprennent en outre des sels de zinc à une concentration comprise entre 0 et 1000 µM.

Dans un mode de réalisation, les compositions selon l'invention comprennent en outre des sels de zinc à une concentration comprise entre 20 et 600 µM.

Dans un mode de réalisation, les compositions selon l'invention comprennent en outre des sels de zinc à une concentration comprise entre 50 et 500 µM.

Dans un mode de réalisation, les compositions selon l'invention comprennent des tampons choisis dans le groupe comprenant le Tris, les citrates et les phosphates à des concentrations comprises entre 0 et 100 mM, de préférence entre 0 et 50 mM ou entre 15 et 50 mM.

Dans un mode de réalisation, les compositions selon l'invention comprennent en outre des conservateurs.

Dans un mode de réalisation, les conservateurs sont choisis dans le groupe constitué par le m-crésol et le phénol seuls ou en mélange.

Dans un mode de réalisation, la concentration des conservateurs est comprise entre 10 et 50 mM.

Dans un mode de réalisation, la concentration des conservateurs est comprise entre 10 et 40 mM.

Les compositions selon l'invention peuvent en outre comprendre des additifs tels que des agents de tonicité comme la glycérine, NaCl, le mannitol et la glycine.

Les compositions selon l'invention peuvent en outre comprendre des additifs conformes aux pharmacopées comme des tensioactifs par exemple du polysorbate.

Les compositions selon l'invention peuvent comprendre en outre tous les excipients conformes aux pharmacopées et compatibles avec les insulines utilisées aux concentrations d'usage.

Dans un mode de réalisation, 0,3 ≤ n ≤ 1,7.

Dans un mode de réalisation, 0,7 ≤ n ≤ 1,5.

Dans un mode de réalisation, 0,9 ≤ n ≤ 1,2.

Dans un mode de réalisation, 0,01 ≤ m ≤ 0,5.

Dans un mode de réalisation, 0,02 ≤ m ≤ 0,4.

Dans un mode de réalisation, 0,03 ≤ m ≤ 0,3.

Dans un mode de réalisation, 0,05 ≤ m ≤ 0,2.

Dans un mode de réalisation, 3 ≤ q ≤ 50.

Dans un mode de réalisation, 3 ≤ q ≤ 40.

Dans un mode de réalisation, 3 ≤ q ≤ 30.

Dans un mode de réalisation, 3 ≤ q ≤ 20

Dans un mode de réalisation, 3 ≤ q ≤ 10.

Dans un mode de réalisation le radical -(*f*-[A]-COOH)ₙ est choisi dans le groupe constitué par les enchaînements suivants, f ayant la signification donnée ci-dessus :

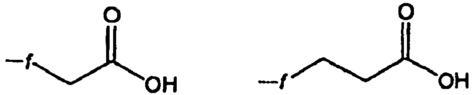

Dans un mode de réalisation le radical -(*g*-[B]-*k*-[D])ₘ est choisi dans le groupe constitué par les enchainements suivants ; *g, k* et D ayant les significations données ci-dessus :

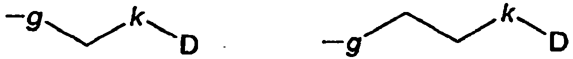

Dans un mode de réalisation, D est tel, que le radical X est un radical au moins divalent issu d'un acide aminé.

Dans un mode de réalisation, D est tel, que le radical X est un radical au moins divalent issu d'un acide aminé choisi dans le groupe constitué par la glycine, la leucine, la phénylalanine, la lysine, l'isoleucine, l'alanine, la valine, l'acide aspartique et l'acide glutamique.

Les radicaux issus d'acides aminés peuvent être soit lévogyre soit dextrogyre.

Dans un mode de réalisation, D est tel, que le radical X est un radical au moins divalent issu d'un mono- ou polyéthylène glycol.

Dans un mode de réalisation, D est tel, que le radical X est un radical au moins divalent issu de l'éthylène glycol.

Dans un mode de réalisation, D est tel, que le radical X est un radical au moins divalent issu d'un polyéthylène glycol choisi dans le groupe constitué par le diéthylène glycol et le triéthylène glycol.

Dans un mode de réalisation, D est tel, que le radical X est un radical au moins divalent issu d'un mono- ou polyéthylène glycol amine.

Dans un mode de réalisation, D est tel, que le radical X est un radical au moins divalent issu d'un mono- ou polyéthylène glycol amine choisi dans le groupe constitué par l'éthanolamine, le diéthylène glycol amine et le triéthylène glycol amine.

Dans un mode de réalisation, D est tel, que le radical X est un radical au moins divalent issu d'un mono- ou polyéthylène glycol diamine.

Dans un mode de réalisation, D est tel, que le radical X est un radical au moins divalent issu de l'éthylènediamine.

Dans un mode de réalisation, D est tel, que le radical X est un radical au moins divalent issu d'un mono- ou polyéthylène glycol diamine choisi dans le groupe constitué parle diéthylène glycol diamine et le triéthylène glycol diamine.

Dans un mode de réalisation, D est tel, que le groupe Y est un groupe alkyle issu d'un alcool hydrophobe.

Dans un mode de réalisation, D est tel, que le groupe Y est un groupe alkyle Issu d'un alcool hydrophobe, choisi dans le groupe constitué par l'octanol (alcool caprylique), le 3,7-diméthyloctan-1-ol, le décanol (alcool décylique), le dodécanol (alcool laurylique), le tétradécanol (alcool myristilique) et l'hexadécanol (alcool cétylique).

Dans un mode de réalisation, D est tel, que le groupe Y est un groupe alkyle issu d'un acide hydrophobe.

Dans un mode de réalisation, D est tel, que le groupe Y est un groupe alkyle issu d'un acide hydrophobe, choisi dans le groupe constitué par l'acide décanoïque, l'acide dodécanoïque, l'acide tétradécanoïque et l'acide hexadécanoïque.

Dans un mode de réalisation, D est tel, que le groupe Y est un groupe issu d'un stérol.

Dans un mode de réalisation, D est tel, que le groupe Y est un groupe issu d'un stérol, choisi dans le groupe constitué par le cholestérol et ses dérivés.

Dans un mode de réalisation, D est tel, que le groupe Y est un groupe issu d'un tocophérol.

Dans un mode de réalisation, D est tel, que le groupe Y est un groupe issu d'un dérivé du tocophérol, choisi parmi le racémique, l'isomère L ou l'isomère D de l'α-tocophérol.

Dans un mode de réalisation, D est tel, que le radical X est issu de la glycine, p = 1, le groupe Y est issu de l'octanol et la fonction *I* est une fonction ester.

Dans un mode de réalisation, D est tel, que le radical X est issu de la glycine, p = 1, le groupe Y est issu du dodécanol et la fonction *I* est une fonction ester.

Dans un mode de réalisation, D est tel, que le radical X est issu de la glycine, p = 1, le groupe Y est issu de l'hexadécanol et la fonction *I* est une fonction ester.

Dans un mode de réalisation, D est tel, que le radical X est issu, de la phénylalanine, p = 1, le groupe Y est issu de l'octanol et la fonction *I* est une fonction ester.

Dans un mode de réalisation, D est tel, que le radical X est issu de la phénylalanine, p = 1, le groupe Y est issu du 3,7-diméthyloctan-1-ol et la fonction 1 est une fonction ester.

Dans un mode de réalisation, D est tel, que le radical X est issu de l'acide aspartique, p = 2, les groupes Y sont issus de l'octanol et les fonctions *I* sont des fonctions esters.

Dans un mode de réalisation, D est tel, que le radical X est issu de l'acide aspartique, p = 2, les groupes Y sont issus du décanol et les fonctions *I* sont des fonctions esters.

Dans un mode de réalisation, D est tel, que le radical X est issu de l'acide aspartique, p = 2, les groupes Y sont issus du dodécanol et les fonctions *I* sont des fonctions esters.

Dans un mode de réalisation, D est tel, que le radical X est issu de l'éthylènediamine, le groupe Y est issu de l'acide dodécanoïque et la fonction *I* est une fonction amide.

Dans un mode de réalisation, D est tel, que le radical X est issu du diéthylène glycol amine, p = 1, le groupe Y est issu de l'acide dodécanoïque et la fonction *I* est une fonction ester.

Dans un mode de réalisation, D est tel, que le radical X est issu du triéthylène glycol diamine, p = 1, le groupe Y est issu de l'acide dodécanoïque et la fonction *I* est une fonction amide.

Dans un mode de réalisation, D est tel, que le radical X est issu du triéthylène glycol diamine, p = 1, le groupe Y est issu de l'acide hexadécanoique et la fonction *I* est une fonction amide.

Dans un mode de réalisation, D est tel, que le radical X est issu du de la leucine, p = 1, le groupe Y est issu du cholestérol et la fonction *I* est une fonction ester.

Dans un mode de réalisation, D est tel, que X est issu de l'éthylènediamine, p = 1, le groupe Y est issu du cholestérol et la fonction *I* est une fonction carbamate.

Dans un mode de réalisation, le radical E est un radical au moins divalent issu d'un acide aminé choisi dans le groupe constitué par la glycine, la leucine, la phénylalanine, la lysine, l'isoleucine, l'alanine, la valine, la sérine, la thréonine, l'acide aspartique et l'acide glutamique.

Les radicaux issus d'acides aminés peuvent être soit lévogyre soit dextrogyre.

Dans un mode de réalisation, le radical E est un radical au moins divalent issu d'un mono- ou polyéthylène glycol amine.

Dans un mode de réalisation, le radical E est un radical au moins divalent issu d'un mono- ou polyéthylène glycol amine choisi dans le groupe constitué par l'éthanolamine, le diéthylène glycol amine et le triéthylène glycol amine.

Dans un mode de réalisation, le radical E est un radical au moins divalent issu d'un mono- ou polyéthylène glycol diamine.

Dans un mode de réalisation, le radical E est un radical au moins divalent issu de l'éthylènediamine.

Dans un mode de réalisation, le radical E est un radical au moins divalent issu d'un mono- ou polyéthylène glycol diamine choisi dans le groupe constitué parle diéthylène glycol diamine et le triéthylène glycol diamine.

Dans un mode de réalisation, le groupe F est un groupe alkyle issu d'un alcool hydrophobe.

Dans un mode de réalisation, le groupe F est un groupe issu d'un alcool hydrophobe, choisi dans le groupe constitué par le dodécanol (alcool laurylique), le tétradécanol (alcool myristilique) et l'hexadécanol (alcool cétylique).

Dans un mode de réalisation, le groupe F est un groupe Issu d'un acide hydrophobe.

Dans un mode de réalisation, le groupe F est un groupe issu d'un acide hydrophobe, choisi dans le groupe constitué par l'acide dodécanoïque, l'acide tétradécanoïque et l'acide hexadécanoïque.

Dans un mode de réalisation, le groupe F est un groupe issu d'un stérol.

Dans un mode de réalisation, le groupe F est un groupe issu d'un stérol, choisi dans le groupe constitué par le cholestérol et ses dérivés.

Dans un mode de réalisation, le groupe F est un groupe issu d'un tocophérol.

Dans un mode de réalisation, le groupe F est un groupe Issu d'un dérivé du tocophérol, choisi parmi le racémique, l'isomère L ou l'isomère D de l'α-tocophérol.

Dans un mode de réalisation, le radical E est issu de l'éthylènediamine, t = 1, o est une fonction carbamate, et le groupe F est issu du cholestérol.

Dans un mode de réalisation :
∘ -(*f*-[A]-COOH)ₙ est tel que, A est le radical -CH₂- et f est une fonction éther ;
∘ -(*g*-[B]-*k*-[D])ₘ est tel que, *g* est une fonction éther, B est le radical -CH₂-, *k* est une fonction amide et D est tel, que X est issu de la glycine, *I* est une fonction ester et Y est issu de l'octanol ;
∘ q = 38, n = 0,9 et m = 0,2.

Dans un mode de réalisation :
∘ (*f*-[A]-COOH)ₙ est tel que, A est le radical -CH₂- et *f* est une fonction éther ;
∘ -(*g*-[B]-*k*-[D])ₘ est tel que, *g* est une fonction éther, B est le radical -CH₂, *k* est une fonction amide et D est tel, que X est issu de la glycine, p = 1, *I* est une fonction ester et Y est issu de l'hexadécanol ;
∘ q = 19, n = 1,0 et m = 0,1.

Dans un mode de réalisation :
∘ -(*f*-(A)-COOH)ₙ est tel que, A est le radical -CH₂- et *f* est une fonction éther ;
∘ -(*g*-[B]-*k*-[D])ₘ est tel que, *g* est une fonction éther, B est le radical -CH₂-, *k* est une fonction amide et D est tel, que X est issu de la phénylalanine, p = 1, *I* est une fonction ester et Y est issu de l'octanol ;
∘ q = 38, n = 1,0 et m = 0,1.

Dans un mode de réalisation :
∘ -(*f*-[A]-COOH)ₙ est tel que, A est le radical -CH₂- et *f* est une fonction éther ;
∘ -(*g*-[B]-*k*-[D])ₘ est tel que, *g* est une fonction éther, B est le radical -CH₂-, *k* est une fonction amide et D est tel, que X est issu de la phénylalanine, p = 1, *I* est une fonction ester et Y est issu de l'octanol ;
∘ q = 19, n = 1,0 et m = 0,2.

Dans un mode de réalisation :
o -(*f*-[A]-COOH)ₙ est tel que, A est le radical -CH₂- et *f* est une fonction éther ;
o -(*g*-[B]-k-[D])ₘ est tel que, *g* est une fonction éther, B est le radical -CH₂-, *k* est une fonction amide et D est tel, que X est issu de la phénylalanine, p = 1, *l* est une fonction ester et Y est issu du 3,7-diméthyloctan-1-ol ;
o q = 38, n = 1,0 et m = 0,1.

Dans un mode de réalisation :
o -(*f-*[A]-COOH)ₙ est tel que, A est le radical -CH₂- et *f* est une fonction éther ;
o -(*g*-[B]-k-[O])ₘ est tel que, *g* est une fonction éther, B est le radical -CH₂-, *k* est une fonction amide et D est tel, que X est issu de l'acide aspartique, p = 2, *l* sont des fonctions esters et Y sont issus de l'octanol ;
o q = 38, n = 1,05 et m = 0,05.

Dans un mode de réalisation :
o -(*f-*[A]-COOH)ₙ est tel que, A est le radical -CH₂- et *f* est une fonction éther ;
o -(*g-*[B]-*k*-[D])ₘ est tel que, *g* est une fonction éther, B est le radical -CH₂-, *k* est une fonction amide et D est tel, que X est issu de l'acide aspartique, p = 2, *l* sont des fonctions esters et Y sont issus du décanol ;
o q = 38, n = 1,05 et m = 0,05.

Dans un mode de réalisation :
o -(*f*-[A]-COOH)ₙ est tel que, A est le radical -CH₂- et *f* est une fonction éther ;
o -(*g*-[B]-*k*-[D])ₘ est tel que, *g* est une fonction éther, B est le radical -CH₂-, *k* est une fonction amide et D est tel, que X est Issu de l'acide aspartique, p = 2, *l* sont des fonctions esters et Y sont issus du dodécanol ;
o q = 19, n = 1,05 et m = 0,05.

Dans un mode de réalisation :
o -(*f*-[A]-COOH)ₙ est tel que, A est le radical -CH₂- et *f* est une fonction éther ;
o -(*g*-[B]-*k*-[D])ₘ est tel que, *g* est une fonction éther, B est le radical -CH₂-, *k* est une fonction amide et D est tel, que X est issu de l'éthylènediamine, p = 1, *l* est une fonction amide et Y est issu de l'acide dodécanoïque ;
o q = 38, n = 1,0 et m = 0,1.

Dans un mode de réalisation :
o -(*f*-[A]-COOH)ₙ est tel que, A est le radical -CH₂-CH₂- et *f* est une fonction ester ;
o -(*g*-[8]-*k*-[D])ₘ est tel que, *g* est une fonction ester, B est le radical -CH₂-CH₂-, *k* est une fonction amide et D est tel, que X est Issu de la glycine, p = 1, *l* est une fonction ester et Y est issu du dodécanol ;
o q = 38, n = 1,3 et m = 0,1.

Dans un mode de réalisation :
o -(*f*-[A]-COOH)ₙ est tel que, A est le radical -CH₂- et *f* est une fonction carbamate ;
o -(*g*-[B]-k-[D])ₘ est tel que, *g* est une fonction carbamate, B est le radical -CH₂-, *k* est une fonction amide et D est tel, que X est issu de l'acide aspartique, p = 2, *l* sont des fonctions esters et Y sont issus de l'octanol ;
o q = 38, n = 1,3 et m = 0,1.

Dans un mode de réalisation :
o -(*f*-[A]-COOH)ₙ est tel que, A est le radical -CH₂- et *f* est une fonction éther ;
o -(*g*-[B]-*k*-[D])ₘ est tel que, *g* est une fonction éther, B est le radical -CH₂-, *k* est une fonction amide et D est tel, que X est issu de l'acide aspartique, p = 2, *l* sont des fonctions esters et Y sont issus du dodécanol ;
o q = 4, n = 0,96 et m = 0,07.

Dans un mode de réalisation :
o -(*f*-[A]-COOH)ₙ est tel que, A est le radical -CH₂- et *f* est une fonction éther ;
o -(*g*-[B]-k-[D])ₘ est tel que, *g* est une fonction éther, B est le radical -CH₂-, *k* est une fonction amide et D est tel, que X est issu du diéthylène glycol amine, p = 1, *l* est une fonction ester et Y est issu de l'acide dodécanoĩque ;
o q = 38, n = 1,0 et m = 0,1.

Dans un mode de réalisation :
o -(*f*-[A]-COOH)ₙ est tel que, A est le radical -CH₂- et *f* est une fonction éther ;
o -(*g*-[BJ]-*k*-[D])ₘ est tel que, *g* est une fonction éther, B est le radical -CH₂-, *k* est une fonction amide et D est tel, que X est issu du triéthylène glycol diamine, p = 1, *l* est une fonction amide et Y est issu de l'acide dodécaoĩque :
o q = 38, n = 1,0 et m = 0,1.

Dans un mode de réalisation :
o -(*f* [A]-COOH)ₙ est tel que, A est le radical -CH₂- et *f* est une fonction éther ;
o -(*g*-[B]-*k*-[D])ₘ est tel que, *g* est une fonction éther, B est le radical -CH₂-, *k* est une fonction amide et D est tel, que X est issu triéthylène glycol diamine, p = 1, / est une fonction amide et Y est issu de l'acide hexadécanoïque ;
o q = 38, n = 1,05 et m = 0,05.

Dans un mode de réalisation :
o -(*f*-[A]-COOH)ₙ est tel que, A est le radical -CH₂- et *f* est une fonction éther ;
o -(*g*-[B]-*k*-[D])ₘ est tel que, *g* est une fonction éther, B est le radical -CH₂-, *k* est une fonction amide et D est tel, que X est issu de la glycine, p = 1, *l* est une fonction ester et Y est issu de l'hexadécanol ;
o q = 19, n = 1,05 et m = 0,05.

Dans un mode de réalisation :
o -(*f-*[A]-COOH)ₙ est tel que, A est le radical -CH₂- et *f* est une fonction éther ;
o -(*g*-[B]-*k*-[D])n, est tel que, *g* est une fonction éther, B est le radical -CH₂-, *k* est une fonction amide et D est tel, que X est issu de la glycine, p = 1, *l* est une fonction ester et Y est issu de l'hexadécanol ;
o q = 38, n = 0,37 et m = 0,05.

Dans un mode de réalisation :
o -(*f*-[A]-COOH)ₙ est tel que, A est le radical -CH₂- et *f* est une fonction éther ;
o -(*g*-[B]-*k*-[D])ₘ est tel que, *g* est une fonction éther, B est le radical -CH₂-, *k* est une fonction amide et D est tel, que X est issu de la leucine, p = 1, *l* est une fonction ester et Y est issu du cholestérol ;
o q = 19, n = 1,61 et m = 0,04.

Dans un mode de réalisation :
o -(*f-*[A]-COOH)ₙ est tel que, A est le radical -CH₂- et *f* est une fonction éther ;
o -(*g*-[B)-*k*-[D])ₘ est tel que, *g* est une fonction éther, B est le radical -CH₂-, *k* est une fonction amide et D est tel, que X est issu de la leucine, p = 1, *l* est une fonction ester et Y est issu du cholestérol ;
o q = 19, n = 1,06 et m = 0,04.

Dans un mode de réalisation :
o -(*f*-[A]-COOH)ₙ est tel que, A est le radical -CH₂- et *f* est une fonction éther ;
o -(*g*-[B]-*k*-[D])ₘ est tel que, *g* est une fonction éther, B est le radical -CH₂-, *k* est une fonction amide et D est tel, que X est Issu de la leucine, p = 1, *l* est une fonction ester et Y est issu du cholestérol ;
o q = 19, n = 0,66 et m = 0,04.

Dans un mode de réalisation :
o -(*f*-[A]-COOH)ₙ est tel que, A est le radical -CH₂- et *f* est une fonction éther ;
o -(*g*-[B]-*k*-[D])ₘ est tel que, *g* est une fonction éther, B est le radical -CH₂-, *k* est une fonction amide et D est tel, que X est issu de la leucine, p = 1, *l* est une fonction ester et Y est issu du cholestérol ;
o q = 19, n = 0,46 et m = 0,04.

Dans un mode de réalisation :
o -(*f*-[A]-COOH)ₙ est tel que, A est le radical -CH₂- et *f* est une fonction éther ;
o -(*g*-[B]-*k*-[D])ₘ est tel que, *g* est une fonction éther, B est le radical -CH₂-, *k* est une fonction amide et D est tel, que X est issu de la leucine, p = 1, *l* est une fonction ester et Y est issu du cholestérol;
o q = 4, n = 1,61 et m = 0,05.

Dans un mode de réalisation :
o -(*f*-[A]-COOH)ₙ est tel que, A est le radical -CH₂- et *f* est une fonction éther ;
o -(*g*-[B-*k*-D])ₘ est tel que, *g* est une fonction éther, B est le radical -CH₂-, *k* est une fonction amide et D est tel, que X est issu de l'éthylènediamine, p = 1, / est une fonction carbamate et Y est issu du cholestérol ;
o q = 19, n = 1,61 et m = 0,04.

Dans un mode de réalisation :
o -(*f-*[A]-COOH)ₙ est tel que, A est le radical -CH₂- et *f* est une fonction carbamate ;
o -(*g*-[B]-*k*-[D])ₘ est tel que, *g* est une fonction carbamate, B est le radical-CH₂-, *k* est une fonction amide et D est tel, que X est issu de la leucine, p = 1, *l* est une fonction ester et Y est issu du cholestérol ;
o q = 19, n = 1,96 et m = 0,04.

Dans un mode de réalisation :
o -(*f*-[A]-COOH)ₙ est tel que, A est le radical -CH₂- et *f* est une fonction éther ;
o -[E]-(*o*-[F])ₜ est tel que, E est Issu de l'éthylènediamine, *o* est une fonction carbamate et F est Issu du cholestérol ;
o q = 19 et = 1,65.

Dans un mode de réalisation :
o -(*f*-[A]-COOH), est tel que, A est le radical -CH₂- et *f* est une fonction éther ;
o -(*g*-[B]-*k*-[D])ₘ est tel que, *g* est une fonction éther, B est le radical -CH₂-, *k* est une fonction amide et D est tel, que X est issu de la leucine, p = 1, *l* est une fonction ester et Y est issu du cholestérol ;
o q = 38, n = 0,99 et m = 0,05.

Dans un mode de réalisation :
o -(*f*-[A]-COOH)ₙ est tel que, A est le radical -CH₂- et *f* est une fonction éther ;
o -(*g*-[B]-*k*-[D])ₘ est tel que, *g* est une fonction éther, B est le radical -CH₂-, *k* est une fonction amide et D est tel, que X est issu de l'acide aspartique, p = 2, / sont des fonctions esters et Y sont issus du dodécanol ;
o q = 4, n = 1,41 et m = 0,16.

Dans un mode de réalisation :
o -(*f-*[A]-COOH)ₙ est tel que, A est le radical -CH₂- et *f* est une fonction éther ;
o -(*g*-[B]-*k*-[D])ₘ est tel que, *g* est une fonction éther, B est le radical -CH₂-, *k* est une fonction amide et D est tel, que X est issu de l'acide aspartique, p = 2, / sont des fonctions esters et Y sont issus du dodécanol ;
o q = 4, n = 1,50 et m = 0,07.

Dans un mode de réalisation :
o -(*f*-[A]-COOH)ₙ est tel que, A est le radical -CH₂- et *f* est une fonction éther ;
o -(*g*-[B]-*k*-[D])ₘ est tel que, *g* est une fonction éther, B est le radical -CH₂-, *k* est une fonction amide et D est tel, que X est issu de l'acide aspartique, p = 2, *l* sont des fonctions esters et Y sont Issus du décanol ;
o q = 4, n = 1,05 et m = 0,05.

Dans un mode de réalisation les compositions selon l'invention comprenne un dextrane choisi dans le groupe constitué par les dextranes de formule I, III: ou IV suivants :
- Dextraneméthylcarboxylate de sodium modifié par le glycinate d'octyle,
- Dextraneméthylcarboxylate de sodium modifié par le glycinate de cétyle,
- Dextraneméthylcarboxylate de sodium modifié par le phénylalaninate d'octyle,
- Dextraneméthylcarboxylate de sodium modifié par le phénylalaninate de 3,7-diméthyle-1-octyle,
- Dextraneméthylcarboxylate de sodium modifié par l'aspartate de dioctyle,
- Dextraneméthylcarboxylate de sodium modifié par l'aspartate de didécyle,
- Dextraneméthylcarboxylate de sodium modifié par l'aspartate de dilauryle,
- Dextraneméthylcarboxylate de sodium modifié par le N-(2-aminoéthyl)dodécanamide,
- Dextranesuccinate de sodium modifié par le glycinate de lauryle,
- N-méthylcarboxylate de sodium dextrane carbamate modifié par l'aspartate de dioctyle,
- Dextraneméthylcarboxylate de sodium modifié par l'aspartate de dilauryle,
- Dextraneméthylcarboxylate de sodium modifié par le 2-(2-amino-éthoxy)éthyle dodécanoate,
- Dextraneméthylcarboxylate de sodium modifié par le 2-(2-{2-[dodécanoylamino]éthoxy}éthoxy)éthylamine,
- Dextraneméthylcarboxylate de sodium modifié par le 2-(2-{2-[hexadécanoylamino]éthoxy}éthoxy)éthytamine,
- Dextraneméthylcarboxylate de sodium modifié par le leucinate de cholestéryle,
- Dextraneméthylcarboxylate de sodium modifié par le 1-éthylènediaminecarboxylate de cholestéryle,
- N-méthylcarboxylate de sodium dextrane carbamate modifié par le leucinate de cholestéryle.

Dans un mode de réalisation les compositions selon l'invention comprenne un dextrane choisi dans le groupe constitué par le dextrane de formule II suivant :
- Dextraneméthylcarboxylate de sodium modifié par le 1-éthylènediaminecarboxylate de cholestéryle greffé par amination réductrice sur le bout de chaîne réducteur.

L'invention concerne également des formulations unidoses à pH compris entre 6,6 et 7,8 comprenant une insuline basale dont le point isoélectrique est compris entre 5,8 et 8,5 et une insuline prandiale.

L'invention concerne également des formulations unidoses à pH compris entre 7 et 7,8 comprenant une insuline basale dont le point isoélectrique est compris entre 5,8 et 8,5 et une insuline prandiale.

Dans un mode de réalisation, l'insuline basale dont le point isoélectrique est compris entre 5,8 et 8,5 est l'insuline glargine.

Dans un mode de réalisation, l'insuline prandiale est choisie dans le groupe comprenant Humulin^{®} (Insuline humaine) et Novolin^{®} (insuline humaine).

Dans un mode de réalisation, l'insuline prandiale est choisie dans le groupe comprenant l'insuline lispro (Humalog^{®}), l'insuline glulisine (Apidra^{®}) et l'insuline aspart (Novolog^{®}) .

La solubilisation à pH compris entre 6,6 et 7,8 des insulines basales dont le point isoélectrique est compris entre 5,8 et 8,5, par les polysaccharides de formule I, II, III ou IV, peut être constatée et contrôlée de manière simple, à l'oeil nu, grâce à un changement d'aspect de la solution

La solubilisation à pH compris entre 7 et 7,8 des insulines basales dont le point isoélectrique est compris entre 5,8 et 8,5, par les polysaccharides de formule 1, II, III ou IV, peut être constatée et contrôlée de manière simple, à l'oeil nu, grâce à un changement d'aspect de la solution.

Par ailleurs et de façon toute aussi importante, la demanderesse a pu vérifier qu'une insuline basale dont le point isoélectrique est compris entre 5,8 et 8,5, solubilisée en présence d'un polysaccharide de formule I, II, III ou IV n'avait en rien perdu son action d'insuline lente.

La préparation d'une composition selon l'invention présente l'avantage de pouvoir être réalisée par simple mélange d'une solution aqueuse d'insuline basale dont le point isoélectrique est compris entre 5,8 et 8,5, d'une solution de d'insuline prandiale, et d'un polysaccharide de formule I, II, III ou IV, en solution aqueuse ou sous forme lyophilisée. Si nécessaire, le pH de la préparation est ajusté à pH 7.

La préparation d'une composition selon l'invention présente l'avantage de pouvoir être réalisée par simple mélange d'une solution aqueuse d'insuline basale dont le point isoélectrique est compris entre 5,8 et 8,5, d'un polysaccharide de formule I, II, III ou IV en solution aqueuse ou sous forme lyophilisée, et d'une Insuline prandiale en solution aqueuse ou sous forme lyophilisée.

Dans un mode de réalisation le mélange d'insuline basale et de polysaccharide est concentré par ultrafiltration avant le mélange avec l'insuline prandiale en solution aqueuse ou sous forme lyophilisée.

Si nécessaire, la composition du mélange est ajustée en excipients tels que glycérine, m-crésol, chlorure de zinc, et tween par ajout de solutions concentrées de ces excipients au sein du mélange. Si nécessaire, le pH de la préparation est ajusté à 7.

### Description des figures :

Les figures 1 à 6 présentent les résultats obtenus sous forme de courbes de pharmacodynamie du glucose. L'axe des ordonnées représente le Dglucose (exprimé en mM) en fonction du temps post injection (exprimé en minutes).
Figure 1 : Courbes Moyenne + écart-type de la moyenne pour les administrations séquentielles de Apidra^{®} et Lantus^{®} (□) en comparaison avec une composition selon l'invention Polysaccharide 4/Lantus^{®}/Apldra^{®} (75/25) (•).
Figure 2 : Courbes individuelles Apidra^{®} Lantus^{®} (testé sur 6 porcs).
Figure 3 : Courbes Individuelles Polysaccharide 4/Apldra^{®}/Lantus^{®} (testé sur 6 porcs).
Figure 4 : Courbes Moyenne + écart-type de la moyenne pour les administrations séquentielles de Humalog^{®} et Lantus^{®} (o) en comparaison avec l'administration d'une composition selon l'invention Polysaccharide 4/Humalog^{®}/lantus^{®} (•).
Figure 5 : Courbes individuelles Humalog^{®} Lantue^{®} (testé sur 6 porcs).
Figure 6 : Courbes individuelles Polysaccharide 4/Humalog^{®}/Lantus^{®} (testé sur 5 porcs).
Les figures 7 à 12 présentent les résultats obtenus sous forme de courbes de pharmacodynamie du glucose. L'axe des ordonnées représente le Dglucose (exprimé en mM) en fonction du temps post injection (exprimé en heures).

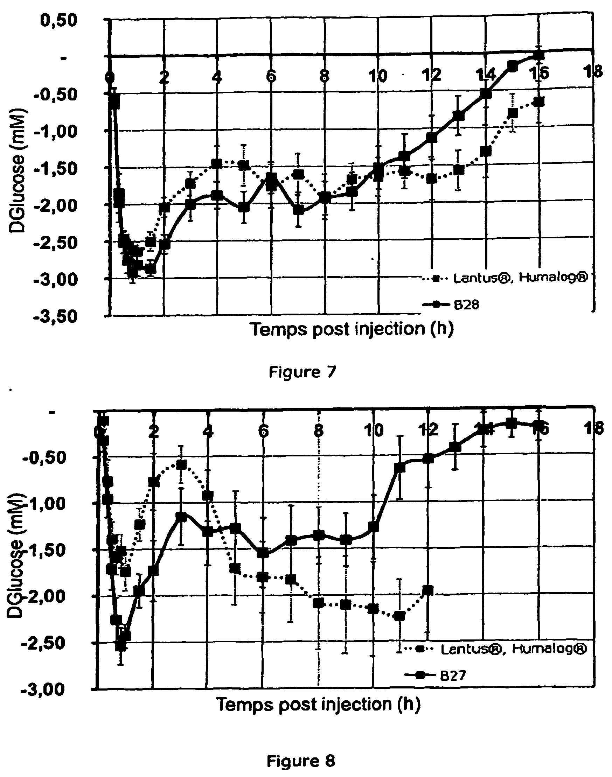

Figure 7 : Courbes Moyenne + écart-type de la moyenne pour les administrations séquentielles de Humalog^{®} (100 UI/ml, 0,13 UI/kg) et Lantus^{®} (100 UI/ml, 0,4 UI/kg) (··■··) en comparaison avec une composition selon l'invention décrite dans l'exemple B28 (0,53 UI/kg) (-■-).
Figure 8 : Courbes Moyenne + écart-type de la moyenne pour les administrations séquentielles de Humalog^{®} (100 UI/ml, 0,13 UI/kg) et Lantus^{®} (100 UI/ml, 0,4 UI/kg) (··■··) en comparaison avec une composition selon l'invention décrite dans l'exemple B27 (0,47 UI/kg) (-■-).

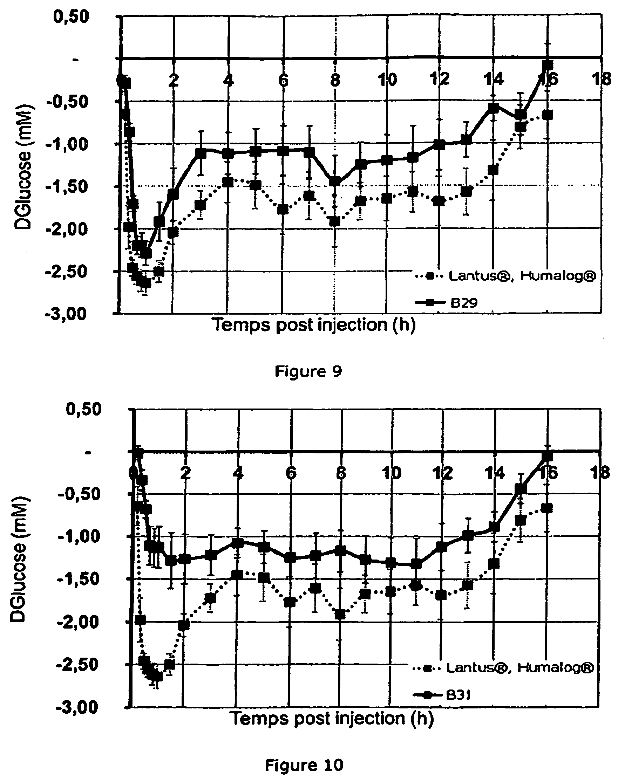

Figure 9 : Courbes Moyenne + écart-type de la moyenne pour les administrations séquentielles de Humalog^{®} (100 UI/ml, 0,13 UI/kg) et Lantus^{®} (100 UI/ml, 0,4 UI/kg) (··■··) en comparaison avec une composition selon l'inventiondécrite dans l'exemple B29 (0,53 UI/kg) (-■-)_
Figure 10 : Courbes Moyenne + écart-type de la moyenne pour les administrations séquentielles de Humalog^{®} (100 UI/ml, 0,13 UI/kg) et Lantus^{®} (100 UI/ml, 0,4 UI/kg) (··■··) en comparaison avec une composition selon l'invention décrite dans l'exemple B31 (0,48 UI/kg) (-■-).

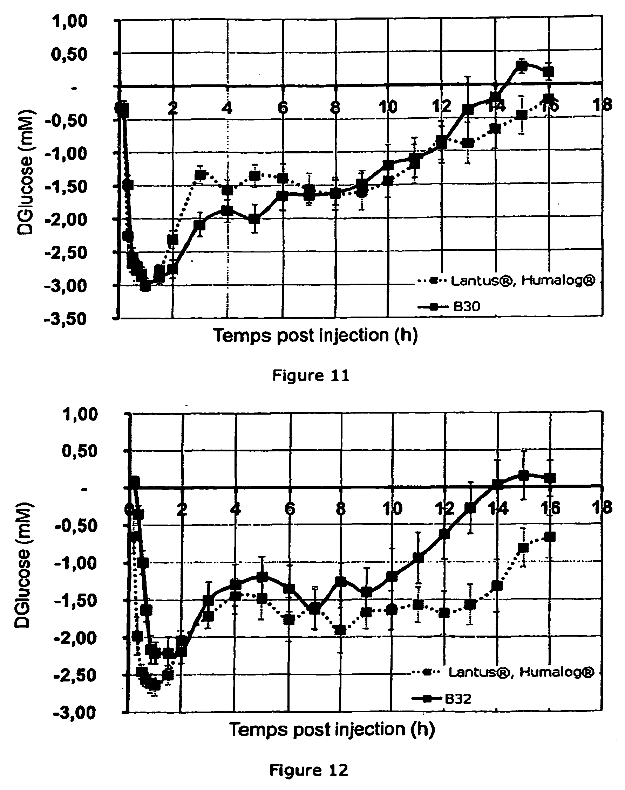

Figure 11 : Courbes Moyenne + écart-type de la moyenne pour les administrations séquentielles de Humalog^{®} (100 UI/ml, 0,24 UI/kg) et Lantus^{®} (100 UI/ml, 0,4 UI/kg) (··■··) en comparaison avec une composition selon l'Invention décrite dans l'exemple 830 (0,64 UI/kg) (-■-).
Figure 12 : Courbes Moyenne + écart-type de la moyenne pour les administrations séquentielles de Humalog^{®} (100 UI/ml, 0,13 UI/kg) et Lantus^{®} (100 UI/ml, 0,4 UI/kg) (··■··) en comparaison avec une composition selon l'invention décrite dans l'exemple B32 (0,53 UI/kg) (-■-).

### Exemples

### Partie A Polysaccharides

Le Tableau 1 ci-après présente, de façon non limitative, des exemples de polysaccharides utilisables dans les compositions selon l'invention.

**Tableau 1**

| POLYSACCARIDES | SUBSTITUANTS | NOM USUEL |
|---|---|---|
| | -*f*-A-COONa -*g*-B-k-D | |
| **Polysaccharide 1** | 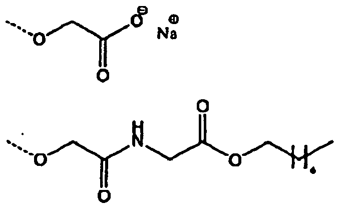 | Dextraneméthylcarboxylate de sodium modifié par le glycinate d'octyle |
| q : 38 | | |
| n : 0,9 | | |
| m: 0,2 | | |
| **Polysaccharide 2** | | |
| q : 19 | | |
| n : 1,0 | | |
| m : 0,1 | 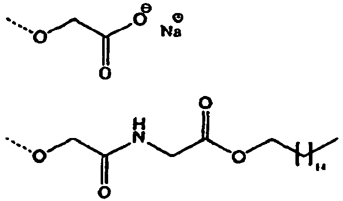 | |
| **Polysaccharide 16** | | Dextraneméthylcarboxylate de sodium modifié par le glycinate de cétyle |
| q : 19 | | |
| n : 1,05 | | |
| m : 0,05 | | |
| **Polysaccharide 17** | | |
| q : 38 | | |
| n : 0,37 | | |
| m : 0.05 | | |
| **Polysaccharide 3** | 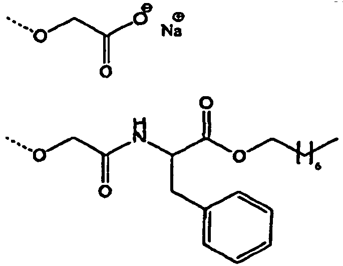 | |
| q : 38 | | |
| n : 1,0 | | Dextraneméthylcarboxylate de sodium modifié par le phénylalanlnate d'octyle |
| m : 0,1 | | |
| **polysaccharide 4** | | |
| q : 19 | | |
| z : 1, 0 | | |
| m : 0,2 | | |
| **Polysaccharide 5** | 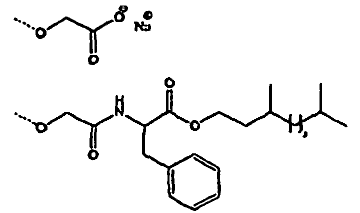 | Dextraneméthylcarboxylate de sodium modifié par le phénylalaninate de 3,7-diméthyle-1-octyle |
| q : 38 | | |
| n : 1,0 | | |
| m : 0,1 | | |
| **Polysaccharide 6** | 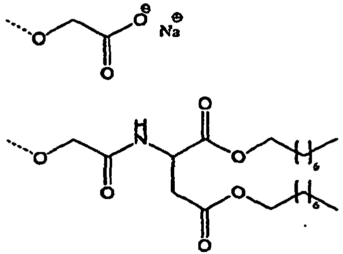 | Dextraneméthylcarboxylate de sodium modifié par l'aspartate de dioctyle |
| q : 38 | | |
| n : 1,05 | | |
| m : 0,05 | | |
| **Polysaccharide 7** | 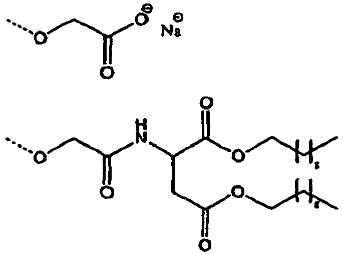 | |
| q : 38 | | |
| n : 1,05 | | Dextraneméthylcarboxytate de sodium modifié par l'aspartate de didécyle |
| m : 0,05 | | |
| **Polysaccharide 29** | | |
| q : 4 | | |
| n : 1,05 | | |
| m : 0,05 | | |
| **Polysaccharide 8** | | |
| q : 19 | | |
| n : 1,05 | 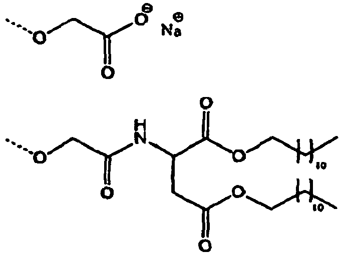 | |
| m : 0,05 | | Dextraneméthylcarboxylate de sodium modifié par l'aspartate de dilauryle |
| **Polysaccharide 27** | | |
| q:4 | | |
| n : 1,41 | | |
| m : 0,16 | | |
| **Polysaccharide 28** | | |
| q : 4 | | |
| n : 1,50 | | |
| m : 0,07 | | |
| **Polysaccharide 9** | 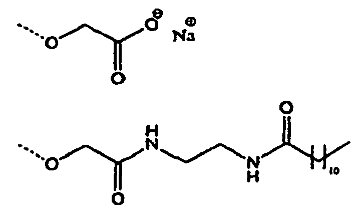 | Dextraneméthylcarboxylate de sodium modifié par le N-(2-aminoéthyl)dodécanamide |
| q : 38 | | |
| n : 1,0 | | |
| m : 0,1 | | |
| **Polysaccharide 10** | 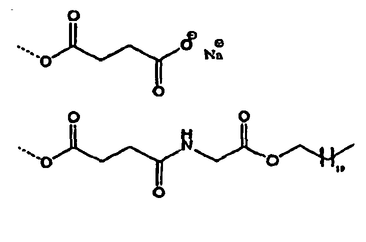 | Dextranesuccinate de sodium modifié par le glycinate de lauryle |
| q : 38 | | |
| n : 1,3 | | |
| m : 0,1 | | |
| **Polysaccharide 11** | 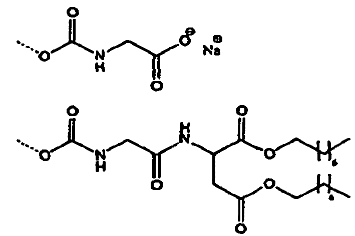 | N-méthylcarboxylate de sodium dextrane carbamate modifié par l'aspartate de dioctyle |
| q : 38 | | |
| n : 1,3 | | |
| m : 0,1 | | |
| **Polysaccharide 12** | 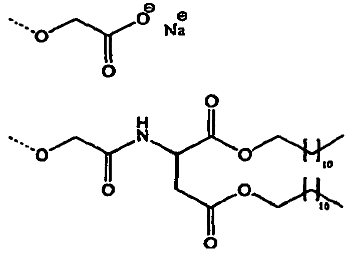 | Dextraneméthylcarboxylate de sodium modifié par l'aspartate de dilauryle |
| q : 4 | | |
| n : 0,96 | | |
| m : 0,07 | | |
| **[Polysaccharide 13** | 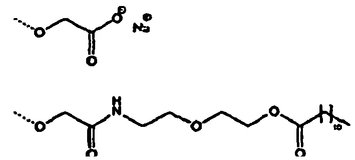 | Dextraneméthylcarboxylate de sodium modifié par le 2-(2-amino-éthoxy)éthyle dodécanoate |
| q : 38 | | |
| n : 1,0 | | |
| m : 0,1 | | |
| **Polysaccharide 14** | 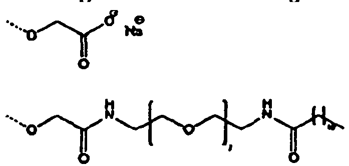 | Dextraneméthylcarboxylate de sodium modifié par le 2-(2-{2-[dodécanoylamino]éthoxy}éth oxy)éthylamine |
| q : 38 | | |
| n : 1,0 | | |
| m : 0,1 | | |
| **Polysaccharide 15** | 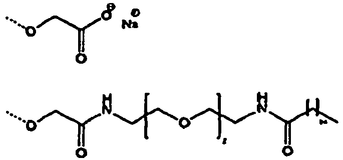 | Dextraneméthylcarboxylate de sodium modifié par le 2-(2-{2-[hexadécanoylamino]éthoxy}é thoxy)éthylamine |
| q : 38 | | |
| n : 1,05 | | |
| m : 0,05 | | |
| **Polysaccharide 18** | | |
| q : 19 | | |
| n : 1,61 | | |
| m : 0,04 | | |
| **Polysaccharide 19** | | |
| q : 19 | | |
| **n : 1,06** | | |
| **m :** 0,04 | | |
| **Polysaccharide 20** | | |
| q : 19 | 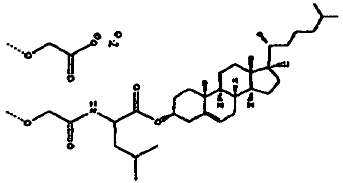 | |
| n : 0,66 | | |
| m : 0,04 | | Dextraneméthylcarboxylate de sodium modifié par le leucinate de cholestéryle |
| **Polysaccharide 21** | | |
| q : 19 | | |
| n : 0,46 | | |
| m : 0,04 | | |
| **Polysaccharide** 22 | | |
| q : 4 | | |
| n : 1,61 | | |
| m : 0,04 | | |
| **Polysaccharide 26** | | |
| q : 38 | | |
| n : 0,99 | | |
| m : 0,05 | | |
| **[Polysaccharide 23** | 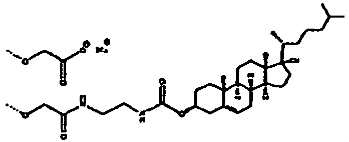 | Dextraneméthylcarboxylate de sodium modifié par le 1-éthylènediaminecarboxylate de cholestéryle |
| q : 19 | | |
| n : 1,61 | | |
| m : 0,04 | | |
| **Polysaccharide 24** | 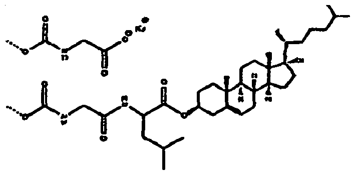 | N-méthylcarboxylate de sodium dextrane carbamate modifié par le leudnate de cholestéryle |
| q : 19 | | |
| n : 1,96 | | |
| m : 0,04 | | |

| POLYSACCHARIDES | SUBSTITUANTS | NOM USUEL |
|---|---|---|
| | -*f*-A-COONa -[E]-*o*-[F] | |
| **Polysaccharide 25** | 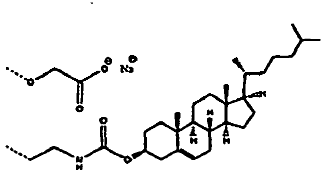 | Dextraneméthylcarboxylate de sodium modifié par le 1-éthylènediaminecarboxylate de cholestéryte greffé par amination réductrice sur le bout de chaîne réducteur |
| q : 19 | | |
| n : 1,65 | | |

### Exemple A1 : Préparation du Polysaccharide 1

16 g (soit 296 mmol d'hydroxyles) de dextrane de masse molaire moyenne en poids d'environ 10 kg/mol (q = 38, PHARMACOSMOS) sont solubilisés dans de l'eau à 420 g/L. A cette solution sont ajoutés 30 mL de NaOH 10 N (296 mmol). Le mélange est porté à 35°C, puis 46 g (396 mmol) de chloroacétate de sodium sont ajoutés. La température du milieu réactionnel est portée à 60°C à 0,5°C/min, puis maintenue à 60°C pendant 100 minutes. Le milieu réactionnel est dilué avec 200 mL d'eau, neutralisé à l'acide acétique et purifié par ultrafiltration sur membrane PES de 5 kDa contre 6 volumes d'eau. La solution finale est dosée par extrait sec pour déterminer la concentration en polysaccharide ; puis dosée par dosage adde/base dans de l'eau/acétone 50/50 (V/V) pour déterminer le nombre moyen de motifs méthylcarboxylates par unité glucosidique.

D'après l'extrait sec : [polysaccharide] = 31,5 mg/g
D'après le dosage acide/base : le nombre moyen de motifs méthylcarboxylates par unité glucosidique est de 1,1.

La solution de dextraneméthylcarboxylate de sodium est passée sur une résine Purolite (anlonique) pour obtenir le dextraneméthylcarboxylique acide qui est ensuite lyophilisé pendant 18 heures.

Le glycinate d'octyle, sel d'adde paratoluènesulfonique est obtenu selon le procédé décrit dans le brevet US 4,826,818.

10 g de dextraneméthylcarboxylique acide (44,86 mmol méthylcarboxylique acide) sont solubilisés dans le DMF à 60 g/L puis refroidis à 0°C. 3,23 g de glycinate d'octyle, sel d'acide paratoluènesulfonique (8,97 mmol) est mis en suspension dans du DMF à 100 g/L. 0,91 g de triéthylamine (8,97 mmol) est ensuite ajouté à cette suspension. Une fois la solution de polysaccharide à 0°C, une solution de NMM (5,24 g, 51,8 mmol) dans le DMF (530 g/L) et 5,62 g (51,8 mmol) de EtOCOCl sont ensuite ajoutés. Après 10 minutes de réaction, la suspension glycinate d'octyle est ajoutée. Le milieu est ensuite maintenu à 10°C pendant 45 minutes. Le milieu est ensuite chauffé à 30°C. Une solution d'imidazole (10,38 g dans 17 mL d'eau) et 52 mL d'eau sont ajoutés dans le milieu réactionnel. La solution de polysaccharide est ultrafiltrée sur membrane PES 10 kDa contre 15 volumes de solution NaCl 0,9 % et 5 volumes d'eau. La concentration de la solution de polysaccharide est déterminée par extrait sec. Une fraction de solution est lyophilisée et analysée par RMN ¹H dans D₂O pour déterminer le degré de substitution des méthylcarboxylate en glycinate d'octyle par unité glucosidique.

D'après l'extrait sec : [Polysaccharide 1] = 36,4 mg/g
D'après le dosage acide/base : n = 0,9
D'après la RMN ¹H : m = 0,2.

### Exemple A2 : Préparation du Polysaccharide 2

Le glycinate de cétyle, sel d'acide paratoluènesulfonique est obtenu selon le procédé décrit dans le brevet US 4,826,818.

Par un procédé similaire à celui décrit à l'Exemple A1, un dextraneméthylcarboxylate de sodium, synthétisé selon le procédé décrit dans l'Exemple A1 en utilisant un dextrane de masse moléculaire moyenne en poids d'environ 5 kg/mol (q = 19, PHARMACOSMOS), modifié par le glycinate de cétyle est obtenu.

D'après l'extrait sec : [Polysaccharide 2] = 15,1 mg/g
D'après le dosage acide/base : n = 1,05
D'après la RMN ¹H : m = 0,05.

### Exemple A3 : Préparation du Polysaccharide 3

Le phénylalaninate d'octyle, sel d'acide paratoluènesulfonique est obtenu selon le procédé décrit dans le brevet US 4,826,818.

Par un procédé similaire à celui décrit à l'Exemple A1, un dextraneméthylcarboxylate de sodium, synthétisé selon le procédé décrit dans l'Exemple A1 en utilisant un dextrane de masse moléculaire moyenne en poids d'environ 10 kg/mol (q = 38, PHARMACOSMOS), modifié par le phénylalaninate d'octyle est obtenu.

D'après l'extrait sec : [Polysaccharide 3] = 27,4 mg/g
D'après le dosage acide/base : n = 1,0
D'après la RMN ¹H : m = 0,1.

### Exemple A4 : Préparation du Polysaccharide 4

Par un procédé similaire à celui décrit à l'Exemple A3, un dextraneméthylcarboxylate de sodium, synthétisé selon le procédé décrit dans l'Exemple A1 en utilisant un dextrane de masse moléculaire moyenne en poids d'environ 5 kg/mol (q = 19, PHARMACOSMOS), modifié par le phénylalaninate d'octyle est obtenu.

D'après l'extrait sec : [Polysaccharide 4] = 21,8 mg/g
D'après le dosage acide/base : n = 1,0
D'après la RMN ¹H : m = 0,2.

### Exemple A5 : Préparation du Polysaccharide 5

Le phénylalaninate de 3,7-diméthyle-1-octyle, sel d'acide paratoluènesulfonique est obtenu selon le procédé décrit dans le brevet US 4,826,818.

Par un procédé similaire à celui décrit à l'Exemple A1, un dextraneméthylcarboxylate de sodium, synthétisé selon le procédé décrit dans l'Exemple A1 en utilisant un dextrane de masse moléculaire moyenne en poids d'environ 10 kg/mol (q = 38, PHARMACOSMOS), modifié par le phénylalaninate de 3,7-diméthyle-1-octyle est obtenu.

D'après l'extrait sec : [Polysaccharide 5] = 24,3 mg/g
D'après le dosage acide/base : n = 1,0
D'après la RMN ¹H : m = 0,1.

### Exemple A6 : Préparation du Polysaccharide 6

L'aspartate de dioctyle, sel d'acide paratoluènesulfonique est obtenu selon le procédé décrit dans le brevet US 4,826,818.

Par un procédé similaire à celui décrit à l'Exemple A1, un dextraneméthylcarboxylate de sodium, synthétisé selon le procédé décrit dans l'Exemple A1 en utilisant un dextrane de masse moléculaire moyenne en poids d'environ 10 kg/mol (q = 38, PHARMACOSMOS), modifié par l'aspartate de dioctyle est obtenu.

D'après l'extrait sec : (Polysaccharide 6] = 22,2 mg/g
D'après le dosage acide/base : n = 1,05
D'après la RMN ¹H : m = 0,05.

### Exemple A7 : Préparation du Polysaccharide 7

L'aspartate de didécyle, sel d'acide paratoluènesulfonique est obtenu selon le procédé décrit dans le brevet US 4,826,818.

Par un procédé similaire à celui décrit à l'Exemple A1, un dextraneméthylcarboxylate de sodium, synthétisé selon le procédé décrit dans l'Exemple A1 en utilisant un dextrane de masse moléculaire moyenne en poids d'environ 10 kg/mol (q = 38, PHARMACOSMOS), modifié par l'aspartate de didécyle est obtenu.

D'après l'extrait sec : [Polysaccharide 7] = 19,8 mg/g
D'après le dosage acide/base : n = 1,05
D'après la RMN ¹H : m = 0,05.

### Exemple A8 : Préparation du Polysaccharide 8

L'aspartate de dilauryle, sel d'acide paratoluènesulfonique est obtenu selon le procédé décrit dans le brevet US 4,826,818.

Par un procédé similaire à celui décrit à l'Exemple A1, un dextraneméthylcarboxylate de sodium, synthétisé selon le procédé décrit dans l'Exemple A1 en utilisant un dextrane de masse moléculaire moyenne en poids d'environ 5 kg/mol (q = 19, PHARMACOSMOS), modifié par l'aspartate de dilauryle est obtenu.

D'après l'extrait sec : [Polysaccharide 8] = 22,8 mg/g
D'après le dosage acide/base : n = 1,05
D'après la RMN ¹H : m = 0,05.

### Exemple A9 : Préparation du Polysaccharide 9

Le N-(2-aminoéthyl)dodécanamide est obtenu selon le procédé décrit dans le brevet US 2,387,201 à partir du méthyle ester de l'acide dodécanoïque (SIGMA) et de l'éthylènediamine (ROTH).

Par un procédé similaire à celui décrit à l'Exemple A1, un dextraneméthylcarboxylate de sodium, synthétisé selon le procédé décrit dans l'Exemple A1 en utilisant un dextrane de masse moléculaire moyenne en poids d'environ 10 kg/mol (q = 38, PHARMACOSMOS), modifié par le N-(2-aminoéthyl)dodécanamide est obtenu.

D'après l'extrait sec : [Polysaccharide 9] = 23,8 mg/g.
D'après le dosage acide/base : n = 1,0
D'après la RMN ¹H : m = 0,1.

### Exemple A10 : Préparation du Polysaccharide 10

Le dextranesuccinate de sodium est obtenu à partir d'un dextrane de masse molaire moyenne en poids d'environ 10 kg/mol (q = 38, PHARMACOSMOS) selon la méthode décrite dans l'article de Sanchez-Chaves et al., 1998 (Manuel et al., Polymer 1998, 39 (13), 2751-2757). D'après la RMN ¹H dans D₂O/NaOD, le nombre moyen de groupements succinates par unité glucosidique est de 1,4.

Le glycinate de lauryle, sel d'acide paratoluènesulfonique est obtenu selon le procédé décrit dans le brevet US 4,826,818.

Par un procédé similaire à celui décrit à l'Exemple A1, un dextranesuccinate de sodium modifié par le glycinate de lauryle est obtenu.

D'après l'extrait sec : [Polysaccharide 10] = 16,1 mg/g
D'après le dosage acide/base : n = 1,3
D'après la RMN ¹H : m = 0,1.

### Exemple A11 : Préparation du Polysaccharide 11

L'aspartate de dioctyle, sel d'acide paratoluènesulfonique est obtenu selon le procédé décrit dans le brevet US 4,826,818.

12 g (soit 0,22 mol d'hydroxyles) de dextrane de masse molaire moyenne en poids d'environ 10 kg/mol (q = 38, PHARMACOSMOS) sont solubilisés dans un mélange DMF/DMSO. Le mélange est porté à 80°C sous agitation. 3,32 g (0,03 mol) de 1,4-diazabicyclo[2.2.2]octane puis 14,35 g (0,11 mol) d'éthyle isocyanatoacétate sont progressivement introduits. Après 5h de réaction, le milieu est dilué en eau et purifié par diafiltration sur membrane PES de 5 kD contre NaOH 0,1N, NaCl 0,9% et de l'eau. La solution finale est dosée par extrait sec pour déterminer la concentration en polysaccharide; puis dosée par dosage acide/base dans de l'eau/acétone 50 / 50 (V/V) pour déterminer le nombre moyen de motifs N-méthylcarboxylates carbamates par unité glucosidique.

D'après l'extrait sec : [polysaccharide] = 30,5 mg/g
D'après le dosage acide/base : le nombre moyen de motifs N-méthylcarboxylates carbamates par unité glucosidique est de 1,4.

Par un procédé similaire à celui décrit à l'Exemple A1, un N-méthylcarboxylate de sodium dextrane carbamate modifié par l'aspartate de dioctyle est obtenu.

D'après l'extrait sec : [Polysaccharide 11] = 17,8 mg/g
D'après le dosage acide/base : n = 1,3
D'après la RMN ¹H : m = 0,1.

### Exemple A12 : Préparation du Polysaccharide 12

L'aspartate de dilauryle, sel d'acide paratoluènesulfonique est obtenu selon le procédé décrit dans le brevet US 4,826,818.

Par un procédé similaire à celui décrit à l'Exemple A1, un dextraneméthylcarboxylate de sodium, synthétisé selon le procédé décrit dans l'Exemple A1 en utilisant un dextrane de masse moléculaire moyenne en poids d'environ 1 kg/mol (q = 4, PHARMACOSMOS), modifié par l'aspartate de dilauryle est obtenu.

D'après l'extrait sec : [Polysaccharide 12] = 12,3 mg/g
D'après le dosage acide/base : n = 0,96
D'après la RMN ¹H : m = 0,07.

### Exemple A13 : Préparation du Polysaccharide 13

Le 2-(2-amino-éthoxy)éthyle dodécanoate, sel d'acide paratoluènesulfonique est obtenu selon le procédé décrit dans le brevet US 4,826,818.

Par un procédé similaire à celui décrit à l'Exemple A1, un dextraneméthylcarboxylate de sodium, synthétisé selon le procédé décrit dans l'Exemple A1 en utilisant un dextrane de masse moléculaire moyenne en poids d'environ 10 kg/mol (q = 38, PHARMACOSMOS), modifié par le 2-(2-amino-éthoxy)éthyle dodécanoate est obtenu.

D'après l'extrait sec : [Polysaccharide 13] = 25,6 mg/g
D'après le dosage acide/base : n = 1,0
D'après la RMN ¹H : m = 0,1.

### Exemple A14 : Préparation du Polysaccharide 14

Le 2-(2-{2-[dodécanoylamino]éthoxy}éthoxy)éthylamine est obtenu selon le procédé décrit dans le brevet US 2,387,201 à partir du méthyl ester de l'acide dodécanoïque (SIGMA) et du triéthylèneglycol diamine (HUNSTMAN).

Par un procédé similaire à celui décrit à l'Exemple A1, un dextraneméthylcarboxylate de sodium, synthétisé selon le procédé décrit dans l'Exemple A1 en utilisant un dextrane de masse moléculaire moyenne en poids d'environ 10 kg/mol (q = 38, PHARMACOSMOS), modifié par le 2-(2-{2-[dodécanoylamino]éthoxy}éthoxy)éthylamine est obtenu.

D'après l'extrait sec : [Polysaccharide 14] = 24,9 mg/g
D'après le dosage acide/base : n = 1,0
D'après la RMN ¹H : m = 0,1.

### Exemple A15 : Préparation du Polysaccharide 15

Le 2-(2-{2-[hexadécanoylamino]éthoxy}éthoxy)éthylamine est obtenu selon le procédé décrit dans le brevet US 2,387,201 à partir du méthyl ester de l'acide palmitique (SIGMA) et du triéthylèneglycol diamine (HUNSTMAN).

Par un procédé similaire à celui décrit à l'Exemple A1, un dextraneméthylcarboxylate de sodium, synthétisé selon le procédé décrit dans l'Exemple A1 en utilisant un dextrane de masse moléculaire moyenne en poids d'environ 10 kg/mol (q = 38, PHARMACOSMOS), modifié par le 2-(2-{2-[hexadécanoylamino]éthoxy}éthoxy)éthylamine est obtenu.

D'après l'extrait sec : [Polysaccharide 15] = 22,2 mg/g
D'après le dosage acide/base : n = 1,05
D'après la RMN ¹H : m = 0,05.

### Exemple A16 : Préparation du Polysaccharide 16

Le glycinate de cétyle, sel d'acide paratoluènesulfonique est obtenu selon le procédé décrit dans le brevet US 4,826,818.

Par un procédé similaire à celui décrit à l'Exemple A1, un dextraneméthylcarboxylate de sodium, synthétisé selon le procédé décrit dans l'Exemple A1 en utilisant un dextrane de masse moléculaire moyenne en poids d'environ 5 kg/mol (q = 19, PHARMACOSMOS), modifié par le glycinate de cétyle est obtenu.

D'après l'extrait sec : [Polysaccharide 16] = 23 mg/g
D'après le dosage acide/base : n = 1,05
D'après la RMN ¹H : m = 0,05.

### Exemple A17 : Préparation du Polysaccharide 17

10 g (soit 185 mmol d'hydroxyles) de dextrane de masse molaire moyenne en poids d'environ 10 kg/mol (q = 38, PHARMACOSMOS) sont solubilisés dans de l'eau à 420 g/L. A cette solution sont ajoutés 19 mL de NaOH 10 N (185 mmol). Le mélange est porté à 35°C, puis 8,6 g (74 mmol) de chloroacétate de sodium sont ajoutés. La température du milieu réactionnel est portée à 60°C à 0,5°C/min, puis maintenue à 60°C pendant 100 minutes. Le milieu réactionnel est dilué avec 200 mL d'eau, neutralisé à l'acide acétique et purifié par ultrafiltration sur membrane PES de 5 kDa contre 6 volumes d'eau. La solution finale est dosée par extrait sec pour déterminer la concentration en polysaccharide ; puis dosée par dosage acide/base dans de l'eau/acétone 50 / 50 (V/V) pour déterminer le nombre moyen de motifs méthylcarboxylates par unité glucosidique.

D'après l'extrait sec : (polysaccharide) = 35,1 mg/g
D'après le dosage acide/base : le nombre moyen de motifs méthylcarboxylates par unité glucosidique est de 0,42.

La solution de dextraneméthylcarboxylate de sodium est passée sur une résine Purolite (anionique) pour obtenir le dextraneméthylcarboxyllque acide qui est ensuite lyophilisé pendant 18 heures.

Le glycinate de cétyle, sel d'acide paratoluènesutfonique est obtenu selon le procédé décrit dans le brevet US 4,826,818.

Par un procédé similaire à celui décrit à l'Exemple A1, un dextraneméthylcarboxylate de sodium modifié par le glycinate de cétyle est obtenu.

D'après l'extrait sec : [Polysaccharide 17] = 18 mg/g
D'après le dosage acide/base : n = 0,37
D'après la RMN ¹H : m = 0,05.

### Exemple A18 : Préparation du Polysaccharide 18

10g de dextraneméthylcarboxylate de sodium caractérisé par un degré de substitution en méthylcarboxylate de 1,10 par unité glucosidique sont synthétisés à partir d'un dextrane de masse molaire moyenne en poids de 5 kg/mol (q = 19, PHARMACOSMOS), selon un procédé similaire à celui décrit pour le Polysaccharide 1, puis lyophilisés.

8 g (soit 64 mmol d'hydroxyles) de dextraneméthylcarboxylate de sodium caractérisé par un degré de substitution en méthylcarboxylate de 1,05 par unité glucosidique sont solubilisés dans de l'eau à 1000 g/L. 6 mL de NaOH 10 N (64 mmol) sont ajoutés. Le mélange est chauffé à 35°C, et 7,6 g de chloroacétate de sodium (65 mmol) sont ajoutés. Le mélange est progressivement porté à une température de 60°C, et est maintenu à cette température pendant 100 minutes supplémentaires. Le mélange est dilué avec de l'eau, neutralisé par l'acide acétique puis purifié par ultrafiltration sur membrane PES de 5 kDa contre de l'eau. La solution finale est dosée par extrait sec pour déterminer la concentration en polysaccharide puis dosée par dosage acide/base dans de l'eau/acétone 50 / 50 (V/V) pour déterminer le nombre moyen de motifs méthylcarboxylates par unité glucosidique.

D'après l'extrait sec : [polysaccharide] = 45,8 mg/g
D'après le dosage acide/base : le nombre moyen de motifs méthylcarboxylates par unité glucosidique est de 1,65.

La solution de dextraneméthylcarboxylate de sodium est passée sur une résine Purolite (anionique) pour obtenir le dextraneméthylcarboxylique acide qui est ensuite lyophilisé pendant 18 heures.

Le leucinate de cholestéryle, sel d'acide paratoluènesulfonique est obtenu selon le procédé décrit dans le brevet US 4,826,818.

Par un procédé similaire à celui décrit à l'Exemple A1, un dextraneméthylcarboxylate de sodium modifié par le leucinate de cholestéryle est obtenu.

D'après l'extrait sec : [Polysaccharide 18] = 21 mg/g
D'après le dosage acide/base : n = 1,61
D'après la RMN ¹H : m = 0,04.

### Exemple A19 : Préparation du Polysaccharide 19

Par un procédé similaire à celui décrit à l'Exemple A1, un dextraneméthylcarboxylate de sodium, synthétisé selon le procédé décrit dans l'Exemple A1 en utilisant un dextrane de masse moléculaire moyenne en poids d'environ S kg/mol (q = 19, PHARMACOSMOS), modifié par le leucinate de cholestryle est obtenu.

D'après l'extrait sec : [Polysaccharide 19] = 19,4 mg/g
D'après le dosage acide/base : n = 1,06
D'après la RMN ¹H : m = 0,04.

### Exemple A20 : Préparation du Polysaccharide 20

16 g (soit 296 mmol d'hydroxyles) de dextrane de masse molaire moyenne en poids d'environ 5 kg/mol (q = 19, PHARMACOSMOS) sont solubilisés dans de l'eau à 420 g/L. A cette solution sont ajoutés 30 mL de MaOH 10 N (296 mmol). Le mélange est porté à 35°C, puis 26 g (222 mol) de chloroacétate de sodium sont ajoutés. La température du milieu réactionnel est progressivement portée à 60°C, puis maintenue à 60°C pendant 100 minutes. Le milieu réactionnel est dilué avec de l'eau, neutralisé à l'acide acétique et purifié par ultrafiltration sur membrane PES de 5 kDa contre de l'eau. La solution finale est dosée par extrait sec pour déterminer la concentration en polysaccharide puis dosée par dosage acide/base dans de l'eau/acétone 50 / 50 (V/V) pour déterminer le nombre moyen de motifs méthylcarboxylates par unité glucosidique.

D'après l'extrait sec : [polysaccharide] = 33,1 mg/g
D'après le dosage acide/base : le nombre moyen de motifs méthylcarboxylates par unité glucosidique est de 0,70.

La solution de dextraneméthylcarboxylate de sodium est passée sur une résine Purolite (anionique) pour obtenir le dextraneméthylcarboxylique acide qui est ensuite lyophilisé pendant 18 heures.

Par un procédé similaire à celui décrit à l'Exemple A1, un dextraneméthylcarboxylate de sodium modifié par le leucinate de cholestéryle est obtenu.

D'après l'extrait sec : [Polysaccharide 20] = 18,9 mg/g
D'après le dosage acide/base : n = 0,66
D'après la RMN ¹H : m = 0,04.

### Exemple A21 : Préparation du Polysaccharide 21

16 g (soit 296 mmol d'hydroxyles) de dextrane de masse molaire moyenne en poids d'environ 5 kg/mol (q = 19, PHARMACOSMOS) sont solubilisés dans de l'eau à 420 g/L. A cette solution sont ajoutés 30 mL de NaOH 10 N (296 mmol). Le mélange est porté à 35°C, puis 18 g (158 mmol) de chloroacétate de sodium sont ajoutés. La température du milieu réactionnel est progressivement portée à 60°C, puis maintenue à 60°C pendant 100 minutes. Le milieu réactionnel est dilué avec de l'eau, neutralisé à l'acide acétique et purifié par ultrafiltration sur membrane PES de 1 kDa contre de l'eau. La solution finale est dosée par extrait sec pour déterminer la concentration en polysaccharide puis dosée par dosage acide/base dans de l'eau/acétone 50 / 50 (V/V) pour déterminer le nombre moyen de motifs méthylcarboxylates par unité glucosidique.

D'après l'extrait sec : [polysaccharide] = 52,6 mg/g
D'après le dosage acide/base : le nombre moyen de motifs méthylcarboxylates par unité glucosidique est de 0,50.

La solution de dextraneméthylcarboxylate de sodium est passée sur une résine Purolite (anionique) pour obtenir le dextraneméthylcarboxylique acide qui est ensuite lyophilisé pendant 18 heures.

Par un procédé similaire à celui décrit à l'Exemple A1, un dextraneméthylcarboxylate de sodium modifié par le leucinate de cholestéryle est obtenu.

D'après l'extrait sec : [Polysaccharide 21] = 18,9 mg/g
D'après le dosage acide/base : n = 0,46
D'après la RMN ¹H : m = 0,04.

### Exemple A22 : Préparation du Polysaccharide 22

Par un procédé similaire à celui décrit à l'Exemple A18, un dextraneméthylcarboxylate de sodium, synthétisé selon le procédé décrit dans l'Exemple A18 en utilisant un dextrane de masse moléculaire moyenne en poids d'environ 1 kg/mol (q = 4, PHARMACOSMOS), modifié par le leucinate de cholestéryle est obtenu.

D'après l'extrait sec : [Polysaccharide 22] = 20,2 mg/g
D'après le dosage acide/base : n = 1,61
D'après la RMN ¹H : m = 0,04.

### Exemple A23 : Préparation du Polysaccharide 23

Le chlorhydrate de 1-éthylènediaminecarboxylate de cholestéryle est obtenu selon le procédé décrit dans le brevet (Akiyoshi, K et al. WO 2010/053140).

Par un procédé similaire à celui décrit à l'Exemple A18, un dextraneméthylcarboxylate de sodium, synthétisé selon le procédé décrit dans l'Exemple A18 en utilisant un dextrane de masse moléculaire moyenne en poids d'environ 5 kg/mol (q = 19, PHARMACOSMOS), modifié par le 1-éthylènediaminecarboxylate de cholestéryle est obtenu.

D'après l'extrait sec : [Polysaccharide 23] = 20,1 mg/g
D'après le dosage acide/base : n = 1,61
D'après la RMN ¹H : m = 0,04.

### Exemple A24 : Préparation du Polysaccharide 24

12 g (soit 0,22 mol d'hydroxyles) de dextrane de masse molaire moyenne en poids d'environ 5 kg/mol (q = 19, PHARMACOSMOS) sont solubilisés dans un mélange DMF/DMSO. Le mélange est porté à 80°C sous agitation. 3,32 g (0,03 mol) de 1,4-diazabicyclo[2.2.2]octane puis 26,8 g (0,21 mol) d'éthyle isocyanatoacétate sont progressivement introduits. Après 5h de réaction, le milieu est dilué en eau et purifié par diafiltration sur membrane PES de 5 kD contre NaOH 0,1N, NaCl 0,9% et de l'eau. La solution finale est dosée par extrait sec pour déterminer la concentration en polysaccharide; puis dosée par dosage acide/base dans de l'eau/acétone 50 / 50 (V/V) pour déterminer le nombre moyen de motifs N-méthylcarboxylates carbamates par unité glucosidique.

D'après l'extrait sec : [polysaccharide] = 30,1 mg/g
D'après le dosage acide/base : le nombre moyen de motifs N-méthylcarboxylates carbamates par unité glucosidique est de 2,0.

Par un procédé similaire à celui décrit à l'Exemple A1, un N-méthylcarboxylate de sodium dextrane carbamate modifié par le leucinate de cholestéryle est obtenu.

D'après l'extrait sec : [Polysaccharide 24] = 17,9 mg/g
D'après le dosage acide/base : n = 1,96
D'après la RMN ¹H : m = 0,04.

### Exemple A25 : Préparation du Polysaccharide 25

Le chlorhydrate de 1-éthylènediaminecarboxylate de cholestéryle est obtenu selon le procédé décrit dans le brevet (Akiyoshi, K et al. WO 2010/053140).

10 g de dextrane de masse molaire moyenne en poids d'environ 5 kg/mol (q = 19, PHARMACOSMOS, 3,2 mmol de bouts de chaines) sont solubilisés dans du DMSO à 80°C. 4,8 g de chlorhydrate de 1-éthylènediaminecarboxylate de cholestéryle (9,5 mmol), 0,96 g de triéthylamine (9,5 mmol) et 2,0 g de cyanoborohydrure de sodium (32 mmol) sont ajoutés au milieu réactionnel qui est agité à 80°C pendant 24 heures. Après refroidissement, le mélange est précipité dans du dichlorométhane puis dans de l'acétone et séché sous vide. D'après la RMN ¹H, un dextrane modifié en bout de chaine par le 1-éthylènediaminecarboxylate de cholestéryle est obtenu. Un dextraneméthylcarboxylate de sodium caractérisé par un degré de substitution en méthylcarboxylate de 1,65 par unité glucosidique et modifié en bout de chaine par le 1-éthylènediaminecarboxylate de cholestéryle a été synthétisé par un procédé similaire à celui décrit dans l'exemple A18 en utilisant le dextrane modifié en bout de chaine par le 1-éthylènediaminecarboxylate de cholestéryle.

D'après l'extrait sec : [Polysaccharide 25] = 13,7 mg/g
D'après le dosage acide/base : n = 1,65
D'après la RMN ¹H : chaque chaîne de polymère porte un groupement 1-éthylènediaminecarboxylate de cholestéryle greffé sur le boût de chaîne réducteur.

### Exemple A26 : Préparation du Polysaccharide 26

Le leucinate de cholestérol, sel d'acide paratoluènesulfonique est obtenu selon le procédé décrit dans le brevet US 4,826,818.

Par un procédé similaire à celui décrit à l'Exemple A1, un dextraneméthylcarboxylate de sodium, synthétisé selon le procédé décrit dans l'Exemple A1 en utilisant un dextrane de masse moléculaire moyenne en poids d'environ 10 kg/mol (q = 38, PHARMACOSMOS), modifié par le leucinate de cholestérol est obtenu.

D'après l'extrait sec : [Polysaccharide 26] = 26,6 mg/g
D'après le dosage acide/base : n = 0,99
D'après la RMN ¹H : m = 0,05.

### Exemple A27 : Préparation du Polysaccharide 27

L'aspartate de dilauryle, sel d'acide paratoluènesulfonique est obtenu selon le procédé décrit dans le brevet US 4,826,818.

Par un procédé similaire à celui décrit à l'Exemple A1, un dextraneméthylcarboxylate de sodium, synthétisé selon le procédé décrit dans l'Exemple A18 en utilisant un dextrane de masse moléculaire moyenne en poids d'environ 1 kg/mol (q = 4, PHARMACOSMOS), modifié par l'aspartate de dilauryle est obtenu.

D'après l'extrait sec : [Polysaccharide 27] = 16,7 mg/g
D'après le dosage acide/base : n = 1,41
D'après la RMN ¹H : m = 0,16.

### Exemple A28 : Préparation du Polysaccharide 28

L'aspartate de dilauryle, sel d'acide paratoluènesulfonique est obtenu selon le procédé décrit dans le brevet US 4,826,818.

Par un procédé similaire à celui décrit à l'Exemple A1, un dextraneméthylcarboxylate de sodium, synthétisé selon le procédé décrit dans l'Exemple A18 en utilisant un dextrane de masse moléculaire moyenne en poids d'environ 1 kg/mol (q = 4, PHARMACOSMOS), modifié par l'aspartate de dilauryle est obtenu.

D'après l'extrait sec : [Polysaccharide 28] = 25 mg/g
D'après le dosage acide/base : n = 1,50
D'après la RMN ¹H : m = 0,07.

### Exemple A29 : Préparation du Polysaccharide 29

L'aspartate de didécyle, sel d'acide paratoluènesulfonique est obtenu selon le procédé décrit dans le brevet US 4,826,818.

Par un procédé similaire à celui décrit à l'Exemple A1, un dextraneméthylcarboxylate de sodium, synthétisé selon le procédé décrit dans l'Exemple A1 en utilisant un dextrane de masse moléculaire moyenne en poids d'environ 1 kg/mol (q = 4, PHARMACOSMOS), modifié par l'aspartate de didécyle est obtenu.

D'après l'extrait sec : [Polysaccharide 29] = 15 mg/g
D'après le dosage acide/base : n = 1,05
D'après la RMN ¹H : m = 0,05.

### Exemples

Partie B Mise en évidence des propriétés des compositions selon l'invention

### Exemple B1 : Solution d'insuline analogue rapide (Novolog^{®}) à 100 UI/mL

Cette solution est une solution commerciale d'insuline aspart commercialisée par la société Novo Nordisk sous le nom de Novolog^{®} aux USA et Novorapid^{®} en Europe. Ce produit est une insuline analogue rapide.

### Exemple B2 : Solution d'insuline analogue rapide (Humalog^{®}) à 100 UI/mL

Cette solution est une solution commerciale d'insuline lispro commercialisée par la société Eli Lilly sous le nom de Humalog^{®}. Ce produit est une insuline analogue rapide.

### Exemple B3 : Solution d'insuline analogue rapide (Apidra^{®}) à 100 UI/mL

Cette solution est une solution commerciale d'insuline glulisine commercialisée par la société Sanofi-Aventis sous le nom Apidra^{®}. Ce produit est une insuline analogue rapide.

### Exemple 84 : Solution d'insuline analogue lente (Lantus^{®}) à 100 UI/mL

Cette solution est une solution commerciale d'insuline glargine commercialisée par la société Sanofi-Aventis sous le nom de Lantus^{®}. Ce produit est une insuline analogue lente.

### Exemple B5 : Solution d'insuline humaine (ActRapid^{®}) à 100 UI/mL

Cette solution est une solution commerciale de Novo Nordisk vendue sous le nom d'Actrapid^{®}. Ce produit est une insuline humaine.

### Exemple B6 : Solubilisation de Lantus^{®}. à 100 UI/mL et à pH 7 à l'aide d'un dextrane substitué

20 mg de Polysaccharide 4 décrit à l'exemple A4 sont pesés précisément. Ce lyophilisat est repris par 2 mL de Lantus^{®} dans sa formulation commerciale. Un précipité provisoire apparaît mais la solution devient limpide au bout d'environ 30 minutes. Le pH de cette solution est de 6,3. Le pH est ajusté à 7 avec une solution de soude 0,1 N. Cette solution limpide est filtrée sur 0,22 µm puis est placée à +4°C.

### Exemple 87 : Préparation d'une composition dextrane substitué/Lantus^{®}/Apidra^{®} 75/25 à pH 7

A 0,75 mL de la solution de Polysaccharide 4/Lantus^{®} préparée à l'exemple 86 est ajouté 0,25 mL d'Apidra^{®} (dans sa formulation commerciale) pour former 1 mL d'une composition à pH 7. La composition est limpide attestant de la bonne solubilité de Lantus^{®} et d'Apidra^{®} dans ces conditions de formulation. Cette solution limpide est filtrée sur 0,22 µm puis est placée à +4°C.

### Exemple B8 : Préparation d'une composition dextrane substitué/Lantus^{®}/Humalog^{®} 75/25 à pH 7

A 0,75 mL de la solution de Polysaccharide 4/Lantus^{®} préparée à l'exemple B6 est ajouté 0,25 mL d'Humalog^{®} (dans sa formulation commerciale) pour former 1 mL d'une composition à pH 7. La composition est limpide attestant de la bonne solubilité de Lantus^{®} et d'Humalog^{®} dans ces conditions de formulation. Cette solution limpide est filtrée sur 0,22 µm puis est placée à +4°C.

### Exemple B9 : Préparation d'une composition dextrane substitué/Lantus^{®}/Novolog^{®} 75/25 à pH 7

A 0,75 mL de la solution de Polysaccharide 4/Lantus^{®} préparée à l'exemple B6 est ajouté 0,25 mL de Novolog^{®} (dans sa formulation commerciale) pour former 1 mL d'une composition à pH 7. La composition est limpide attestant de la bonne solubilité de Lantus^{®} et de Novolog^{®} dans ces conditions de formulation. Cette solution limpide est filtrée sur 0,22 µm puis est placée à +4°C.

### Exemple B10 : Préparation d'une composition dextrane substitué/Lantus^{®}/ActRapid^{®} 75/25 à pH 7

A 0,75 mL de la solution de Polysaccharide 4/Lantus^{®} préparée à l'exemple 86 est ajouté 0,25 mL d'ActRapid^{®} (dans sa formulation commerciale) pour former 1 mL d'une composition à pH 7. La composition est limpide attestant de la bonne solubilité de Lantus^{®} et d'ActRapid^{®} dans ces conditions de formulation. Cette solution limpide est filtrée sur 0,22 µm puis est placée à +4°C.

### Exemple 811 : Préparation d'une composition dextrane substitué/Lantus^{®}/Apidra^{®} 60/40 à pH 7

A 0,6 mL de la solution de Polysaccharide 4/Lantus^{®} préparée à l'exemple B6 est ajoutés 0,4 mL d'Apidra^{®} (dans sa formulation commerciale) pour former 1 mL d'une composition à pH 7. La composition est limpide attestant de la bonne solubilité de Lantus^{®} et d'Apidra^{®} dans ces conditions de formulation. Cette solution limpide est filtrée sur 0,22 µm puis est placée à +4°C.

### Exemple B12 : Préparation d'une composition dextrane substltué/Lantus^{®}/Apidra^{®} 40/60 à pH 7

A 0,4 mL de la solution de Polysaccharide 4/Lantus^{®} préparée à l'exemple B6 est ajouté 0,6 mL d'Apidra^{®} (dans sa formulation commerciale) pour former 1 mL d'une composition à pH 7. La composition est limpide attestant de la bonne solubilité de Lantus^{®} et d'Apidra^{®} dans ces conditions de formulation. Cette solution limpide est filtrée sur 0,22 µm puis est placée à +4°C.

### Exemple B13 : Précipitation de Lantus^{®}

1 mL de Lantus^{®} est ajouté dans 2 mL d'une solution de PBS contenant 20 mg/mL de BSA (bovine serum albumin). Le mélange PBS/BSA simule la composition du milieu sous-cutané. Un précipité apparaît ce qui est en bon accord avec le mécanisme de fonctionnement de Lantus^{®} (précipitation à l'injection due à l'augmentation du pH).

Une centrifugation à 4000 trs/min est effectuée pour séparer le précipité du surnageant. Ensuite Lantus^{®} est dosé dans le surnageant. Il en résulte que 86% de Lantus^{®} se retrouve sous une forme précipitée.

### Exemple B14 : Précipitation d'une composition dextrane substitué/Lantus^{®}

1 mL de solution Polysaccharide 4/Lantus^{®} préparée à l'exemple B6 est ajouté dans 2 mL d'une solution de PBS contenant 20 mg/mL de BSA (bovine sérum albumin). Le mélange PBS/BSA simule la composition du milieu sous-cutané. Un précipité apparaît.

Une centrifugation à 4000 trs/min est effectuée pour séparer le précipité du surnageant. Ensuite Lantus^{®}) est dosé dans le surnageant. Il en résulte que 85% de Lantus^{®} se retrouve sous une forme précipitée. Ce pourcentage de précipitation de Lantus^{®} est identique à celui obtenu pour le contrôle décrit dans l'exemple B13.

Des essais de solubilisation et précipitation Identiques à ceux décrits aux exemples B6 et B14 ont été effectués avec d'autres dextranes substitués à la même concentration de 10 mg/mL de polysaccharide pour 100 UI/mL de Lantus^{®}. 20 mg de polysaccharide sous forme de lyophilisat sont pesés précisément. Ce lyophilisat est repris par 2 mL de Lantus^{®} dans sa formulation commerciale. Un précipité provisoire apparaît mais la solution devient limpide au bout d'environ 30 minutes à quelques heures (selon la nature du polysaccharide). Le pH de cette solution est de 6,3. Le pH est ajusté à 7 avec une solution de soude 0,1 N. Cette solution limpide est filtrée sur 0,22 µm puis est placée à +4°C. Les résultats sont résumés dans le tableau 2.

**Tableau 2**

| Polysaccharide n° | Solubilisation de Lantus^{®} | Précipitation de Lantus^{®} | % de précipitation |
|---|---|---|---|
| 2 | Oui | Oui | 85 |
| 1 | Oui | Oui | Non mesuré |
| 4 | Oui | Oui | 87 |
| 3 | Oui | Oui | Non mesuré |
| 5 | Oui | Oui | 94 |
| 6 | Oui | Oui | Non mesuré |
| 7 | Oui | Oui | Non mesuré |
| 8 | Oui | Oui | Non mesuré |
| 9 | Oui | Oui | 94 |
| 10 | Oui | Oui | Non mesuré |
| 15 | Oui | Oui | Non mesuré |
| 14 | Oui | Oui | Non mesuré |
| 13 | Oui | Oui | Non mesuré |
| 12 | Oui | Oui | Non mesuré |
| 11 | Oui | Oui | Non mesuré |
| 16 | Oui | Oui | Non mesuré |
| 17 | Oui | Oui | Non mesuré |
| 18 | Oui | Oui | Non mesuré |
| 19 | Oui | Oui | Non mesuré |
| 20 | Oui | Oui | Non mesuré |
| 21 | Oui | Oui | Non mesuré |
| 22 | Oui | Oui | Non mesuré |
| 23 | Oui | Oui | Non mesuré |
| 24 | Oui | Oui | Non mesuré |
| 25 | Oui | Oui | Non mesuré |
| 26 | Oui | Oui | Non mesuré |

### Exemple B15 : Précipitation d'une composition dextrane substitué/Lantus^{®}/Apidra^{®} 75/25 à pH 7

1 mL de la composition dextrane substitué/Lantus^{®}/Apidra^{®} 75/25 (contenant 7,5 mg/mL de polysaccharide, 75 UI/mL de Lantus^{®} et 25 UI/mL d'Apidra^{®}) préparée à l'exemple B7 est ajouté dans 2 mL d'une solution de PBS contenant 20 mg/mL de BSA (bovine sérum albumin). Le mélange PBS/BSA simule la composition du milieu sous-cutané. Un précipité apparaît.

Une centrifugation à 4000 trs/min est effectuée pour séparer le précipité du surnageant. Ensuite Lantus^{®} est dosé dans le surnageant. Les pourcentages de précipitation de Lantus^{®} sont similaires au contrôle décrit dans l'exemple B13.

### Exemple 816 : Précipitation de différentes compositions en faisant varier la nature du dextrane substitué

D'autres essais dans les mêmes conditions que celles de l'exemple B15 ont été effectués en présence d'autres dextranes substitués.

Les résultats sont regroupés dans le tableau 3 suivant et on observe que la solubilisation et la précipitation de Lantus^{®} sont conservées.

**Tableau 3**

| Polysaccharide n° | Solubilisation Lantus^{®}/Apidra^{®} 75/25 | Pourcentage de précipitation de Lantus^{®} |
|---|---|---|
| 2 | Oui | 85 |
| 1 | Oui | Non mesuré |
| 4 | Oui | 87 |
| 3 | Oui | Non mesuré |
| 5 | Oui | 86 |
| 6 | Oui | Non mesuré |
| 7 | Oui | Non mesuré |
| 8 | Oui | Non mesuré |
| 9 | Oui | 86 |
| 10 | Oui | 85 |
| 15 | Oui | 87 |
| 14 | Oui | 86 |
| 13 | Oui | 88 |
| 12 | Oui | 91 |
| 18 | Oui | Non mesuré |
| 19 | Oui | Non mesuré |
| 20 | Oui | Non mesuré |
| 21 | Oui | Non mesuré |
| 22 | Oui | Non mesuré |
| 23 | Oui | Non mesuré |
| 24 | Oui | Non mesuré |
| 25 | Oui | Non mesuré |
| 26 | Oui | Non mesuré |

### Exemple B17 : Précipitation de différentes compositions en faisant varier la nature de l'insuline prandiale

Des compositions sont préparées en mélangeant 0,75 mL de la solution de Polysaccharide 4/Lantus^{®} préparée à l'exemple B6 avec 0,25 mL d'une insuline prandiale pour forme 1 ml de composition dextrane substitué/Lantus^{®}/insuline prandiale (contenant 7,5 mg/mL de polysaccharide, 75 UI/mL de Lantus^{®} et 25 UI/mL d'insuline prandiale).

Cette composition est ajoutée dans 2 mL de PBS contenant 20 mg/mL de BSA (bovine serum albumin). Le mélange PBS/BSA simule la composition du milieu sous-cutané. Un précipité apparaît.

Une centrifugation à 4000 trs/min est effectuée pour séparer le précipité du surnageant. Ensuite Lantus^{®} est dosé dans le surnageant. En présence des 4 insulines prandiales testées, Lantus^{®} précipite à environ 90%. Ce pourcentage de précipitation de Lantus^{®} est similaire au contrôle décrit dans l'exemple B13, les résultats sont regroupés dans le tableau 4.

**Tableau 4**

| Nature de l'insuline prandiale | Solubilisation Lantus^{®}linsuline prandiale 75/25 | Pourcentage de précipitation de Lantus^{®} |
|---|---|---|
| Apidra^{®} | Oui | 88 |
| Novolog^{®} | Oui | 92 |
| Humalog^{®} | Oui | 89 |
| ActRapid^{®} | Oui | 90 |

### Exemple B18: Préparation d'une solution d'insuline analogue lente (glargine) concentrée.

Une solution commerciale d'insuline glargine commercialisée par la société Sanofi-Aventis sous le nom de Lantus^{®} est concentrée par ultrafiltration sur une membrane 3 kDa en cellulose régénérée (Amicon^{®} Ultra-15 commercialisée par la société Millipore). A l'issue de cette étape d'ultraflltration la concentration en insuline glargine est dosée dans le rétentat par chromatographie liquide en phase inverse (RP-HPLC). La concentration finale en insuline glargine est ensuite ajustée par l'ajout de solution commerciale de glargine à 100 UI/mL pour obtenir la concentration finale souhaitée. Ce procédé permet d'obtenir des solutions concentrées de glargine notées C_{glargine} à diverses concentrations supérieures à 100 UI/mL, telles que C_{glargine} = 200, 250, 300 et 333 IU/mL. Les solutions concentrées sont filtrées sur 0,22 µm puis stockées à +4°C.

Exemple B19 : Dialyse d'une solution commerciale d'insuline analogue rapide (lispro). Une solution commerciale d'insuline lispro commercialisée par la société Lilly sous le nom d'Humalog^{®} est dialysée par ultrafiltration sur une membrane 3 kDa en cellulose régénérée (Amicon^{®} Ultra-15 commercialisée par la société Millipore). La dialyse est réalisée dans un tampon phosphate 1 mM à pH 7. A l'issue de cette étape de dialyse la concentration C_{Humalog dialysé} en lispro dans le rétentat est déterminée par chromatographie liquide en phase inverse (RP-HPLC). La solution dialysée est conservée dans un congélateur à -80°C.

### Exemple 820 : Lyophilisation d'une solution d'insuline analogue rapide (lispro) sous sa forme commerciale.

Un volume V_{Humalog} d'une solution d'insuline rapide lispro à une concentration de 100 UI/mL dans sa forme commerciale est placée dans un lyogard^{®}préalablement stérilisé dans un autoclave. Le lyogard^{®} est placé dans un congélateur à -80°C pendant environ 1h avant de subir une lyophilisation pendant une nuit à une température de 20°C et une pression de 0,31 mbar.

Le lyophilisat stérile ainsi obtenu est conservé à température ambiante.

### Exemple B21 : Lyophilisation d'une solution commerciale d'insuline analogue rapide (lispro) dialysée.

Un volume V_{Humalog dialysé} d'une solution d'insuline rapide lispro obtenu selon l'exemple 619 à une concentration de C_{Humalog dialysé} est placée dans un lyogard^{®} préalablement stérilisé dans un autoclave. Le lyogard^{®} est placé dans un congélateur à -80°C pendant environ 1h avant de subir une lyophilisation pendant une nuit à une température de 20°C et une pression de 0,31 mbar.

Le lyophilisat stérile ainsi obtenu est conservé à température ambiante.

### Exemple B22 : Préparation d'une composition dextrane substitué/glargine à pH 7 à l'aide d'un dextrane substitué, suivant un procédé utilisant glargine sous forme liquide (en solution) et un polysacharide sous forme solide (lyophilisée).

Une masse m_{polys.} de Polysaccharide 18 est pesée précisément. Ce lyophilisat est repris par un volume V_{glargine} d'une solution concentrée de glargine préparée selon l'exemple B18 de manière à obtenir une composition présentant une concentration de polysaccharide C_{polys.}(mg/mL) = m_{polys}/V_{glargine} et une concentration en glargine C_{glargine} (UI/mL). La solution est opalescente. Le pH de cette solution est d'environ 6,3. Le pH est ajusté à 7 par ajout de NaOH concentrée puis la solution est placée en statique dans une étuve à 37°C pendant environ 1 heure. Un volume V_{polys}._{/glargine} de cette solution visuellement limpide est placée à +4°C.

### Exemple B23 : Préparation d'une composition dextrane substitué/glargine à pH 7 à l'aide d'un dextrane substitué, suivant un procédé utilisant glargine sous forme liquide (en solution) et un polysacharide sous forme liquide (en solution).

A une solution mère de Polysaccharide 20 à pH 7 présentant une concentration C_{mèrepolys.} sont ajoutées des solutions concentrées de m-crésol, glycérine et tween 20 de manière à obtenir une solution de polysaccharide de concentration C_{mèrepolys./exctpients} (mg/mL) en présence de ces excipients à des teneurs équivalentes à celles décrites dans la solution commerciale Lantus^{®} en flacon de 10 mL.

Dans un pot stérile, un volume V_{Lantus} d'une solution commerciale d'insuline lente glargine commercialisée sous le nom de Lantus^{®} à une concentration de 100 IU/mL est ajoutée à un volume V _{mèrepolys./exctpients} d'une solution de polysaccharide à la concentration C_{mèrepolys./exctpients} (mg/mL). Un trouble apparaît. Le pH est ajusté à pH 7 par ajout de NaOH 1M et la solution est placée en statique dans une étuve à 37°C pendant environ 1h. Cette solution visuellement limpide est placée à +4°C.

### Exemple 824 : Préparation d'une composition polysaccharide/glargine concentrée à pH=7 à l'aide d'un dextrane substitué, suivant un procédé de concentration d'une composition diluée.

Une composition Polysaccharide 20/glargine diluée à pH 7 décrite dans l'exemple B23 est concentrée par ultrafiltration sur une membrane 3 kDa en cellulose régénérée (Amicon^{®} Ultra-15 commercialisée par la société Millipore). A l'issue de cette étape d'ultrafiltration, le rétentat est limpide et la concentration en Insuline glargine dans la composition est dosée par chromatographie en phase inverse (RP-HPLC). Si nécessaire, la concentration en insuline glargine dans la composition est ensuite ajustée à la valeur souhaitée par dilution dans une solution d'excipients m-crésol/Glycérine/tween20 présentant, pour chaque espèce une concentration équivalente à celle décrite dans la solution commerciale Lantus^{®} (en flacon de 10 mL). Cette solution à pH 7, visuellement limpide, présentant une concentration en glargine C_{glargine} (UI/mL) et une concentration en polysaccharide C_{polys.}(mg/mL), est placée à +4°C.

### Exemple B25 : Préparation d'une composition dextrane substitué/glargine/lispro à pH 7, à partir d'une insuline rapide lispro sous sa forme commerciale.

Un volume V_{polysach./glargine} de solution de polysaccharide/glargine pH 7 présentant une concentration en glargine C_{glargine} (UI/mL) et une concentration de Polysaccharide 18 C_{polys}. (mg/mL) préparé selon l'exemple B22 est ajouté sur un lyophilisat d'insuline lispro obtenu par lyophilisation d'un volume Vₗᵢₛₚᵣₒ dont la préparation est décrite dans l'exemple B19, de telle manière que le ratio V_{polysach./glargine}/ Vₗᵢₛₚᵣₒ = 100 / Cₗᵢₛₚᵣₒ où Cₗᵢₛₚᵣₒ est la concentration en lispro (UI/mL) visée dans la composition. La solution est limpide. La teneur en zinc de la formulation est ajustée à la concentration C_{zinc} (µM) souhaitée par ajout d'une solution concentrée de chlorure de zinc. Le pH final est ajusté à 7 par ajout de NaOH ou HCl concentré.

La formulation est limpide, attestant de la bonne solubilité de glargine et lispro dans ces conditions de formulation. Cette solution est filtrée sur 0,22 µm et placée à +4°C.

### Exemple B26 : Préparation d'une composition dextrane substitué/glargine/lispro à pH 7, à partir d'une insuline rapide lispro obtenue par dialyse d'une solution commerciale.

Un volume V_{polysach./glargine} de solution de polysaccharide/glargine pH 7 présentant une concentration en glargine C_{glargine} (UI/mL) et une concentration de Polysaccharide 20 C_{polys.} (mg/mL) préparé selon l'exemple B24 est ajouté sur un lyophilisat d'insuline lispro obtenu par lyophilisation d'un volume V_{Humalog dialysé} dont la préparation est décrite dans l'exemple B21, de telle manière que le ratio V_{polysach./glargine}/V_{Humalog dialysé} = C_{Humalog dialysé}/Cₗᵢₛₚᵣₒ où C_{Humalog dialysé} est la concentration en lispro (UI/mL) obtenue à l'issue de la dialyse de la solution commerciale, étape décrite dans l'exemple B19, et Cₗᵢₛₚᵣₒ est la concentration en lispro (UI/mL) visée dans la composition. La solution est limpide. La teneur en zinc de la formulation est ajustée à la concentration C_{zinc} (µM) souhaitée par ajout d'une solution concentrée de chlorure de zinc. Le pH final est ajusté à 7 par ajout de NaOH ou HCl concentré.

La formulation est limpide, attestant de la bonne solubilité de glargine et lispro dans ces conditions de formulation. Cette solution est filtrée sur 0,22 µm et placée à +4°C.

### Exemple B27 : Préparation d'une composition dextrane substitué/glargine/lispro à pH 7 présentant une concentration en glargine de 200 UI/mL et une concentration de lispro de 33 UI/mL (proportion en pourcentage d'insuline : 85/15 en glargine/lispro).

Une solution de glargine concentrée à 200 UI/mL est préparée selon l'exemple B18. Une composition Polysaccharide 18 (13 mg/mL)/glargine 300 UI/mL pH 7 est préparée à partir du Polysaccharide 18 et selon le mode de préparation décrit à l'exemple B22. Cette composition Polysaccharide 18/glargine 200 UI/mL est ajoutée à un lyophilisat d'insuline lispro obtenu par lyophilisation de la solution d'analogue rapide sous sa forme commerciale, selon le mode de préparation décrit dans l'exemple B25. La solution est limpide. La teneur en zinc de la formulation est ajustée à la concentration C_{zinc} (µM) = 750 µM par ajout d'une solution concentrée de chlorure de zinc. Le pH final est ajusté à 7 par ajout de NaOH ou HCl concentré.

Cette composition est décrite dans le tableau 5.

### Exemple B28 : Préparation d'une composition dextrane substitué/glargine/lispro à pH 7 présentant une concentration en glargine de 200 UI/mL et une concentration de lispro de 66 UI/mL (proportion en pourcentage d'insuline : 75/25 en glargine/lispro).

Une solution de glargine concentrée à 200 UI/mL est préparée selon l'exemple B18. Une composition Polysaccharide 18 (13 mg/mL)/glargine 300 UI/mL pH 7 est préparée à partir du Polysaccharide 18 et selon le mode de préparation décrit à l'exemple B22. Cette composition Polysaccharide 18/glargine 200 UI/mL est ajoutée à un lyophilisat d'insuline lispro obtenu par lyophilisation de la solution d'analogue rapide sous sa forme commerciale, selon le mode de préparation décrit dans l'exemple B25. La solution est limpide. La teneur en zinc de la formulation est ajustée à la concentration C_{zinc} (µM) = 1500 µM par ajout d'une solution concentrée de chlorure de zinc. Le pH final est ajusté à 7 par ajout de NaOH ou HCl concentré.

La formulation est limpide, attestant de la bonne solubilité de glargine et lispro dans ces conditions de formulation. Cette solution est filtrée sur 0,22 µm et placée à +4°C. Cette composition est décrite dans le tableau 5.

### Exemple B29 : Préparation d'une composition dextrane substitué/glargine/lispro à pH 7 présentant une concentration en glargine de 300 UI/mL et une concentration en lispro de 100 UI/mL (proportion en pourcentage d'insuline : 75/25 en glargine/lispro).

Une solution de glargine concentrée à 300 UI/mL est préparée selon l'exemple B18. Une composition Polysaccharide 18 (23 mg/mL)/glargine 300 UI/mL pH 7 est préparée à partir du Polysaccharide 18 et selon le mode de préparation décrit à l'exemple B22. Cette composition Polysaccharide 18/glargine 300 UI/mL est ajoutée à un lyophilisat d'insuline lispro obtenu par lyophilisation de la solution d'analogue rapide sous sa forme commerciale, selon le mode de préparation décrit dans l'exemple B25. La solution est limpide. La teneur en zinc de la formulation est ajustée à la concentration C_{zinc} (µM) = 2000 µM par ajout d'une solution concentrée de chlorure de zinc. Le pH final est ajusté à 7 par ajout de NaOH ou HCl concentré.

La formulation est limpide, attestant de la bonne solubilité de glargine et lispro dans ces conditions de formulation. Cette solution est filtrée sur 0,22 µm et placée à +4°C. Cette composition est décrite dans le tableau 5.

### Exemple B30 : Préparation d'une composition dextrane substitué/glargine/lispro à pH 7 présentant une concentration en glargine de 250 UI/mL et une concentration en lispro de 150 UI/mL (proportion en pourcentage d'insuline : 63/37 en glargine/lispro).

Une solution de glargine concentrée à 300 UI/mL est préparée selon l'exemple B18. Une composition Polysaccharide 18 (19 mg/mL)/glargine 300 UI/mL pH 7 est préparée à partir du Polysaccharide 18 et selon le mode de préparation décrit à l'exemple B22. Cette composition Polysaccharide 18/glargine 250 UI/mL est ajoutée à un lyophilisat d'insuline lispro obtenu par lyophilisation de la solution d'analogue rapide sous sa forme commerciale, selon le mode de préparation décrit dans l'exemple B25. La solution est limpide. La teneur en zinc de la formulation est ajustée à la concentration C_{zinc} (µM) = 1500 µM par ajout d'une solution concentrée de chlorure de zinc. Le pH final est ajusté à 7 par ajout de NaOH ou HCl concentré.

La formulation est limpide, attestant de la bonne solubilité de glargine et lispro dans ces conditions de formulation. Cette solution est filtrée sur 0,22 µm et placée à +4°C. Cette composition est décrite dans le tableau 5.

### Exemple B31 : Préparation d'une composition dextrane substitué/glargine/lispro à pH 7 présentant une concentration en glargine de 333 UI/mL et une concentration en lispro de 67 UI/mL (proportion en pourcentage d'insuline : 83/17 en glargine/lispro).

Une solution de glargine concentrée à 333 UI/mL est préparée selon l'exemple B18. Une composition Polysaccharide 18 (20 mg/mL)/glargine 300 UI/mL pH 7 est préparée à partir du Polysaccharide 18 et selon le mode de préparation décrit à l'exemple 822. Cette composition Polysaccharide 18/glargine 333 UI/mL est ajoutée à un lyophilisat d'insuline lispro obtenu par lyophilisation de la solution d'analogue rapide sous sa forme commerciale, selon le mode de préparation décrit dans l'exemple 825. La solution est limpide. La teneur en zinc de la formulation est ajustée à la concentration C_{zinc} (µM) = 2000 µM par ajout d'une solution concentrée de chlorure de zinc. Le pH final est ajusté à 7 par ajout de NaOH ou HCl concentré.

La formulation est limpide, attestant de la bonne solubilité de glargine et lispro dans ces conditions de formulation. Cette solution est filtrée sur 0,22 µm et placée à +4°C. Cette composition est décrite dans le tableau 5.

### Exemple B32 : Préparation d'une composition dextrane substitué/glargine/lispro à pH 7 présentant une concentration en glargine de 300 UI/mL et une concentration en lispro de 100 UI/mL (proportion en pourcentage d'insuline : 75/25 en glargine/lispro).

Une solution de glargine concentrée à 300 UI/mL est préparée selon l'exemple B18. Une composition Polysaccharide 19 (23 mg/mL)/glargine 300 UI/mL pH 7 est préparée à partir du Polysaccharide 19 et selon le mode de préparation décrit à l'exemple B22. Cette composition Polysaccharide 19/glargine 300 UI/mL est ajoutée à un lyophilisat d'insuline lispro obtenu par lyophilisation de la solution d'analogue rapide sous sa forme dyalisée, selon le mode de préparation décrit dans l'exemple B26. La solution est limpide. La teneur en zinc de la formulation est ajustée à la concentration C_{zinc} (µM) = 3000 µM par ajout d'une solution concentrée de chlorure de zinc. Le pH final est ajusté à 7 par ajout de NaOH ou HCl concentré.

La formulation est limpide, attestant de la bonne solubilité de glargine et lispro dans ces conditions de formulation. Cette solution est filtrée sur 0,22 µm et placée à +4°C. Cette composition est décrite dans le tableau 5.

### Exemple B33 : Préparation d'une composition dextrane substitué/glargine/lispro à pH 7 présentant une concentration en glargine de 300 UI/mL et une concentration en lispro de 100 UI/mL (proportion en pourcentage d'insuline : 75/25 en glargine/lispro).

Une composition Polysaccharide 20 (23 mg/mL)/glargine 300 UI/mL pH 7 est préparée à partir du Polysaccharide 20 et selon le mode de préparation décrit à l'exemple B23. Cette composition Polysaccharide 20/glargine 300 UI/mL est ajoutée à un lyophilisat d'insuline lispro obtenu par lyophilisation de la solution d'analogue rapide issue de la dialyse d'une solution commerciale, selon le mode de préparation décrit à l'exemple B26. La solution est limpide. La teneur en zinc de la formulation est ajustée à la concentration C_{zinc} (µM) = 1500 µM par ajout d'une solution concentrée de chlorure de zinc. Le pH final est ajusté à 7 par ajout de NaOH ou HCl concentré.

La formulation est limpide, attestant de la bonne solubilité de glargine et lispro dans ces conditions de formulation. Cette solution est filtrée sur 0,22 µm et placée à +4°C. Cette composition est décrite dans le tableau 5.

**Tableau 5 : Compositions dextrane substitué/glargine/lispro à pH 7.**

| Exemple N° | Polysaccharide N° | C_{polysach.} (mg/mL) | C_{glargine} (UI/mL) | Cₗᵢₛₚᵣₒ (UI/mL) | C_{glargine}/ Cₗᵢₛₚᵣₒ (%/%) | PH |
|---|---|---|---|---|---|---|
| B27 | 18 | 13 | 200 | 33 | 85/15 | 7 |
| 828 | 18 | 13 | 200 | 66 | 75/25 | 7 |
| B29 | 18 | 23 | 300 | 100 | 75/25 | 7 |
| B30 | 18 | 19 | 250 | 150 | 63/37 | 7 |
| 831 | 18 | 20 | 333 | 67 | 83/17 | 7 |
| B32 | 19 | 23 | 300 | 100 | 75/25 | 7 |
| B33 | 20 | 23 | 300 | 100 | 75/25 | 7 |

### Exemple B34 : Prédpitation de différentes compositions dextrane substitué/glargine/lispro à pH 7 présentant différentes concentrations en insulines glargine et lispro et différentes proportions relatives des 2 insulines.

1 mL de composition dextrane substitué/Lantus^{®}/Humalog^{®} préparée à l'exemple B27 à 833 est ajouté dans 2 mL d'une solution de PBS contenant 20 mg/mL de BSA (bovine serum albumin). Le mélange PBS/BSA simule la composition du milieu sous-cutané. Un précipité apparaît.

Une centrifugation à 4000 trs/min est effectuée pour séparer le précipité du surnageant. Ensuite Lantus^{®} est dosé dans le surnageant. Les pourcentages de précipitation de Lantus^{®} sont similaires au contrôle décrit dans l'exemple B13. Les résultats sont résumés dans le tableau 6

**Tableau 6**

| Exemple N° | Polysaccharide N° | C_{polysach.} (mg/mL) | C_{glargine} (UI/mL) | Cₗᵢₛₚᵣₒ (UI/ mL) | C_{glargine}/ Cₗᵢₛₚᵣₒ (%/%) | Solubilisation de glargine et de lispro à pH 7 | Précipitation de glargine | % Précipitation |
|---|---|---|---|---|---|---|---|---|
| B27 | 18 | 13 | 200 | 33 | 85/15 | OUI | OUI | 96 |
| B28 | 18 | 13 | 200 | 66 | 75/25 | OUI | OUI | 86 |
| B29 | 18 | 23 | 300 | 100 | 75/25 | OUI | OUI | 91 |
| B30 | 18 | 19 | 250 | 150 | 63/37 | OUI | OUI | 90 |
| B31 | 18 | 20 | 333 | 67 | 83/17 | OUI | OUI | 93 |
| B32 | 19 | 23 | 300 | 100 | 75/25 | OUI | OUI | 98 |
| B33 | 20 | 23 | 300 | 100 | 75/25 | OUI | OUI | Non mesuré |

### Exemple B35 : Stabilité chimique des compositions :

Les compositions dextrane substitué/Lantus^{®}/insuline prandiale décrites aux exemples B7, B27, B28 et B29 ainsi que les contrôles correspondants sont placés à 30°C pendant 4 semaines. La réglementation requiert 95% d'insuline native (non dégradée) après 4 semaines à 30°C.

Après 4 semaines, les formulations étudiées remplissent le cahier des charges défini par la réglementation. Les résultats sont regroupés dans le tableau 7.

**Tableau 7**

| Compositions | Pourcentage de glargine natif après 4 semaines à 30°C | Pourcentage d'insuline prandiale native après 4 semaines à 30°C |
|---|---|---|
| Lantus^{®} (formulation commerciale) | 97 | na |
| Apidra^{®} (formulation commerciale) | na | 95 |
| Humalog^{®} (formulation commerciale) | na | 98 |
| B7 | 96 | 98 |
| 827 | 97 | 99 |
| 828 | 95 | 97 |
| B29 | 98 | 100 |

On obtient donc, quelque soit la formulation étudiée, un pourcentage d'insuline native supérieur à 95%, ce qui est conforme aux exigences de la réglementation.

### Exemple 836 : Injectabilité des solutions

Toutes les compositions préparées sont injectables avec les systèmes habituels d'injection d'insuline. Les solutions décrites dans les exemples B7 à B12 ainsi que les compositions décrites dans les exemples B27 à B33 sont injectées sans aucune difficulté aussi bien avec des seringues à insuline équipées d'aiguilles de 31 Gauge qu'avec des stylos à insuline de Novo Nordisk, commercialisés sous le nom de Novopen^{®}, équipés d'aiguilles de 31 Gauge.

### Exemple B37 : Protocole de mesure de la pharmacodynamie des solutions d'insuline Des études pré-cliniques ont été réalisées sur porcs en vue d'évaluer deux compositions selon invention :

Lantus^{®} / Apidra^{®} (75/25), formulée avec le Polysaccharide 4 (6 mg/mL) décrit à l'exemple B7, et

Lantus^{®} / Humalog^{®} (75/25), formulée avec le Polysaccharide 4 (6 mg/ml) décrit à l'exemple B8.

Les effets hypoglycémiants de ces compositions ont été comparés par rapport à des injections réalisées avec des injections simultanées, mais séparées, de Lantus^{®} (pH 4) puis d'une insuline prandiale Apidra^{®} ou Humalog^{®}.

Six cochons domestiques d'environ 50 kg, préalablement cathétérisés au niveau de la jugulaire, sont mis à jeun 2 à 3 heures avant le début de l'expérience. Dans l'heure précédant l'injection d'insuline, 3 prélèvements sanguins sont réalisés afin de déterminer le niveau basal de glucose.

L'injection d'insuline à la dose de 0,4 UI/kg est réalisée en injection sous-cutanée au niveau du cou, sous l'oreille de l'animal à l'aide du stylo à insuline Novopen^{®} équipé d'une aiguille 31 G.

Des prélèvements sanguins sont ensuite réalisés après 4, 8, 12, 16, 20, 30, 40, 50, 60, 90, 120, 240, 360, 480, 600, 660 et 720 minutes. Après chaque prélèvement, le cathéter est rincé avec une solution diluée d'héparine.

Une goutte de sang est prélevée pour déterminer la glycémie au moyen d'un glucomètre. Les courbes de pharmacodynamie du glucose sont ensuite tracées.

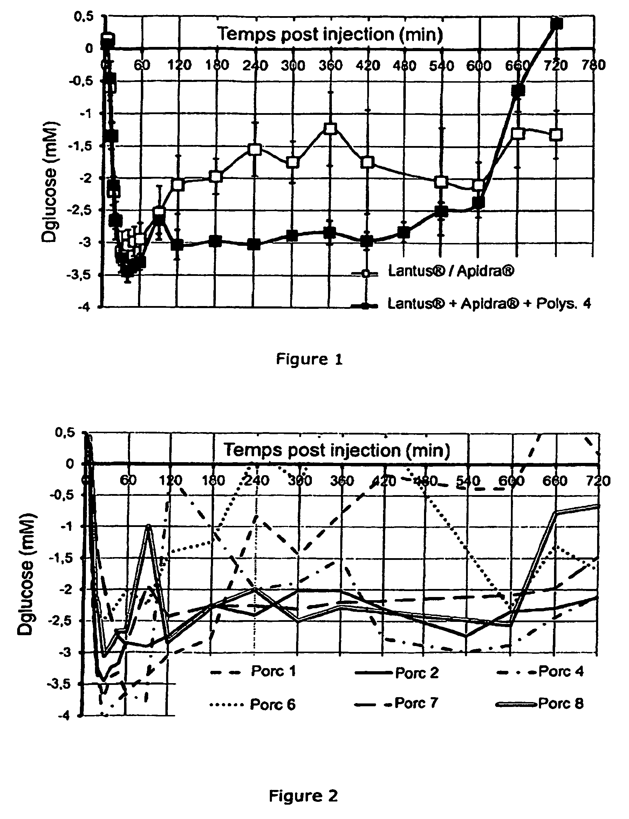

Les résultats obtenus sont présentés sous forme de courbes de pharmacodynamie du glucose représentées aux Figures 1 à 6.

Lantus^{®} / Apidra^{®} (75/25), formulée avec le Polysaccharide 4 (6 mg/mL).

Figure 1 : Courbes Moyenne + écart-type de la moyenne pour les administrations séquentielles de Apidra^{®} et Lantus^{®} en comparaison avec une composition selon l'invention Polysaccharide 4/ Lantus^{®} /Apidra^{®} (75/25)

Figure 2 : Courbes individuelles Apidra^{®} Lantus^{®}

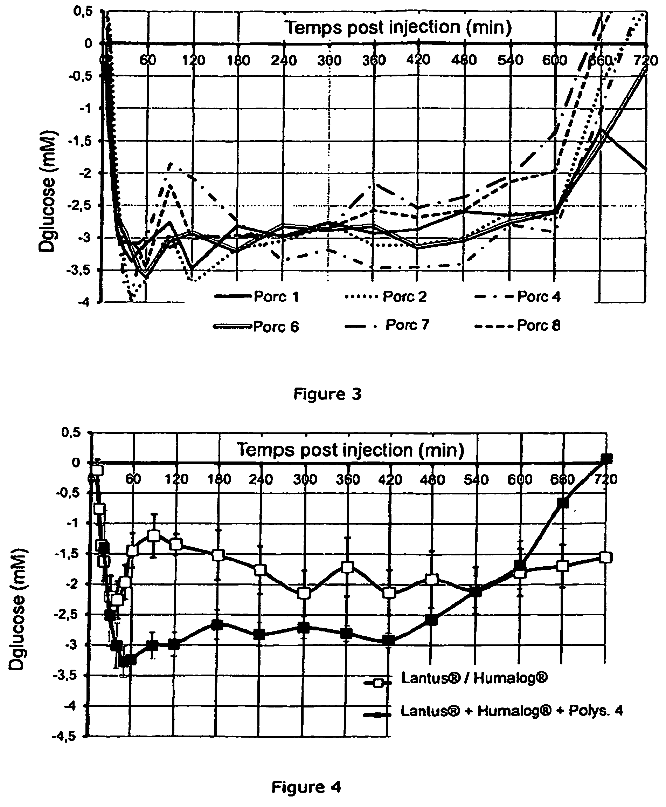

Figure 3 : Courbes individuelles Polysaccharide 4/Apidra^{®}/Lantus^{®}

La Figure 1 présente les courbes moyennes de chute de glycémie ainsi que les écarts-type de la moyenne pour les porcs testés pour chaque formulation. La chute de glycémie dans les 30 premières minutes est similaire pour les deux formulations indiquant que la présence d'un polysaccharide ne perturbe pas le caractère rapide d'Apidra^{®}.

En revanche, entre 90 min et 10 h (600 minutes) l'administration séquentielle de Apidra^{®} et Lantus^{®} induit une chute de glucose hétérogène avec une réponse en plateau homogène sur trois porcs et une réponse hétérogène sur les trois autres porcs (Figure 2). Au contraire, les 6 porcs testés avec la formulation Polysaccharide 4/Apidra^{®}/Lantus^{®} ont une réponse homogène (figure 3). Ceci se traduit par l'analyse des coefficients de variation (CV) entre 60 min et 10 h qui sont en moyenne de 54% (entre 21% et 113%) pour le contrôle Apidra^{®} Lantus^{®} et de 12% (entre 5% et 25%) pour Polysaccharide 4/Apidra^{®}/Lantus^{®}.

Lantus^{®}/Humalog^{®} (75/25), formulée avec le Polysaccharide 4 (6 mg/ml).

Figure 4 : Courbes Moyenne + écart-type de la moyenne pour les administrations séquentielles de Humalog^{®} et Lantus^{®} en comparaison avec l'administration d'une composition selon l'invention Polysaccharide 4/Humalog^{®}/Lantus^{®}

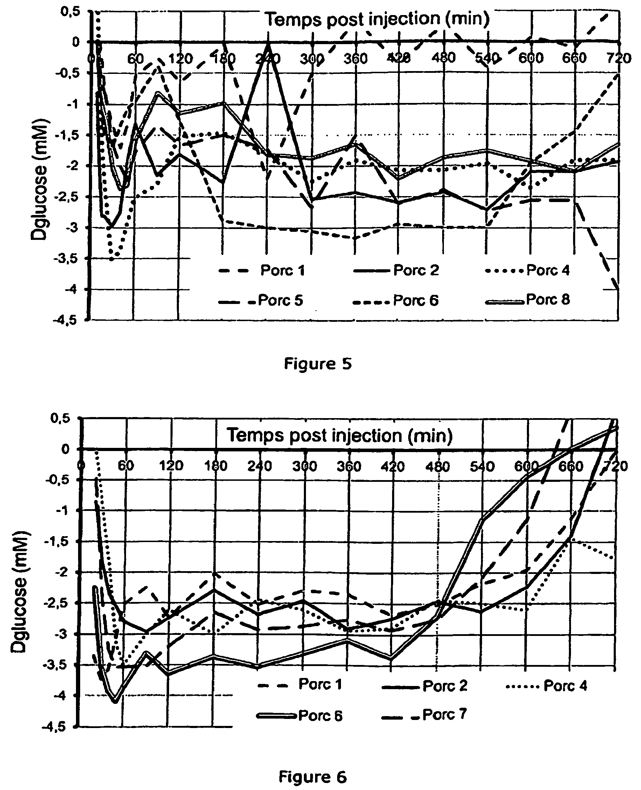

Figure 5 : Courbes individuelles Humalog^{®} Lantus^{®}

Figure 6 : Courbes Individuelles Polysaccharide 4/Humalog^{®}/Lantus^{®}

La figure 4 présente les courbes moyennes de chute de glycémie ainsi que les écart-types de la moyenne pour les porcs testés sur chaque formulation. La chute de glycémie dans les 30 premières minutes est similaire pour les deux formulations indiquant que la présence de Polysaccharide 4 ne perturbe pas le caractère rapide d'Humalog^{®}. En revanche, entre 60 min et 8 h (480 minutes), l'administration séquentielle de Humalog^{®} et Lantus^{®} induit une chute de glucose hétérogène avec une réponse en plateau homogène sur quatre porcs et une réponse hétérogène sur les deux autres porcs (Figure 5). Au contraire, les 5 porcs testés avec la formulation Polysaccharide 4/Humalog^{®}/Lantus^{®} ont une réponse homogène (figure 6). Ceci se traduit par l'analyse des coefficients de variation (CV) sur les données de chute de glycémie entre 60 min et 8h qui sont en moyenne de 54% (entre 31% et 72%) pour le contrôle Humalog^{®} Lantus^{®} et de 15% (entre 6% et 28%) pour Polysaccharide 4/Humalog^{®}/Lantus^{®}. La présence de Polysaccharide 4 réduit donc fortement la variabilité de Lantus^{®} sur la chute de glycémie.

### Exemple B38 : Protocole de mesure de la pharmacodynamie des solutions d'insuline. Des études pré-cliniques ont été réalisées sur chiens en vue d'évaluer 6 compositions selon l'invention :

Les effets hypoglycémiants de ces compositions ont été comparés par rapport à des injections réalisées avec des injections simultanées, mais séparées, de Lantus^{®} à 100 UI/ml (pH 4) puis d'une insuline prandiale Humalog^{®} à 100 UI/ml.

10 chiens domestiques (Beagle) d'environ 12 kg sont mis à jeun 18 heures avant le début de l'expérience. Dans l'heure précédant l'injection d'insuline, 3 prélèvements sanguins sont réalisés afin de déterminer le niveau basal de glucose.

L'injection d'insuline à la dose de 0,53 UI/kg (sauf mention contraire reportée dans les exemples ci-dessous) est réalisée en injection sous-cutanée au niveau du cou de l'animal à l'aide du stylo à insuline Novopen^{®} équipé d'une aiguille 31 G.

Des prélèvements sanguins sont ensuite réalisés après 10, 20, 30, 40, 50, 60, 90, 120, 180, 240, 300, 360, 420, 480, 540, 600, 660, 720, 780, 840, 900, 960 minutes. Les premiers prélèvements sont réalisés via un cathéter (jusqu'à 180 min), puis directement au niveau de la jugulaire. Après chaque prélèvement, le cathéter est rincé avec une solution diluée d'héparine.

Une goutte de sang est prélevée pour déterminer la glycémie au moyen d'un glucomètre. Les courbes de pharmacodynamie du glucose sont ensuite tracées.

Les résultats obtenus sont présentés sous forme de courbes de pharmacodynamie du glucose représentées aux Figures 7 à 12.

### La solution de l'exemple B28.

Figure 7 : Courbes Moyenne + écart-type de la moyenne pour les administrations séquentielles de Humalog^{®} (100 UI/ml, 0,13 UI/kg) et Lantus^{®} (100 UI/ml, 0,4 UI/kg) en comparaison avec une composition selon l'invention décrite dans l'exemple 828 (0,53 UI/kg).

La Figure 7 présente les courbes moyennes de chute de glycémie ainsi que les écarts-type de la moyenne pour les chiens testés pour chaque formulation. Les deux courbes sont similaires jusqu'à 12 heures avec une chute rapide de glycémie indiquant que le polysaccharide n'influe pas sur l'effet rapide d'Humalog^{®}, un retour marqué entre le pic dû à Humalog^{®} et le plateau dû à glargine puis un plateau du glargine jusqu'à 12h indiquant que l'effet basal de glargine est bien conservé.

### La solution de l'exemple B27.

Figure 8: Courbes Moyenne + écart-type de la moyenne pour les administrations séquentielles de Humalog^{®} (100 UI/ml, 0,13 UI/kg) et Lantus^{®} (100 UI/ml, 0,4 UI/kg) en comparaison avec une composition selon l'invention décrite dans l'exemple B27 (0,47 UI/kg).

La Figure 8 présente les courbes moyennes de chute de glycémie ainsi que les écarts-type de la moyenne pour les chiens testés pour chaque formulation. Dans cette comparaison, la dose d'insuline basale (Lantus^{®}) est identique alors que la dose d'Humalog^{®} est deux fois plus faible pour la combinaison par rapport au contrôle. La chute de glucose est plus importante dans le cas de la formulation B27 par rapport au contrôle alors que ce contrôle contient deux fois plus de Humalog^{®}. En revanche, la durée du plateau est plus faible dans le cas de la combinaison par rapport au contrôle. Ce qui indique que, dans cette composition, une partie de Lantus^{®} n'est pas précipitée à l'injection et agit avec Humalog^{®}.

### La solution de l'exemple B29.

Figure 9 : Courbes Moyenne + écart-type de la moyenne pour les administrations séquentielles de Humalog^{®} (100 UI/ml, 0,13 UI/kg) et Lantus^{®} (100 UI/ml, 0,4 UI/kg) en comparaison avec une composition selon l'inventiondécrite dans l'exemple B29 (0,53 UI/kg).

La Figure 9 présente les courbes moyennes de chute de glycémie ainsi que les écarts-type de la moyenne pour les chiens testés pour chaque formulation. Les deux courbes sont similaires avec une chute rapide de glycémie indiquant que le polysaccharide n'influe pas sur l'effet rapide d'Humalog^{®}, un retour marqué entre le pic dû à Humalog^{®} et le plateau dû à Lantus^{®} puis un plateau du Lantus^{®} jusqu'à 13h indiquant que l'effet basal de glargine est bien conservé.

### La solution de l'exemple B31.

Figure 10 : Courbes Moyenne + écart-type de la moyenne pour les administrations séquentielles de Humalog^{®} (100 UI/ml, 0,13 UI/kg) et Lantus^{®} (100 UI/ml, 0,4 UI/kg) en comparaison avec une composition selon l'invention décrite dans l'exemple B31 (0,48 UI/kg).

La Figure 10 présente les courbes moyennes de chute de glycémie ainsi que les écarts-type de la moyenne pour les chiens testés pour chaque formulation. Dans cette comparaison, la dose d'insuline basale (Lantus^{®}) est identique alors que la dose d' Humalog^{®} est deux fois plus faible pour la combinaison par rapport au contrôle. La chute de glucose est plus importante dans le cas du contrôle par rapport à la combinaison correspondant à l'exemple 831. Cette réponse était attendue compte tenu de la concentration deux fois plus faible d'Humalog^{®} dans la combinaison comparée au contrôle. Par ailleurs, la durée du plateau de Lantus^{®} est identique dans le cas de la combinaison par rapport au contrôle. Ceci indique que dans cette composition, et par comparaison avec la composition décrite dans l'exemple B29 (Figure 9), il est possible de moduler la quantité d'Humalog^{®} dans la combinaison sans modifier l'effet basal de Lantus^{®}.

### La solution de l'exemple 830.

Figure 11 : Courbes Moyenne + écart-type de la moyenne pour les administrations séquentielles de Humalog^{®} (100 UI/ml, 0,24 UI/kg) et Lantus^{®} (100 UI/ml, 0,4 UI/kg) en comparaison avec une composition selon l'invention décrite dans l'exemple 830 (0,64 UI/kg).

La Figure 11 présente les courbes moyennes de chute de glycémie ainsi que les écarts-type de la moyenne pour les chiens testés pour chaque formulation. Les deux courbes sont similaires avec une chute rapide de glycémie indiquant que le polysaccharide n'influe pas sur l'effet rapide d'Humalog^{®}, un retour marqué entre le pic dû à Humalog^{®}et le plateau dû à Lantus^{®} puis un plateau du Lantus^{®} jusqu'à 10h indiquant que l'effet basal de glargine est bien conservé.

### La solution de l'exemple B32,

Figure 12 : Courbes Moyenne + écart-type de la moyenne pour les administrations séquentielles de Humalog^{®} (100 UI/ml, 0,13 UI/kg) et Lantus^{®} (100 UI/ml, 0,4 UI/kg) en comparaison avec une composition selon l'invention décrite dans l'exemple B32 (0,53 UI/kg).

La Figure 12 présente les courbes moyennes de chute de glycémie ainsi que les écarts-type de la moyenne pour les chiens testés pour chaque formulation. Les deux courbes sont similaires jusqu'à 10 heures avec une chute rapide de glycémie indiquant que le polysaccharide n'influe pas sur l'effet rapide d'Humalog^{®}, un retour marqué entre le pic dû à Humalog^{®} et le plateau dû à Lantus^{®} puis un plateau de glargine indiquant que l'effet basal de glargine est conservé jusqu'à 10h.

En conclusion, les Figues 7 à 12 montrent qu'en modulant la composition du polysacharide, les concentrations de lispro et de glargine, il est possible d'obtenir des profils identiques à une double injection avec des proportions différentes d'insuline rapide et d'insuline basale. Il est aussi possible de moduler la durée de l'insuline basale sans Impacter l'Insuline rapide ou de moduler la quantité d'insuline rapide sans impacter l'effet de l'insuline basale.

### Exemples

Partie C : Mise en évidence des propriétés des compositions comprenant un analogue ou un dérivé de GLP-1 selon l'invention.

### Exemple C1 : Solution de GLP-1 analogue Exenatide (Byetta^{®}) à 0,25 mg/mL

Cette solution est une solution d'Exenatide commercialisée par la société Eli Lilly and Company sous le nom de Byetta^{®}.

### Exemple C2 : Solution de GLP-1 dérivé Liraglutide (Victoza^{®}) à 6mg/mL

Cette solution est une solution de Liraglutide commercialisée par la sodété Novo Nordisk sous le nom de Victoza^{®}.

### Exemple C3 : Solubilisation de Lantus^{®} à 100 UI/mL et à pH 7 à l'aide d'un dextrane substitué à la concentration de 10 mg/mL

20 mg d'un dextrane substitué choisi parmi ceux décrits dans le tableau 1 sont pesés précisément. Ce lyophilisat est repris par 2 mL de la solution d'insuline Glargine de l'exemple B4 afin d'obtenir une solution dont la concentration en polysaccharide est égale à 10 mg/mL. Après agitation mécanique sur rouleaux à température ambiante, la solution devient limpide. Le pH de cette solution est de 6,3. Le pH est ajusté à 7 avec une solution de soude 0,1 N. Cette solution limpide est filtrée sur membrane (0,22 µm) puis est placée à +4°C.

Généralisation : Des solutions limpides de Lantus à 100 UI/mL et à pH 7 ont également été obtenues avec des concentrations en dextranes substitués de 20 et 40 mg/mL en suivant le même protocole que celui décrit dans l'exemple C3. Ainsi, une masse de polysaccharide lyophilisé parmi ceux décrits dans le tableau 1 est pesée précisément. Ce tyophitisat est repris par la solution d'insuline Glargine de l'exemple B4 de manière à obtenir une solution dont la concentration en dextrane substitué est de 20 ou 40 mg/mL comme décrit dans le tableau 8. Après agitation mécanique sur rouleaux à température ambiante, la solution devient limpide. Le pH de cette solution est inférieur à 7. Le pH est ensuite ajusté à 7 avec une solution de soude 0,1 N. Cette solution finale limpide est filtrée sur membrane (0,22 µm) puis est placée à +4°C.

**Tableau 8 : Préparation d'une solution de Lantus^{®} à 100 UI/mL et à pH 7 à l'aide d'un dextrane substitué à la concentration de 10, 20, ou 40 mg/mL**

| Concentration finale en dextrane substitué (mg/mL) | Masse de dextrane substitué pesée (mg) | Volume de la solution d'insuline Glargine de l'exemple B4 ajouté (mL) |
|---|---|---|
| 10 | 20 | 2 |
| 20 | 40 | 2 |
| 40 | 80 | 2 |

### Exemple C4 : Préparation d'une composition Lantus^{®}/Byetta^{®} 70/30 à pH 7,5

A 0,21 mL de la solution d'insuline Glarglne de l'exemple B4 est ajouté 0,09 mL de la solution d'Exenatide de l'exemple C1 pour obtenir 0,3 mL d'une composition dont le pH est de 4,5 au mélange. La composition contenant 70 UI/mL de Lantus^{®} et 0,075 mg/mL de Byetta^{®} est limpide attestant de la bonne solubilité de Lantus^{®} et de Byetta^{®} dans ces conditions de formulation (pH 4,5). Le pH est ensuite ajusté à 7,5 avec une solution de soude 0,1 N. La composition devient alors trouble attestant de la mauvaise solubilité de la composition Lantus^{®}/Byetta^{®}à pH 7,5.

Des compositions Lantus^{®}/Byetta^{®} 70/30 ont également été préparées à pH 4,5 - 5,5 - 6,5 - 8,5 et 9,5 en suivant un protocole similaire à celui décrit dans l'exemple C4. Pour chacune de ces compositions, à 0,21 mL de la solution d'insuline Glargine de l'exemple B4 est ajouté 0,09 mL de la solution d'Exenatide de l'exemple C1 pour obtenir 0,3 mL d'une composition dont le pH est de 4,5 au mélange. La composition est limpide attestant de la bonne solubilité de Lantus^{®} et de Byetta^{®} dans ces conditions de formulation (pH 4,5). Le pH est ajusté à 5,5 ou 6,5 ou 8,5 ou 9,5 avec une solution de soude 0,1 N. Après ajustement du pH, la composition à 5,5 est légèrement trouble, les compositions 6,5 - 7,5 et 8,5 sont très troubles et la composition à pH 9,5 est limpide. Ces compositions sont placées à +4°C pendant 48h. Après 48h à +4°C, seule la composition à pH 4,5 reste limpide. L'aspect visuel après 48h des compositions Lantus^{®}/Byetta^{®} 70/30 à différents pH est résumé dans le tableau 9.

**Tableau 9 : Aspect visuel après 48h des compositions Lantus^{®}/Byetta^{®} 70/30 à différents pH**

| **Compositions Lantus^{®}/Byetta^{®} 70/30 à différents pH** | |
|---|---|
| **pH** | **Aspect Visuel à t = 48h** |
| 4,5 | Limpide |
| 5,5 | Présence d'un précipité |
| 6,5 | Présence d'un précipité |
| 7,5 | Présence d'un précipité |
| 8,5 | Présence d'un précipité |
| 9,5 | Présence d'un précipité |

### Exemple C5 : Préparation d'une composition Lantus^{®}/Victoza^{®} 70/30 à pH 7,5

A 0,21 mL de la solution d'insuline Glargine de l'exemple B4 est ajouté 0,09 ml de la solution de Liraglutide de l'exemple C2 pour obtenir 0,3 mL d'une composition dont le pH est de 7 au mélange. La composition contenant 70 UI/mL de glargine et 1,8 mg/mL d'Exenatide est trouble attestant de la mauvaise solubilité de la composition Lantus^{®}/Victoza^{®} dans ces conditions de formulation. Le pH est ajusté à 7,5 avec une solution de soude 0,1 N. Après ajustement du pH, la composition reste trouble. Cette composition est placée à +4°C pendant 48h.

Des compositions Lantus^{®}/Victoza^{®} 70/30 ont également été préparées à pH 4,5 - 5,5 - 6,5 - 8,5 et 9,5 en suivant un protocole similaire à celui décrit dans l'exemple C5. Pour chacune de ces compositions, à 0,21 mL de la solution d'insuline Glarglne de l'exemple B4 sont ajoutés 0,09 mL de la solution de Liraglutide de l'exemple C1 pour obtenir 0,3 mL d'une composition dont le pH est de 7. La composition est trouble attestant de la mauvaise solubilité de la composition Lantus^{®}/Victoza^{®} dans ces conditions de formulation (pH 7). Le pH est ajusté à 4,5 ou 5,5 ou 6,5 avec une solution d'acide chlorhydrique 0,1 N ou à pH 9,5 avec une solution de soude 0,1 N. Après ajustement du pH, les compositions à pH 4,5 - 5,5 et 6,5 sont troubles, attestant de la mauvaise solubilité de la composition Lantus^{®}/Victoza^{®} dans ces conditions de formulation. Ces compositions sont placées à +4°C pendant 48h. Après 48h à 4°C, seule la composition à pH 9,5 est limpide. L'aspect visuel après 48h des compositions Lantus^{®}/Vidoza^{®} 70/30 à différents pH est résumé dans le tableau 10.

**Tableau 10 : Aspect visuel après 48h des compositions Lantus^{®}/Victoza^{®} 70/30 à différents pH**

| **Compositions Lantus^{®}/Victoza^{®} 70/30 à différents pH** | |
|---|---|
| **pH** | **Aspect Visuel à t = 48h** |
| 4,5 | Présence d'un précipité |
| 5,5 | Présence d'un précipité |
| 6,5 | Présence d'un précipité |
| 7,5 | Présence d'un précipité |
| 8,5 | Présence d'un précipité |
| 9,5 | Limpide |

### Exemple C6 : Préparation d'une composition dextrane substitué-Lantus^{®}/Byetta^{®}70/30 à pH 7

A 0,21 mL de la solution de dextrane substitué/Lantus^{®} préparée à l'exemple C3 est ajouté 0,09 mL de la solution d'Exenatide de l'exemple C1 pour obtenir 0,3 mL d'une composition à pH 5,3. Le pH est ajusté à 7 avec une solution de soude 0,1 N. La composition contenant 7 mg/mL de polysaccharide, 70 UI/mL de Lantus^{®} et 0,075 mg/mL de Byetta^{®} est limpide attestant de la bonne solubilité de Lantus^{®} et de Byetta^{®} en présence du dextrane substitué à pH 7. Cette solution limpide est placée à +4°C.

Généralisation : Des compositions dextrane substitué-Lantus^{®}/Byetta^{®} à pH 7 ont également été préparées à des ratios volumiques V_{Lantus}/V_{Byetta} de 90/10, 50/50, 30/70, et 10/90 en suivant le même protocole que celui décrit dans l'exemple C6. Ainsi, à un volume V_{Lantus} de la solution de dextrane substitué/Lantus^{®} préparée à l'exemple C3 est ajouté un volume V_{Byetta} de la solution d'Exenatide de l'exemple C1 pour obtenir une composition dont le pH est ajusté à 7 avec une solution de soude 0,1 N. Les compositions obtenues (voir tableau 11) sont limpides attestant de la bonne solubilité de Lantus^{®} et de Byetta^{®} en présence d'un dextrane substitué à pH 7. Ces solutions limpides sont placées à +4°C.

### Exemple C7 : Préparation d'une composition dextrane substitué-Lantus^{®}/Byetta^{®} 100/50 à pH 7

0,150 mL de la solution d'Exenatide de l'exemple C1 sont lyophilisés, puis 0,3 mL d'une solution de dextrane substitué/Lantus^{®} préparée à l'exemple C3 sont ajoutés au lyophilisat afin d'obtenir une composition dont le pH est ajusté à 7 avec une solution de soude 0,1 N. La composition contenant 10mg/mL de polysaccharide, 100 UI/mL de Lantus^{®} et 0,125 mg/mL de Byetta^{®} est limpide attestant de la bonne solubilité de Lantus^{®} et de Byetta^{®} en présence du dextrane substitué à pH 7. Cette solution limpide est placée à +4°C.

**Tableau 11 : Concentrations finales des compositions obtenues aux exemples C6 et C7 en Lantus^{®}, dextrane substitué et Byetta^{®}**

| | Lantus^{®} | | [polysaccharide n°] (mg/mL) | Byetta^{®} (mg/mL) |
|---|---|---|---|---|
| | UI/mL | mg/mL | | |
| 100/50 | 100 | 3,5 | 10 | 0,125 |
| 90/10 | 90 | 3,15 | 9 | 0,025 |
| 70/30 | 70 | 2,45 | 7 | 0,075 |
| 50/50 | 50 | 1,75 | 5 | 0,125 |
| 30/70 | 30 | 1,05 | 3 | 0,175 |

### Exemple C8 : Préparation d'une composition dextrane substitué-Lantus^{®}/Victoza^{®} 70/30 à pH 7

A 0,21 mL de la solution de dextrane substitué/Lantus^{®} préparée à l'exemple C3 est ajouté 0,09 mL de la solution de Uraglutide de l'exemple C2 pour obtenir 0,3 mL d'une composition à pH 7,6. Le pH est ajusté à 7 avec une solution d'acide chlorhydrique 0,1 N. La composition contenant 7 mg/mL de polysaccharide, 70 UI/mL de Lantus^{®} et 1,8 mg/mL de Victoza^{®} est limpide attestant de la bonne solubilité de Lantus^{®} et de Victoza^{®} en présence du dextrane substitué à pH 7. Cette solution limpide est placée à +4°C.

Généralisation: Des compositions dextrane substitué-Lantus^{®}/Victoza^{®} à pH 7 ont également été préparées à des ratios volumiques V_{Lantus}/V_{Victoza} de 90/10, 50/50, 30/70, et 90/10 en suivant le même protocole que celui décrit dans l'exemple C6. Ainsi, à un volume V_{Lantus} de la solution de dextrane substitué/Lantus^{®} préparée à l'exemple B3 est ajouté un volume V_{Victoza} de la solution de Liraglutide de l'exemple C2 pour obtenir une composition dont le pH est ajusté à 7 avec une solution d'acide chlorhydrique 0,1 N.

Les compositions obtenues voir tableau 12 sont limpides attestant de la bonne solubilité de Lantus^{®} et de Victoza^{®} en présence d'un dextrane substitué à pH 7. Ces solutions limpides sont placées à +4°C.

### Exemple C9 : Préparation d'une composition dextrane substitué-Lantus^{®}/Victoza^{®} 100/50 à pH 7

0,150 mL de la solution de Liraglutide de l'exemple C2 sont lyophilisés, puis 0,3 mL d'une solution de dextrane substitué/Lantus^{®} préparée à l'exemple C3 sont ajoutés au lyophilisat afin d'obtenir une composition dont le pH est ajusté à 7 avec une solution de soude 0,1 N. La composition contenant 10 mg/mL de polysaccharide 100 UI/mL de Lantus^{®} et 3 mg/mL de Victoza^{®}est limpide attestant de la bonne solubilité de Lantus^{®} et de Victoza^{®} en présence du dextrane substitué à pH 7. Cette solution limpide est placée à +4°C.

**Tableau 12 : Concentrations finales des compositions obtenues aux exemples C8 et C9 en Lantus^{®}, dextrane substitué et Victoza^{®}**

| | Lantus^{®} | | [polysaccharide n°] (mg/ml) | Victoza^{®} (mg/mL) |
|---|---|---|---|---|
| | UI/mL | mg/mL | | |
| 100/50 | 100 | 3,5 | 10 | 3 |
| 90/10 | 90 | 3,15 | 9 | 0,6 |
| 70/30 | 70 | 2,45 | 7 | 1,8 |
| 50/50 | 50 | 1,75 | 5 | 3 |
| 10/70 | 30 | 1,05 | 3 | 4,2 |

### Exemple C10 : Préparation d'une composition dextrane substitué-Lantus^{®}/Apidra^{®}/Byetta^{®} 60/20/20 à pH 7

20 mg de Polysaccharide 4 lyophilisé décrit à l'exemple A3 sont pesés précisément. Ce lyophilisat est repris par 2 mL de la solution d'insuline Glargine de l'exemple 84. Après agitation mécanique sur rouleaux à température ambiante, la solution devient limpide. Le pH de cette solution est de 6,3. Le pH est ajusté à 7 avec une solution de soude 0,1 N. A 0,6 mL de la solution dextrane substitué/Lantus^{®} préparée précédemment sont ajoutés 0,2 mL de la solution d'Exenatide de l'exemple C1 et 0,2 mL de la solution d'insuline Glulisine de l'exemple 83 pour obtenir 1 mL d'une composition à pH 7. La composition contenant 6 mg/mL de polysaccharide, 60 UI/mL de Lantus^{®}, 20 UI/mL d'Apidra^{®} et 0,05 mg/mL de Byetta^{®} est limpide attestant de la bonne solubilité de Lantus^{®}, d'Apidra^{®} et de Byetta^{®}en présence du dextrane substitué à pH 7. Cette solution limpide est filtrée sur membrane (0,22 µm) puis est placée à +4°C.

### Exemple C11 : Précipitation de Lantus^{®}

0,250 mL de Lantus^{®} est ajouté dans 0,5 mL d'une solution de PBS (Phosphate Buffer Solution, solution de tampon phosphate) contenant 20 mg/mL de BSA (Bovine Serum Albumin, albumine de serum bovin). Le mélange PBS/BSA simule la composition du milieu sous-cutané.

Un précipité apparaît ce qui est en bon accord avec le mécanisme de fonctionnement de Lantus^{®} (précipitation à l'injection due à l'augmentation du pH).

Une centrifugation à 4000 trs/min est effectuée pour séparer le précipité du surnageant. Ensuite Lantus^{®} est dosé dans le surnageant. Il en résulte que 90% de Lantus^{®} se retrouve sous une forme précipitée.

### Exemple C12 : Précipitation d'une composition dextrane substitué/Lantus^{®}

0,250 mL de solution dextrane substitué/Lantus^{®} préparée à l'exemple C3 est ajouté dans 0,5 mL d'une solution de PBS contenant 20 mg/mL de BSA. Le mélange PBS/BSA simule la composition du milieu sous-cutané. Un précipité apparaît.

Une centrifugation à 4000 trs/min est effectuée pour séparer le précipité du surnageant. Ensuite Lantus^{®} est dosé dans le surnageant. Il en résulte que 90% de Lantus^{®} se retrouve sous une forme précipitée. Ce pourcentage de précipitation de Lantus^{®} est identique à celui obtenu pour le contrôle décrit dans l'exemple C11.

### Exemple C13 : Précipitation d'une composition dextrane substitué-Lantus^{®}/Byetta^{®}

0,250 mL de la composition dextrane substitué-Lantus^{®}/Byetta^{®} préparée à l'exemple C6 est ajouté dans 0,5 mL d'une solution de PBS contenant 20 mg/mL de BSA. Le mélange PBS/BSA simule la composition du milieu sous-cutané. Un précipité apparaît.

Une centrifugation à 4000 trs/min est effectuée pour séparer le précipité du surnageant. Ensuite Lantus^{®} et Byetta^{®} sont dosés dans le surnageant. Le pourcentage de précipitation de Lantus^{®} est similaire au contrôle décrit dans l'exemple C11.

### Exemple C14 : Précipitation d'une composition dextrane substitué-Lantus^{®}/Victoza^{®} 70/30

0,250 mL de la composition dextrane substitué-Lantus^{®}/Victoza^{®} préparée à l'exemple C8 est ajouté dans 0,5 mL d'une solution de PBS contenant 20 mg/mL de BSA (bovine serum albumin). Le mélange PBS/BSA simule la composition du milieu sous-cutané. Un précipité apparait.

Une centrifugation à 4000 trs/min est effectuée pour séparer le précipité du surnageant. Ensuite Lantus^{®} et Victoza^{®} sont dosés dans le surnageant. Le pourcentage de précipitation de Lantus^{®} est similaire au contrôle décrit dans l'exemple C11.

### Exemple C15: Précipitation de différentes compositions en faisant varier la nature du dextrane substitué

D'autres essais dans les mêmes conditions que ceux des exemples C13 et C14 ont été effectués en présence d'autres dextranes.

Des résultats avec au plus 20 mg/mL de dextrane substitué et une composition Lantus^{®}/Byetta^{®} 70/30 sont regroupés dans le tableau 13 suivant. On observe que la solubilisation et la précipitation de Lantus^{®} sont conservées.

**Tableau 13 : Résultats des essais de solubilisation et de précipitation obtenus avec au plus 20 mg/mL de dextrane substitué et une composition Lantus^{®}/Byetta^{®} 70/30**

| Polysaccharide n° | Solubilisation Lantus^{®}/Byetta^{®} 70/30 | Pourcentage de précipitation de Lantus^{®} |
|---|---|---|
| 1 | Oui | 94 |
| 2 | Oui | 96 |
| 5 | Oui | 88 |
| 7 | Oui | 95 |
| 10 | Oui | Non mesuré |
| 11 | Oui | 81 |
| 14 | Oui | Non mesuré |
| 16 | Oui | 96 |
| 26 | Oui | 81 |
| 27 | Oui | 96 |
| 28 | Oui | 96 |
| 29 | Oui | 95 |

Des résultats avec au plus 20 mg/mL de dextrane substitué et diverses compositions Lantus^{®}/Byetta^{®} sont regroupés dans le tableau 14 suivant. On observe que la solubilisation et la précipitation de Lantus^{®} sont conservées.

**Tableau 14 : Résultats des essais de solubilisation et de précipitation obtenus avec au plus 20 mg/mL de dextrane substitué et diverses compositions Lantus^{®}/Byetta^{®}**

| Polysaccharide n° | Ratio Lantus^{®}/Byetta^{®} | Solubilisation Lantus^{®}/Byetta^{®} | Pourcentage de précipitation de Lantus^{®} |
|---|---|---|---|
| 4 | 100/50 | Oui | 95 |
| 4 | 90/10 | Oui | 94 |
| 4 | 70/30 | Oui | 95 |
| 4 | 50/50 | Oui | 90 |
| 4 | 30/70 | Oui | 82 |
| 8 | 100/50 | Oui | 96 |
| 8 | 90/10 | Oui | 94 |
| 8 | 70/30 | Oui | 96 |
| 8 | 50/50 | Oui | 90 |
| 8 | 30/70 | Oui | 81 |

Des résultats avec au plus 40 mg/mL de dextrane substitué et une composition Lantus^{®}/Victoza^{®} 70/30 sont regroupés dans le tableau 15 suivant. On observe que la solubilisation et la précipitation de Lantus^{®} sont conservées.

**Tableau 15 : Résultats des essais de solubilisation et de précipitation obtenus avec au plus 40 mg/mL de dextrane substitué et une composition Lantus^{®}/Victoza^{®} 70/30**

| Polysaccharide n° | Solubilisation Lantus^{®}/Victoza^{®} 70/30 | Pourcentage de précipitation de Lantus^{®} |
|---|---|---|
| 1 | Oui | 95 |
| 2 | Oui | 97 |
| 5 | Oui | Non mesuré |
| 7 | Oui | 97 |
| 10 | Oui | Non mesuré |
| 11 | Oui | Non mesuré |
| 14 | Oui | 90 |
| 16 | Oui | 97 |
| 26 | Oui | 74 |
| 27 | Oui | 96 |
| 28 | Oui | 95 |
| 29 | Oui | 94 |

Des résultats avec au plus 20 mg/mL de dextrane substitué et diverses compositions Lantus^{®}Nictoza^{®} sont regroupés dans le tableau 16 suivant. On observe que la solubilisation et la précipitation de Lantus^{®} sont conservées.

**Tableau 16 : Résultats des essais de solubilisation et de précipitation obtenus avec au plus 20 mg/mL de dextrane substitué et diverses compositions Lantus^{®}/Victoza^{®}**

| Polysaccharide n° | Ratio Lantus^{®}/Victoza^{®} | Solubilisation Lantus^{®}/Victoza^{®} | Pourcentage de précipitation de Lantus^{®} |
|---|---|---|---|
| 4 | 90/10 | Oui | 94 |
| 4 | 70/30 | Oui | Non mesuré |
| 4 | 50/50 | Oui | 90 |
| 4 | 30/70 | Oui | 86 |
| 8 | 100/50 | Oui | 93 |
| 8 | 90/10 | Oui | 95 |
| 8 | 70/30 | Oui | 98 |
| 8 | 50/50 | Oui | 89 |
| 8 | 30/70 | Oui | 85 |

### Exemple C16 : Précipitation d'une composition dextrane substitué-Lantus^{®}/Apidra^{®}/Byetta^{®} 60/20/20 à pH 7

0,250 mL de la composition dextrane substitué-Lantus^{®}/Apidra^{®}/Byetta^{®} préparée à l'exemple C10 sont ajoutés dans 0,5 mL d'une solution de PBS contenant 20 mg/mL de BSA. Le mélange PBS/BSA simule la composition du milieu sous-cutané. Un précipité apparaît.

Une centrifugation à 4000 trs/min est effectuée pour séparer le précipité du surnageant. Ensuite, Lantus^{®} est dosé dans le surnageant. Le pourcentage de précipitation de Lantus^{®} est similaire au contrôle décrit dans l'exemple C11.

## Revendications

1. Composition sous forme d'une solution aqueuse injectable, dont le pH est compris entre 6,0 et 8,0, comprenant au moins :
a) une insuline basale dont le point isoélectrique pI est compris entre 5,8 et 8,5;
b) un dextrane substitué par des radicaux porteurs de charges carboxylates et des radicaux hydrophobes de formule I ou de formule II : 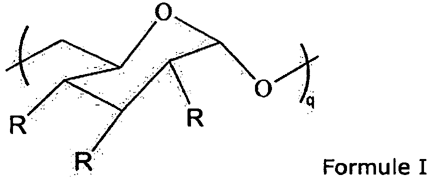 dans laquelle,
• R est -OH ou choisi dans le groupe constitué par les radicaux :
∘ -(*f*-[A]-COOH)ₙ ;
∘ -(*g*-[B]-*k*-[D])ₘ, D comportant au moins une chaîne alkyle comportant au moins 8 atomes de carbone ;
• n représente le degré de substitution des unités glucosidiques par -*f*-[A]-COOH et 0,1 ≤ n ≤ 2 ;
• m représente le degré de substitution des unités glucosidiques par -*g*-[B]-*k*-[D] et 0 < m ≤ 0,5 ;
• q représente le degré de polymérisation en unités glucosidiques, c'est-à-dire le nombre moyen d'unités glucosidiques par chaîne de polysaccharide et 3 ≤ q ≤ 50 ;
• -(*f-*[A]-COOH)ₙ ;
∘ -A- est un radical linéaire ou ramifié comprenant 1 à 4 atomes de carbone ; ledit radical -A- :
∘ étant lié à une unité glucosidique par une fonction f choisie dans le groupe constitué par les fonctions éther, ester et carbamate ;
• -(*g*-[B]-*k*-[D])ₘ ;
∘ -B- est un radical au moins divalent linéaire ou ramifié comprenant 1 à 4 atomes de carbone ; ledit radical -B- :
∘ étant lié à une unité glucosidique par une fonction g choisie dans le groupe constitué par les fonctions éther, ester et carbamate ;
∘ étant lié à un radical -D par une fonction *k* ; *k* choisie dans le groupe constitué par les fonctions ester, amide, et carbamate ; ledit radical -D :
• étant un radical -X(-/-Y)ₚ, X étant un radical au moins divalent comprenant de 1 à 12 atomes choisis dans le groupe constitué par des atomes de C, N ou O, éventuellement porteur de fonctions carboxyle ou amine et/ou issu d'un acide aminé, d'un dialcool, d'une diamine ou d'un mono- ou polyéthylène glycol mono- ou diamine ; Y étant un groupe alkyle, linéaire ou cyclique, un alkylaryle ou un arylalkyle, en C8 à C30, éventuellement substitué par un ou plusieurs groupes alkyles en C1 à C3 ; p ≥ 1 et / une fonction choisie dans le groupe constitué par les fonctions ester, amide et carbamate ;
• *f, g* et *k* étant identiques ou différentes ;
• les fonctions acides libres étant sous forme de sels de cations alcalins choisis dans le groupe constitué par Na⁺ et K⁺ ;
• et lorsque p = 1, si Y est un alkyle en C8 à C14, alors q*m ≥ 2, si Y est un alkyle en C15, alors q*m ≥ 2 ; et si Y est un alkyle en C16 à C20, alors q*m ≥ 1 ;
• et lorsque p ≥ 2, si Y est un alkyle en C8 à C9, alors q*m ≥ 2 et, si Y est alkyle en C10 à C16, alors q*m ≥ 0,2 ; 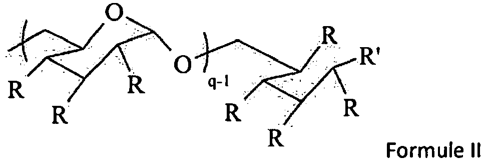 dans laquelle,
• R est -OH ou un radical -(*f*-[A]-COOH)ₙ :
∘ -A- est un radical linéaire ou ramifié comprenant 1 à 4 atomes de carbone ; ledit radical -A- :
∘ étant lié à une unité glucosidique par une fonction f choisie dans le groupe constitué par les fonctions éther, ester ou carbamate ;
∘ n représente le degré de substitution des unités glucosidiques par -*f-*[A]-COOH et 0,1 ≤ n ≤ 2 ;
• R' est choisi dans le groupe constitué par les radicaux :
∘ -C(O)NH-[E]-(*o*-[F])ₜ ;
∘ -CH₂N(L)_{z}-[E]-(*o*-[F])ₜ ; dans lesquels,
∘ z est un entier positif égal à 1 ou 2,
∘ L est choisi dans le groupe constitué de :
▪ H et z est égal à 1, et/ou
▪ -[A]-COOH et z est égal à 1 ou 2, si f est une fonction éther,
▪ -CO-[A]-COOH et z est égal à 1 si f est une fonction ester, et
▪ -CO-NH-[A]-COOH et z est égal à 1 si f est une fonction carbamate ;
∘ -[E]-(o-[F])ₜ :
▪ -E- est un radical au moins divalent linéaire ou ramifié comprenant 1 à 8 atomes de carbone et comprenant éventuellement des hétéroatomes tels que O, N ou S, ;
▪ -F étant un groupe alkyle, linéaire ou cyclique, un alkylaryle ou un arylalkyle, en C12 à C30 éventuellement substitué par un ou plusieurs groupes alkyles en C1 à C3 ;
▪ *o* est une fonction choisie dans le groupe constitué par les fonctions éther, ester, amide ou carbamate ;
▪ t est un entier positif égal à 1 ou 2 ;
• q représente le degré de polymérisation en unités glucosidiques, c'est-à-dire le nombre moyen d'unités glucosidiques par chaîne de polysaccharide et 3 ≤ q ≤ 50 ;
• les fonctions acides libres étant sous forme de sels de cations alcalins choisis dans le groupe constitué par Na⁺ et K⁺ ;
• lorsque z = 2, l'atome d'azote est sous forme d'un ammonium quaternaire.

2. Composition selon la revendication 1, **caractérisée en ce que** le dextrane substitué par des radicaux porteurs de charges carboxylates et des radicaux hydrophobes est choisi parmi les dextranes de formule I.

3. Composition selon l'une quelconque des revendications 1 à 2, **caractérisée en ce que** le dextrane substitué par des radicaux porteurs de charges carboxylates et des radicaux hydrophobes est choisi parmi les dextranes de formule I dans laquelle, le radical -(*f*-[A]-COOH)ₙ est choisi dans le groupe constitué par les enchainements suivants, *f* étant telle que définie à la revendication 1 :

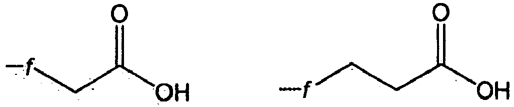

4. Composition selon l'une quelconque des revendications 1 à 3, **caractérisée en ce que** le dextrane substitué par des radicaux porteurs de charges carboxylates et des radicaux hydrophobes est choisi parmi les dextranes de formule I dans laquelle, le radical -(*g*-[B]-*k*-[D])ₘ est choisi dans le groupe constitué par les enchainements suivants ; *g, k* et D étant tels que définis à la revendication 1 :

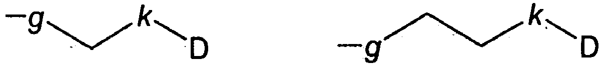

5. Composition selon l'une quelconque des revendications 1 à 4, **caractérisée en ce que** le dextrane substitué par des radicaux porteurs de charges carboxylates et des radicaux hydrophobes est choisi parmi les dextranes de formule I dans laquelle, le radical -(*g*-[B]-*k*-[D])ₘ est tel que :
• -B- est un radical comprenant 1 atome de carbone ; ledit radical -B- étant lié à une unité glucosidique par une fonction *g* éther, et, X est un radical issu d'un acide aminé.

6. Composition selon l'une quelconque des revendications 1 à 5, **caractérisée en ce que** le dextrane substitué par des radicaux porteurs de charges carboxylates et des radicaux hydrophobes est choisi parmi les dextranes de formule I dans laquelle, le radical X est un radical au moins divalent issu d'un acide aminé choisi dans le groupe constitué par la glycine, la leucine, la phénylalanine, la lysine, l'isoleucine, l'alanine, la valine, l'acide aspartique et l'acide glutamique.

7. Composition selon l'une quelconque des revendications 1 à 6, **caractérisée en ce que** le dextrane substitué par des radicaux porteurs de charges carboxylates et des radicaux hydrophobes est choisi parmi les dextranes de formule I dans laquelle, le groupe Y est choisi dans le groupe constitué d'un alcool hydrophobe, d'un acide hydrophobe, d'un stérol ou d'un tocophérol.

8. Composition selon l'une quelconque des revendications 1 à 7, **caractérisée en ce que** le dextrane substitué par des radicaux porteurs de charges carboxylates et des radicaux hydrophobes est choisi parmi les dextranes de formule I dans laquelle, le groupe Y est un stérol choisi parmi les dérivés du cholestérol.

9. Composition selon l'une quelconque des revendications 1 à 8, **caractérisée en ce que** le dextrane substitué par des radicaux porteurs de charges carboxylates et des radicaux hydrophobes est choisi dans le groupe constitué par les dextranes de formule I suivants :
- Dextraneméthylcarboxylate de sodium modifié par le glycinate d'octyle,
- Dextraneméthylcarboxylate de sodium modifié par le glycinate de cétyle,
- Dextraneméthylcarboxylate de sodium modifié par le phénylalaninate d'octyle,
- Dextraneméthylcarboxylate de sodium modifié par le phénylalaninate de 3,7-diméthyle-1-octyle,
- Dextraneméthylcarboxylate de sodium modifié par l'aspartate de dioctyle,
- Dextraneméthylcarboxylate de sodium modifié par l'aspartate de didécyle,
- Dextraneméthylcarboxylate de sodium modifié par l'aspartate de dilauryle,
- Dextraneméthylcarboxylate de sodium modifié par le N-(2-aminoéthyl)dodécanamide,
- Dextranesuccinate de sodium modifié par le glycinate de lauryle,
- N-méthylcarboxylate de sodium dextrane carbamate modifié par l'aspartate de dioctyle,
- Dextraneméthylcarboxylate de sodium modifié par le 2-(2-amino-éthoxy)éthyle dodécanoate,
- Dextraneméthylcarboxylate de sodium modifié par le 2-(2-{2-[dodécanoylamino]éthoxy}éthoxy)éthylamine,
- Dextraneméthylcarboxylate de sodium modifié par le 2-(2-{2-[hexadécanoylamino]éthoxy}éthoxy)éthylamine,
- Dextraneméthylcarboxylate de sodium modifié par le leucinate de cholestéryle,
- Dextranméthylcarboxylate de sodium modifié par le 1-éthylènediaminecarboxylate de cholestéryle,
- N-méthylcarboxylate de sodium dextrane carbamate modifié par le leucinate de cholestéryle.

10. Composition selon l'une quelconque des revendications précédentes, **caractérisée en ce que** l'insuline basale dont le point isoélectrique est compris entre 5,8 et 8,5 est l'insuline glargine.

11. Composition selon l'une quelconque des revendications précédentes, **caractérisée en ce qu'**elle comprend entre 40 UI/mL et 500 UI/mL d'insuline basale dont le point isoélectrique est compris entre 5,8 et 8,5.

12. Composition selon l'une quelconque des revendications précédentes, **caractérisée en ce qu'**elle comprend en outre une insuline prandiale.

13. Composition selon l'une quelconque des revendications précédentes, **caractérisée en ce qu'**elle contient entre 40 et 800 UI/mL d'insuline totale.

14. Composition selon l'une quelconque des revendications 12 et 13, **caractérisée en ce qu'**elle contient des proportions exprimées en pourcentage entre l'insuline basale dont le point isoélectrique est compris entre 5,8 et 8,5 et une insuline prandiale de 25/75, 30/70, 40/60, 50/50, 60/40, 70/30, 80/20, 90/10.

15. Composition selon l'une quelconque des revendications 1 à 11, **caractérisée en ce qu'**elle comprend en outre un GLP-1, un analogue ou un dérivé de GLP-1.

16. Composition selon l'une quelconque des revendications précédentes, **caractérisée en ce qu'**elle comprend en outre des sels de zinc à une concentration comprise entre 0 et 5000 µM.

17. Composition selon l'une quelconque des revendications 12 à 14, **caractérisée en ce que** ladite insuline prandiale est choisie dans groupe formé par l'insuline humaine, l'insuline glulisine, l'insuline lispro et l'insuline aspart.

18. Formulation unidose contenant une composition selon l'une quelconque des revendications 1 à 14 et 16 à 17, à pH compris entre 6,6 et 7,8 et une insuline prandiale.

## Patentansprüche

1. Zusammensetzung in Form einer injizierbaren wässrigen Lösung, deren pH-Wert zwischen 6,0 und 8,0 beträgt, umfassend wenigstens:
a) ein Basal-Insulin, dessen isoelektrischer Punkt pI zwischen 5,8 und 8,5 beträgt;
b) ein Dextran, das substituiert ist durch Radikale, die Carboxylat-Ladungen tragen, und hydrophobe Radikale der Formel I oder der Formel II: 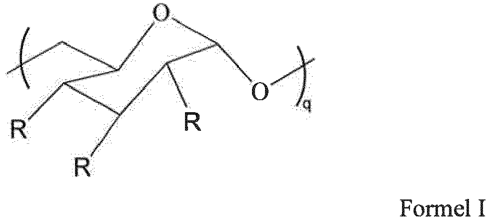 in der
• R -OH oder ausgewählt ist aus der Gruppe bestehend aus den Radikalen
∘ -(*ƒ*-[A]-COOH)ₙ ;
∘ -(*g*-[B]-*k*-[D])ₘ, wobei D wenigstens eine Alkylkette umfasst, welche wenigstens acht Kohlenstoffatome umfasst;
• n den Grad der Substitution der Glukosid-Einheiten durch -f-[A]-COOH und 0,1 ≤ n ≤ 2 angibt;
• m den Grad der Substitution der Glukosid-Einheiten durch *g*-[B]-*k*-[D] und 0 < m ≤ 0,5 angibt;
• q den Polymerisationsgrad als Glukosid-Einheiten angibt, das heißt die durchschnittliche Anzahl von Glukosid-Einheiten pro Polysaccharidkette und 3 ≤ q ≤ 50 ;
• -(*ƒ-*[A]-COOH)ₙ ;
∘ wobei -A- ein lineares oder verzweigtes Radikal ist umfassend ein bis vier Kohlenstoffatome, wobei das Radikal -A- :
∘ mit einer Glukosid-Einheit durch eine funktionelle Gruppe *ƒ* verbunden ist, die ausgewählt ist aus der Gruppe bestehend aus funktionellen Ether-, Ester- und Carbamat-Gruppen;
• -(*g*-[B]-*k*-[D])ₘ ;
∘ wobei -B- ein wenigstens divalentes lineares oder verzweigtes Radikal ist umfassend ein bis vier Kohlenstoffatome; wobei das Radikal -B- :
∘ mit einer Glukosid-Einheit durch eine funktionelle Gruppe g verbunden ist, die ausgewählt ist aus der Gruppe bestehend aus funktionellen Ether-, Ester- und Carbamat-Gruppen;
∘ mit einem Radikal D durch eine funktionelle Gruppe *k* verbunden ist; wobei *k* ausgewählt ist aus der Gruppe bestehend aus funktionellen, Ester-, Amid- und Carbamat-Gruppen; wobei das Radikal D:
▪ ein Radikal -X(-/-Y)ₚ ist, wobei X ein wenigstens divalentes Radikal ist umfassend 1 bis 12 Atomen, die ausgewählt sind aus der Gruppe bestehend aus C-, N- und O-Atomen, gegebenenfalls funktionelle Carboxyl- oder Amin-Gruppen tragend, und/oder resultierend aus einer Aminosäure, einem Dialkohol, einem Diamin oder einem Mono- oder Polyethylenglycol-Mono- oder -Diamin; wobei Y eine lineare oder zyklische Alkylgruppe, ein Alkylaryl oder ein Arylalkyl mit 8 bis 30 C-Atomen ist; optional substituiert mit einer oder mehreren Alkylgruppen mit ein bis drei C-Atomen, p ≥ 1 und / eine funktionelle Gruppe ist, die ausgewählt ist aus der Gruppe bestehend aus funktionellen Ester-, Amid- und Carbamat-Gruppen;
• *ƒ, g* und *k* identisch oder verschieden sind;
• die freien funktionellen Säuregruppen als Salze von Alkalimetalkationen vorliegen, die ausgewählt sind aus der Gruppe bestehend aus Na⁺ und K⁺;
• und, sofern p = 1 ist, wenn Y ein Alkyl aus 8 bis 14 C-Atomen ist, ist q*m ≥ 2, wenn Y ein Alkyl aus 15 C-Atomen ist, dann ist q*m ≥ 2; und wenn Y ein Alkyl mit 16 bis 20 C-Atomen ist, dann ist q*m ≥ 1 ;
• und, sofern p ≥ 2 ist, wenn Y ein Alkyl aus 8 oder 9 C-Atomen ist, dann ist q*m ≥ 2, und wenn Y ein Alkyl aus 10 bis 16 C-Atomen ist, dann ist q*m ≥ 2 ; 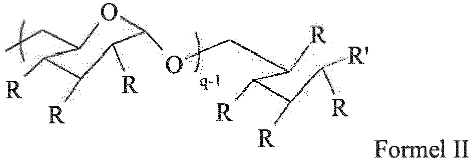 in der
• R -OH oder ein Radikal -(*ƒ*-[A]-COOH)ₙ ist;
∘ wobei -A- ein lineares oder verzweigtes Radikal ist umfassend ein bis vier Kohlenstoffatome; wobei das Radikal -A- ;
∘ mit einer Glucosid-Einheit durch eine funktionelle Gruppe *f* verbunden ist, die ausgewählt ist aus der Gruppe bestehend aus funktionellen Ether-, Ester und Carbamat-Gruppen
∘ n den Grad der Substitution der Glukosid-Einheiten durch -*ƒ-*[A]-COOH und 0,1 ≤ n ≤ 2 angibt;
• R' ausgewählt ist aus der Gruppe bestehend aus den Radikalen:
∘ -C(O)NH-[E]-*o*-[F])ₜ ;
∘ -CH₂N(L)_{z}-[E]-(*o*-[F])ₜ ;
bei denen
∘ z eine ganze positive Zahl von 1 oder 2 ist,
∘ L ausgewählt ist aus der Gruppe bestehend aus
▪ H und z ist 1, und/oder
▪ -[A]-COOH und z ist 1 oder 2, wenn *ƒ* eine funktionelle Ether-Gruppe ist,
▪ -CO-[A]-COOH und z ist 1, wenn *ƒ* eine Esterfunktion ist, und
▪ -CO-NH-[A]-COOH und z 1 ist, wenn *ƒ* eine funktionelle Carbamat-Gruppe ist;
∘ -[E]-(o-[F])ₜ :
▪ -E- ein wenigstens divalentes lineares oder verzweigtes Radikal ist, das 1 bis 8 Kohlenstoffatome umfasst und gegebenenfalls Heteroatome wie bspw. O, N oder S umfasst;
▪ -F eine zyklische oder lineare Alkylgruppe, ein Alkylaryl oder ein Arylakyl mit 12 bis 30 C-Atomen ist; optional substituiert mit einer oder mehreren Alkylgruppen mit ein bis drei C-Atomen;
▪ *o* eine funktionelle Ester-, Ether-, Amid- oder Carbamat-Gruppe ist;
▪ t eine ganze positive Zahl von 1 oder 2 ist;
• q den Polymerisationsgrad als Glukosid-Einheiten angibt, das heißt die durchschnittliche Anzahl von Glukosid-Einheiten pro Polysaccharidkette und 3 ≤ q ≤ 50;
• die freien funktionellen Säuregruppen als Salze von Alkalimetalkationen vorliegen, die ausgewählt sind aus der Gruppe bestehend aus Na⁺ und K⁺;
• wenn z = 2 ist, dann liegt das Stickstoffatom als quartäres Ammonium vor.

2. Zusammensetzung nach Anspruch 1, **dadurch gekennzeichnet, dass** das Dextran, das substituiert ist durch Radikale, die Carboxylat-Ladungen tragen, und hydrophobe Radikale, ausgewählt ist aus den Dextranen der Formel I.

3. Zusammensetzung nach einem der Ansprüche 1 bis 2, **dadurch gekennzeichnet, dass** das Dextran, das substituiert ist durch Radikale, die Carboxylat-Ladungen tragen, und hydrophobe Radikale, ausgewählt ist aus den Dextranen der Formel I, wobei das Radikal -(*ƒ*-[A]-COOH)ₙ ausgewählt ist aus der Gruppe bestehend aus den folgenden Sequenzen, wobei *ƒ* wie in Anspruch 1 definiert ist:

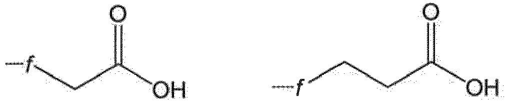

4. Zusammensetzung nach einem der Ansrpüche 1 bis 3, **dadurch gekennzeichnet, dass** das Dextran, das substituiert ist durch Radikale, die Carboxylat-Ladungen tragen, und hydrophobe Radikale, ausgewählt ist aus den Dextranen der Formel I, wobei das Radikal -(*g*-[B]-*k*-[D])ₘ ausgewählt ist aus der Gruppe bestehend aus den folgenden Sequenzen; wobei g, *k* und D wie in Anspruch 1 definiert ist:

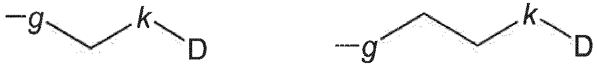

5. Zusammensetzung nach einem der Ansprüche 1 bis 4, **dadurch gekennzeichnet, dass** das Dextran, das substituiert ist durch Radikale, die Carboxylat-Ladungen tragen, und hydrophobe Radikale, ausgewählt ist aus den Dextranen der Formel I, wobei das Radikal -(*g*-[B]-*k*-[D])ₘ wie folgt ist:
• -B- ist ein Radikal umfassend ein Kohlenstoffatom; wobei das Radikal -B- mit einer Gukosid-Einheit verbunden ist durch eine funktionelle Gruppe *g*, die ein Ether ist, und X ist ein aus einer Aminosäure gebildetes Radikal.

6. Zusammensetzung nach einem der Ansprüche 1 bis 5, **dadurch gekennzeichnet, dass** das Dextran, das substituiert ist durch Radikale, die Carboxylat-Ladungen tragen, und hydrophobe Radikale, ausgewählt ist aus den Dextranen der Formel I, wobei das Radikal X ein wenigstens divalentes Radikal ist, das aus einer Aminosäure gebildet ist, die ausgewählt ist aus der Gruppe bestehend aus Glycin, Leucin, Phenylalanin, Lysin, Isoleucin, Alanin, Valin, Asparaginsäure und Glutaminsäure.

7. Zusammensetzung nach einem der Ansprüche 1 bis 6, **dadurch gekennzeichnet, dass** das Dextran, das substituiert ist durch Radikale, die Carboxylat-Ladungen tragen, und hydrophobe Radikale, ausgewählt ist aus den Dextranen der Formel I, wobei die Gruppe Y ausgewählt ist aus der Gruppe bestehend aus einem hydrophoben Alkohol, einer hydrophoben Säure, einem Sterol oder einem Tocopherol.

8. Zusammensetzung nach einem der Ansprüche 1 bis 7, **dadurch gekennzeichnet, dass** das Dextran, das substituiert ist durch Radikale, die Carboxylat-Ladungen tragen, und hydrophobe Radikale, ausgewählt ist aus den Dextranen der Formel I, wobei die Gruppe Y ein Sterol ist, das ausgewählt ist aus Cholesterolderivaten.

9. Zusammensetzung nach einem der Ansprüche 1 bis 8, **dadurch gekennzeichnet, dass** das Dextran, das substituiert ist durch Radikale, die Carboxylat-Ladungen tragen, und hydrophobe Radikale, ausgewählt ist aus den folgenden Dextranen der Formel I:
- durch Octylglycinat modifiziertes Natrium-Dextranmethylcarboxylat;
- durch Cetylglycinat modifiziertes Natrium-Dextranmethylcarboxylat;
- durch Octylphenylalaninat modifiziertes Natrium-Dextranmethylcarboxylat;
- durch 3,7-Dimethyl-1-octyl-phenylalaninat modifiziertes Natrium-Dextranmethylcarboxylat;
- durch Dioctylaspartat modifiziertes Natrium-Dextranmethylcarboxylat;
- durch Didecyl-Aspartet modifiziertes Natrium-Dextranmethylcarboxylat;
- durch Dilauryl-Aspartat modifiziertes Natrium-Dextranmethylcarboxylat;
- durch N-(2-Aminoethyl)dodecanamid modifiziertes Natrium-Dextranmethylcarboxylat;
- durch Laurylglycinat modifiziertes Natrium-Dextransuccinat;
- durch Dioctylaspartat modifiziertes N-(Natrium-Methylcarboxylat)Dextrancarbamat;
- durch 2-(2-Aminoethoxy)ethyldodecanoat modifiziertes Natrium-Dextranmethylcarboxylat;
- durch 2-(2-{2-[Dodecanoylamino]ethoxy}ethoxy)ethylamin modifiziertes Natrium-Dextranmethylcarboxylat;
- durch 2-(2-{2-[hexadecanoylamino]ethoxy}ethoxy)ethylamin modifiziertes Natrium-Dextranmethylcarboxylat;
- durch Cholesterylleucinat modifiziertes Natrium-Dextranmethylcarboxylat;
- durch Cholesteryl-1-ethylendiamincarboxylat modifiziertes Natrium-Dextranmethylcarboxylat;
- durch Cholesteryl-Leucinat modifiziertes Natrium-Dextrancarbamat- N-methylcarboxylat.

10. Zusammensetzung nach einem der vorangehenden Ansprüche, **dadurch gekennzeichnet, dass** das Basal-Insulin, dessen isoelektrischer Punkt zwischen 5,8 und 8,5 beträgt, das Insulin glargin ist.

11. Zusammensetzung nach einem der vorangehenden Ansprüche, **dadurch gekennzeichnet, dass** sie zwischen 40 IU/ml und 500 IU/ml Basal-Insulin umfasst, dessen isoelektrischer Punkt zwischen 5,8 und 8,5 beträgt.

12. Zusammensetzung nach einem der vorangehenden Ansprüche, **dadurch gekennzeichnet, dass** sie unter anderem prandiales Insulin umfasst.

13. Zusammensetzung nach einem der vorangehenden Ansprüche, **dadurch gekennzeichnet, dass** sie zwischen 40 und 800 IU/ml Gesamt-Insulin enthält.

14. Zusammensetzung nach einem der Ansprüche 12 und 13, **dadurch gekennzeichnet, dass** die Anteile von Basal-Insulin, dessen isoelektrischer Punkt zwischen 5,8 und 8,5 beträgt, und einem prandialen Insulin, ausgedrückt als Prozentsatz, 25/75, 30/70, 40/60, 50/50, 60/40, 70/30, 80/20, 90/10 betragen.

15. Zusammensetzung nach einem der Ansprüche 1 bis 11, **dadurch gekennzeichnet, dass** sie unter anderem ein GLP-1, ein Analog oder ein Derivat von GLP-1 umfasst.

16. Zusammensetzung nach einem der vorangehenden Ansprüche, **dadurch gekennzeichnet, dass** sie unter anderem Zink-Salze umfasst mit einer Konzentration von 0 bis 5000 µM.

17. Zusammensetzung nach einem der Ansprüche 12 bis 14, **dadurch gekennzeichnet, dass** das prandiale Insulin ausgewählt ist aus der Gruppe, bestehend aus menschlichem Insulin, Insulin glulisin, Insulin lispro und Insulin aspart.

18. Zusammensetzung nach einem der Ansprüche 1 bis 14 und 16 bis 17, mit einem pH-Wert zwischen 6,6 und 7,8 und einem prandialen Insulin.

## Claims

1. Composition in the form of an injectable aqueous solution, the pH of which is between 6.0 and 8.0, comprising at least:
a) a basal insulin, the isoelectric point pI of which is between 5.8 and 8.5;
b) a dextran substituted by radicals carrying carboxylate charges and hydrophobic radicals of formula I or of formula II: 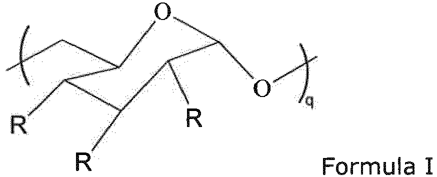 in which:
• R is -OH or chosen from the group consisting of the radicals:
∘ -(*f*-[A]-COOH)ₙ ;
∘ -(*g*-[B]-*k*-[D])ₘ, D comprising at least one alkyl chain comprising at least 8 carbon atoms;
• n represents the degree of substitution of the glucoside units by -*f*-[A]-COOH and 0.1 ≤ n ≤ 2;
• m represents the degree of substitution of the glucoside units by -*g*-[B]-*k*-[D] and 0 < m ≤ 0.5;
• q represents the degree of polymerization as glucoside units, that is to say the mean number of glucoside units per polysaccharide chain, and 3 ≤ q ≤ 50;
• -(*f*-[A]-COOH)ₙ :
∘ -A- is a linear or branched radical comprising from 1 to 4 carbon atoms; the said -A- radical:
∘ being bonded to a glucoside unit *via* a functional group *f* chosen from the group consisting of ether, ester and carbamate functional groups;
• -(*g*-[B]-*k*-[D])ₘ :
∘ -B- is a linear or branched, at least divalent, radical comprising from 1 to 4 carbon atoms; the said -B- radical:
∘ being bonded to a glucoside unit *via* a functional group *g* chosen from the group consisting of ether, ester and carbamate functional groups;
∘ being bonded to a -D radical via a functional group *k*; *k* chosen from the group consisting of ester, amide and carbamate functional groups; the said -D radical:
• being an -X(-/-Y)ₚ radical, X being an at least divalent radical comprising from 1 to 12 atoms chosen from the group consisting of C, N and O atoms, optionally carrying carboxyl or amine functional groups and/or resulting from an amino acid, a dialcohol, a diamine or a mono- or polyethylene glycol mono- or diamine; Y being a linear or cyclic alkyl group, an alkylaryl or an arylalkyl, of 8 to 30 carbon atoms, optionally substituted by one or more C₁ to C₃ alkyl groups; p ≥ 1 and / a functional group chosen from the group consisting of ester, amide and carbamate functional groups;
• *f*, *g* and *k* being identical or different;
• the free acid functional groups being in the form of salts of alkali metal cations chosen from the group consisting of Na⁺ and K⁺;
• and, when p = 1, if Y is a C₈ to C₁₄ alkyl, then q*m ≥ 2, if Y is a C₁₅ alkyl, then q*m ≥ 2; and if Y is a C₁₆ to C₂₀ alkyl, then q*m ≥ 1;
• and, when p ≥ 2, if Y is a C₈ to C₉ alkyl, then q*m ≥ 2 and, if Y is a C₁₀ to C₁₆ alkyl, then q*m ≥ 0.2; 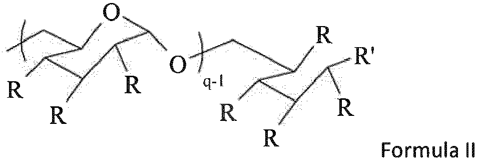 in which:
• R is -OH or a -(*f*-[A]-COOH)ₙ radical:
o -A- is a linear or branched radical comprising from 1 to 4 carbon atoms; the said radical -A-:
∘ being bonded to a glucoside unit *via* a functional group *f* chosen from the group consisting of ether, ester or carbamate functional groups;
∘ n represents the degree of substitution of the glucoside units by -f-[A]-COOH and 0.1 ≤ n ≤ 2;
• R' is chosen from the group consisting of the radicals:
∘ -C(O)NH-[E]-(*o*-[F])ₜ;
∘ -CH₂N(L)_{z}-[E]-(*o*-[F])ₜ;
in which:
o z is a positive integer equal to 1 or 2,
∘ L is chosen from the group consisting of:
▪ -H and z is equal to 1, and/or
▪ -[A]-COOH and z is equal to 1 or 2, if *f* is an ether functional group,
▪ -CO-[A]-COOH and z is equal to 1 if *f* is an ester functional group, and
▪ -CO-NH-[A]-COOH and z is equal to 1 if *f* is a carbamate functional group;
∘ -[E]-(o-[F])ₜ:
▪ -E- is a linear or branched, at least divalent, radical comprising from 1 to 8 carbon atoms and optionally comprising heteroatoms, such as O, N or S;
▪ -F- is a linear or cyclic alkyl group, an alkylaryl or an arylalkyl, of 12 to 30 carbon atoms, optionally substituted by one or more C₁ to C₃ alkyl groups;
▪ *o* is a functional group chosen from the group consisting of ether, ester, amide or carbamate functional groups;
▪ t is a positive integer equal to 1 or 2;
• q represents the degree of polymerization as glucoside units, that is to say the mean number of glucoside units per polysaccharide chain, and 3 ≤ q ≤ 50;
• the free acid functional groups being in the form of salts of alkali metal cations chosen from the group consisting of Na⁺ and K⁺;
• when z = 2, the nitrogen atom is in the form of a quaternary ammonium.

2. Composition according to Claim 1, **characterized in that** the dextran substituted by radicals carrying carboxylate charges and hydrophobic radicals is chosen from the dextrans of formula I.

3. Composition according to either one of Claims 1 and 2, **characterized in that** the dextran substituted by radicals carrying carboxylate charges and hydrophobic radicals is chosen from the dextrans of formula I in which the - (*f-*[A]-COOH)ₙ radical is chosen from the group consisting of the following sequences, *f* having the meaning as defined in claim 1:

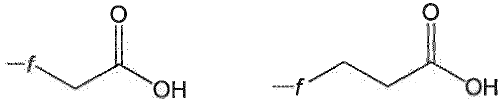

4. Composition according to any one of Claims 1 to 3, **characterized in that** the dextran substituted by radicals carrying carboxylate charges and hydrophobic radicals is chosen from the dextrans of formula I in which the -(*g*-[B]-*k*-[D])ₘ radical is chosen from the group consisting of the following sequences, *g, k* and D having the meanings as defined in claim 1:

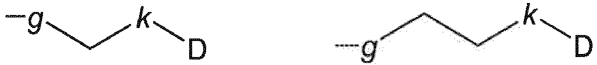

5. Composition according to any one of Claims 1 to 4, **characterized in that** the dextran substituted by radicals carrying carboxylate charges and hydrophobic radicals is chosen from of formula I in which the -(*g*-[B]-*k*-[D])ₘ radical is such that:
• -B- is a radical comprising one carbon atom; the said -B- radical being bonded to a glucoside unit *via* an ether functional group *g*, and X is a radical resulting from an amino acid.

6. Composition according to any one of Claims 1 to 5, **characterized in that** the dextran substituted by radicals carrying carboxylate charges and hydrophobic radicals is chosen from the dextrans of formula I in which the X radical is an at least divalent radical resulting from an amino acid chosen from the group consisting of glycine, leucine, phenylalanine, lysine, isoleucine, alanine, valine, aspartic acid and glutamic acid.

7. Composition according to any one of Claims 1 to 6, **characterized in that** the dextran substituted by radicals carrying carboxylate charges and hydrophobic radicals is chosen from the dextrans of formula I in which the Y group is chosen from the group consisting of a hydrophobic alcohol, a hydrophobic acid, a sterol or a tocopherol.

8. Composition according to any one of Claims 1 to 7, **characterized in that** the dextran substituted by radicals carrying carboxylate charges and hydrophobic radicals is chosen from the dextrans of formula I in which the Y group is a sterol chosen from cholesterol derivatives.

9. Composition according to any one of Claims 1 to 8, **characterized in that** the dextran substituted by radicals carrying carboxylate charges and hydrophobic radicals is chosen from the group consisting of the following dextrans of formula I:
- Sodium dextranmethylcarboxylate modified by octyl glycinate,
- Sodium dextranmethylcarboxylate modified by cetyl glycinate,
- Sodium dextranmethylcarboxylate modified by octyl phenylalaninate,
- Sodium dextranmethylcarboxylate modified by 3,7-dimethyl-1-octyl phenylalaninate,
- Sodium dextranmethylcarboxylate modified by dioctyl aspartate,
- Sodium dextranmethylcarboxylate modified by didecyl aspartate,
- Sodium dextranmethylcarboxylate modified by dilauryl aspartate,
- Sodium dextranmethylcarboxylate modified by N-(2-aminoethyl)dodecanamide,
- Sodium dextransuccinate modified by lauryl glycinate,
- N-(sodium methylcarboxylate) dextran carbamate modified by dioctyl aspartate,
- Sodium dextranmethylcarboxylate modified by 2-(2-aminoethoxy)ethyl dodecanoate,
- Sodium dextranmethylcarboxylate modified by 2-(2-{2-[dodecanoylamino]ethoxy}ethoxy)ethylamine,
- Sodium dextranmethylcarboxylate modified by 2-(2-{2-[hexadecanoylamino]ethoxy}ethoxy)ethylamine,
- Sodium dextranmethylcarboxylate modified by cholesteryl leucinate,
- Sodium dextranmethylcarboxylate modified by cholesteryl 1-ethylenediaminecarboxylate,
- N-(sodium methylcarboxylate) dextran carbamate modified by cholesteryl leucinate.

10. Composition according to any one of the preceding claims, **characterized in that** the basal insulin whose isoelectric point is between 5.8 and 8.5 is insulin glargine.

11. Composition according to any one of the preceding claims, **characterized in that** it comprises between 40 IU/ml and 500 IU/ml of basal insulin whose isoelectric point is between 5.8 and 8.5.

12. Composition according to any one of the preceding claims, **characterized in that** it additionally comprises a prandial insulin.

13. Composition according to any one of the preceding claims, **characterized in that** it comprises between 40 and 800 IU/ml of total insulin.

14. Composition according to either one of Claims 12 and 13, **characterized in that** it comprises proportions, expressed as percentage, between the basal insulin whose isoelectric point is between 5.8 and 8.5 and a prandial insulin of 25/75, 30/70, 40/60, 50/50, 60/40, 70/30, 80/20 and 90/10.

15. Composition according to any one of Claims 1 to 11, **characterized in that** it additionally comprises a GLP-1, a GLP-1 analogue or a GLP-1 derivative.

16. Composition according to any one of the preceding claims, **characterized in that** it additionally comprises zinc salts at a concentration of between 0 and 5000 µM.

17. Composition according to any one of Claims 12 to 14, **characterized in that** the said prandial insulin is chosen from the group formed by human insulin, insulin glulisine, insulin lispro and insulin aspart.

18. Single-dose formulation comprising a composition according to any one of Claims 1 to 14 and 16 to 17, at a pH of between 6.6 and 7.8, and a prandial insulin.
